# EUROPEAN PATENT APPLICATION

(11) **EP 3 543 231 A1**
(43) Date of publication of application: **25.09.2019**
(21) Application number: 18162579.9
(22) Date of filing: 19.03.2018
(51) Int. Cl.: C07D 403/04, C07D 405/04, C07D 405/06, C07D 405/12, C07D 417/12, A61P 25/00, A61K 31/4015, A61K 31/4427, A61K 31/541

(54) **COMPOUNDS FOR TREATING CNS- AND NEURODEGENERATIVE DISEASES**

(71) Applicant: ETH Zurich, 8092 Zurich (CH)
(72) Inventor: QUITTERER, Ursula, 8057 Zürich (CH); ABDALLA, Said, 55122 Mainz (DE)

(57) **Abstract**

**SUMMARY**

The present invention is directed to compounds and corresponding pharmaceutical formulations for use in the medical treatment of CNS- and neurodegenerative diseases, for example, for use in the treatment and prophylaxis of familial or sporadic Alzheimer's disease. The invention further relates to corresponding methods of treatment.

## Description

The present invention is directed to compounds and corresponding pharmaceutical formulations for use in the medical treatment of CNS- and neurodegenerative diseases, for example, for use in the treatment and prophylaxis of familial or sporadic Alzheimer's disease. The invention further relates to corresponding methods of treatment.

Alzheimer's disease (AD), the most frequent form of dementia, is a protein aggregation-associated disease. Age is the best-established risk factor for AD, and with increasing life expectancy, the incidence of AD is increasing worldwide. Treatment options for AD are limited. Currently, there are only four different drugs approved for the treatment of AD: three different acetylcholinesterase inhibitors, which enhance the availability of the cognition-enhancing acetylcholine, and the NMDA receptor antagonist, memantine, (Kulshreshtha & Piplani, Neurol. Sci. 37, 1403-1435, 2016). All these drugs cannot halt disease progression and relief AD symptoms only for a short time period. Therefore, there is an urgent need for disease-modifying treatment approaches. A possible target is the aberrant protein aggregation process leading finally to Abeta plaque formation. However, approaches that only interfere with Abeta plaque formation have not demonstrated efficacy in retarding AD progression, and even showed major side effects (Kulshreshtha & Piplani, 2016). The underlying reason could be the fact that the sole increase in Abeta aggregates does not cause substantial neuronal loss (AbdAlla et al., J. Biol. Chem. 284, 6554-6565, 2009; AbdAlla et al., J. Biol. Chem. 284, 6566-6574, 2009).

New approaches could possibly target Abeta-independent factors with neuropathological relevance in AD, e.g. Tau hyperphosphorylation, neurodegenerative AT2 receptor aggregation, the excessive generation of reactive oxygen species (ROS), inflammation and ACE-dependent angiotensin II AT1 receptor activation (Kulshreshtha & Piplani, 2016); AbdAlla et al., J. Biol. Chem. 284, 6554-6565 (2009); AbdAlla et al., J. Biol. Chem. 284, 6566-6574 (2009); AbdAlla et al., Int. J. Mol. Sci. 14, 16917-16942 (2013); AbdAlla et al., Biomed. Res. Int. 2015:917156 (2015)). In addition, environmental factors such as chronic mild stress, which also play a major role in the progression of neurodegenerative symptoms, need to be considered (AbdAlla et al., J. Biol. Chem. 284, 6554-6565 (2009); AbdAlla et al., J. Biol. Chem. 284, 6566-6574 (2009); Briones et al., Br. J. Pharmacol. 165, 897-907 (2012); AbdAlla et al., Biomed. Res. Int. 2015:917156 (2015)).

Mitochondrial dysfunction and mitochondrial damage has been identified as common factor underlying all major neurodegenerative and ageing-induced pathomechanisms (Valero T, Curr. Pharm. Des. 20, 5507-5509 (2014); Onyango et al., Aging Dis 7, 201-214 (2016); Onyango, Neural Regen Res. 13, 19-25 (2018); Jeong S. Mol. Cells 40, 613-620 (2017)). But to date, there are no successful approaches, which can treat mitochondrial dysfunction (Frozza et al., Front. Neurosci. 12:37 (2018)).

The problem underlying the present invention is the identification and provision of new compounds for use in the medical treatment of CNS- (central nervous system) and neurodegenerative diseases such as, for example, but not limited to dementia-associated CNS- and neurodegenerative disorders, preferably schizophrenia with dementia, depression-associated CNS- and neurodegenerative disorders, preferably depression and depression-related symptoms, preferably anhedonia and anorexia, brain injury, preferably traumatic brain injury, cerebrovascular disease-induced neurodegeneration (i.e. ischemic stroke-induced neurodegeneration, hypertension-induced neurodegeneration, atherosclerosis-induced neurodegeneration, amyloid angiopathy-induced neurodegeneration), and preferably small-vessel cerebrovascular disease, motor neuron disease, ALS (amyotrophic lateral sclerosis), multiple sclerosis, familial and sporadic forms of Alzheimer's Disease, vascular dementia, Morbus Parkinson, chromosome-17-linked Morbus Parkinson, frontotemporal dementia, Korsakoff's psychosis, Lewy Body diseases, progressive supranuclear palsy, corticobasal degeneration, Pick's disease, Huntington's disease, thalamic degeneration, prion-associated diseases, preferably Creutzfeld-Jacob disease, HIV-associated dementia, diabetes-induced neuropathy, neurodegenerative symptoms of ageing, preferably loss of appetite or greying of hair, cognitive-related disorders, mild cognitive impairment, age-associated memory impairment, age-associated cognitive decline, vascular cognitive impairment, central and peripheral neuronal symptoms of atherosclerosis and ischemia, stress-related CNS- and neurodegenerative disorders, attention deficit disorders, attention deficit hyperactivity disorders, memory disturbances in children, and progeria infantilis.

In a first aspect, the problem underlying the present invention is solved by a compound according to Formula (I) or (II): 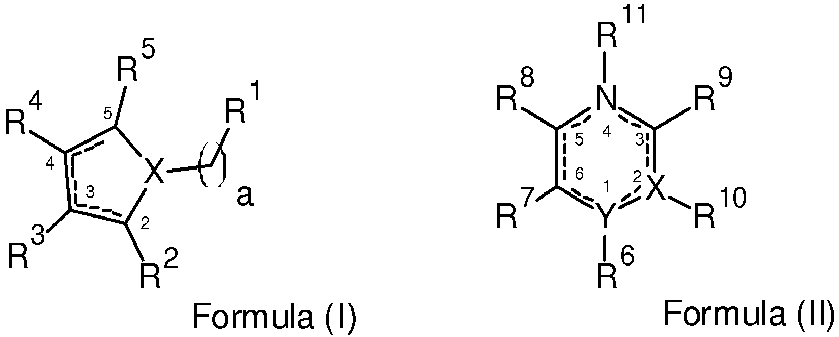 wherein:
the dotted lines between positions (2), (3), (4) and (5) in Formula (I) and between positions (1), (2), (3), (4), (5) and (6) in Formula (II) represent single bonds or double bonds between the respective positions;
**X** is selected from the group consisting of N and C;
**Y** is selected from the group consisting of S and C, with the proviso that when **Y** is C, **X** is N;
**a** is an integer between 0 and 15, preferably between 0 and 10, more preferably between 0 and 5, most preferably is 0 or 1;
**R¹** is selected from the group consisting of
   (i) hydrogen, hydroxyl, F, CI, Br and oxo, preferably if **X** is not N or if **X** is N and **a** is not 0, **R¹** is selected from the group consisting of hydroxyl, F, CI, Br and oxo;
   (ii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
   (iii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
   (iv) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of CI, F, Br, substituted or non-substituted methyl, preferably -(CF₃), ethyl, propyl and cyclopropyl; and
   (v) substituted or non-substituted (C₃₋₆)heterocycle and (C₇-C₁₀)carbo- or heterobicycle having 1 to 3 heteroatoms each independently selected from N, O and S, preferably substituted or non-substituted (C₇)heterobicycle having 2 heteroatoms selected from N and S, more preferably substituted or non-substituted indazolyl, benzimidazolyl and benzodioxolyl, preferably indazolyl, benzimidazolyl and benzodioxolyl connected via position (5) or (6), more preferably via position (6) of the indazolyl or benzodioxolyl or position (5) of the benzimidazolyl;
**R²** is selected from the group consisting of
   (i) hydrogen, hydroxyl, O-**R¹⁴**, -O-C(=O)-**R¹⁴**, F, CI, Br and oxo wherein **R¹⁴** is selected from the group consisting of
      (aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
      (bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in para position by (C₃)carbocycle or -(CF₃) or di-substituted in meta position by (C₃)carbocycle or -(CF₃); and
      (cc) substituted or non-substituted aromatic or non-aromatic, preferably aromatic, (C₃₋₆)heterocycle having 1 to 3 heteroatoms each independently selected from N, O and S;
   (ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl, and (C₃₋₁₀)carbocyc!e, preferably (C₃₋₆)cylcoalkyl;
   (iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether; and
   (iv) substituted or non-substituted (C₃₋₆)heterocycle and (C₇₋C₁₀)carbo- or heterobicycle having 1 to 3 heteroatoms each independently selected from N, O and S, preferably substituted or non-substituted (C₇)heterobicycle having 2 heteroatoms selected from N and S, more preferably substituted or non-substituted indazolyl, benzimidazolyl and benzodioxolyl, preferably indazolyl, benzimidazolyl and benzodioxolyl connected via position (5) or (6) of the indazolyl, benzodioxolyl or benzimidazolyl;
**R³** and **R⁴** are independently selected from the group consisting of
   (i) hydrogen, -O-**R¹⁴**, -O-C(=O)-**R¹⁴**, F, CI, Br and oxo, wherein **R¹⁴** is selected from the group consisting of
      (aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
      (bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in para position by (C₃)carbocycle or -(CF₃) or di-substituted in meta position by (C₃)carbocycle or -(CF₃); and
      (cc) substituted or non-substituted aromatic or non-aromatic, preferably aromatic, (C₃₋₆)heterocycle having 1 to 3 heteroatoms each independently selected from N, O and S;
   (ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl and (C₃₋₁₀)carbocycle, preferably substituted or non-substituted (C₃₋₆)cylcoalkyl and (C₃₋₆)heterocycle having 1 to 3 heteroatoms each independently selected from N, O and S;
   (iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
   (iv) 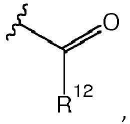 wherein **R¹²** is selected from the group consisting of
      (aa) hydrogen, hydroxyl, substituted or non-substituted N, F, Cl and Br;
      (bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
      (cc) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in para position by (C₃)carbocycle or -(CF₃) or di-substituted in meta position by (C₃)carbocycle or -(CF₃); and
      (dd) substituted or non-substituted aromatic or non-aromatic, preferably aromatic, (C₃₋₆)heterocycle having 1 to 3 heteroatoms each independently selected from N, O and S; and
   (v) 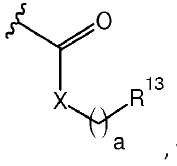 wherein X is N or C, a is an integer between 0 and 15, preferably between 0 and 10, more preferably between 0 and 5, most preferably is 0 or 1, and **R¹³** is selected from the group consisting of
      (aa) hydrogen, hydroxyl, F, Cl and Br;
      (bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
      (cc) substituted or non-substituted (C₃₋₁₀)carbocycle, preferably (C₃₋₆)cylcoalkyl, (C₇-C₁₀)carbo- or heterobicycle and (C₃₋₆)heterocycle having 1 to 3 heteroatoms each independently selected from N, O and S,
         more preferably, for **R³, R¹³** is (C₇)heterobicycle having 2 heteroatoms selected from N and S, most preferably substituted or non-substituted indazolyl, benzimidazolyl and benzodioxolyl, preferably indazolyl, benzimidazolyl and benzodioxolyl connected via position (5) or (6), more preferably via position (5) of the indazolyl and benzodioxolyl or position (6) of the benzimidazolyl, and
         most preferably, for **R⁴, R¹³** is substituted or non-substituted aromatic (C₆)carbocycle, preferably (C₆)carbocycle that is mono- or di-substituted in meta position by (C₃)-carbocycle or -(CF₃), or mono-substituted in para position by (C₃)-carbocycle or -(CF₃); and
      (dd) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
      wherein, if positions (2), (3) and/or (4) of the ring of Formula (I) are sp³-hybridized, **R²** and **R⁴** and/or **R³** and **R⁴** are preferably in cis or trans configuration to each other, more preferably in trans configuration, preferably, **R²** is (R)- or (S)-, **R³** is (R)- or (S)- and/or **R⁴** is (R)- or (S)-configured, more preferably, **R²** is (R)-, **R³** is (R)- and/or **R⁴** is (R)-configured, or **R²** is (S)-, **R³** is (S)- and/or **R⁴** is (S)-configured.
**R⁵** and **R⁹** are selected from the group consisting of
   (i) hydrogen, hydroxyl, F, CI, Br and oxo, with the proviso that **R⁹** is not oxo if **X** is N and **Y** is C;
   (ii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
   (iii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
   (iv) substituted or non-substituted (C₃₋₁₀)carbocyc!e, preferably substituted or non-substituted (C₃)carbocycle, substituted or non-substituted aromatic (C₅₋₆)carbocycle, more preferably cyclopenta-2,4-dien-1-yl and aromatic (C₆)carbocycle, most preferably phenyl that is non-substituted or substituted in para position by a substituent selected from the group consisting of Cl, F, Br, substituted or non-substituted methyl, preferably -(CF₃), ethyl, propyl and cyclopropyl; and
   (v) (C₃₋₆)heterocycle having 1 to 3 heteroatoms each independently selected from N, O and S, preferably substituted or non-substituted imidazolyl and pyrazolyl, more preferably imidazolyl and pyrazolyl connected via imidazolyl-/pyrazolyl-position-(1)-nitrogen to the rings of Formula (I);
   wherein, if position (5) of the ring of Formula (I) is sp³-hybridized, **R⁵** is preferably (S)- or (R)-configured, more preferably (R)-configured;
   and wherein, if position (3) of the ring of Formula (II) is sp³-hybridized, **R⁹** is preferably (S)- or (R)-configured, more preferably (S)-configured;
**R⁶** and **R¹¹** are independently selected from the group consisting of
   (i) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
   (ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
   (iii) substituted or non-substituted carbocycle selected from the group consisting of (C₃₋₁₀)carbocycle, preferably (C₃)carbocycle and (C₅₋₆)carbocycle, more preferably aromatic (C₆)carbocycle, most preferably phenyl that is non-substituted or mono- or di-substituted in meta and para position by a substituent selected from the group consisting of CI, F, Br, substituted or non-substituted methyl, ethyl, propyl and cyclopropyl; and
   (iv) substituted or non-substituted (C₃₋₆)heterocycle and (C₇-C₁₀)carbo- or heterobicycle having 1 to 3 heteroatoms each independently selected from N, O and S, preferably substituted or non-substituted (C₇)heterobicycle having 2 heteroatoms selected from N and S, most preferably substituted or non-substituted indazolyl, benzimidazolyl and benzodioxolyl, preferably indazolyl, benzimidazolyl and benzodioxolyl connected via position (5) or (6), more preferably via position (6) of the indazolyl or benzodioxolyl or position (5) of the benzimidazolyl,
   wherein **R⁶** is not present if **Y** is S; and/or
   wherein **R¹¹** is absent if the ring of Formula (II) has a double bond between positions (4) and (5) or between positions (3) and (4) of the ring of Formula (II),
   and with the proviso that **R⁶** is not 1,2,4-triazolyl if **X** is N, **Y** is C and the ring of Formula (II) is aromatic;
**R⁷** is selected from the group consisting of
   (i) hydrogen, hydroxyl, F, Cl, Br and oxo;
   (ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl and (C₃₋₁₀)carbocycle, preferably (C₃₋₆)cylcoalkyl and (C₃₋₆)heterocycle having 1 to 3 heteroatoms each independently selected from N, O and S;
   (iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether; and
   (iv) 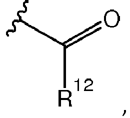 wherein **R¹²** is selected from the group consisting of
      (aa) hydrogen, hydroxyl, substituted or non-substituted N, F, Cl and Br;
      (bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl and aromatic or non-aromatic (C₃₋₁₀)carbocycle, preferably (C₃₋₆)cylcoalkyl, more preferably (C₃)carbocycle, most preferably aromatic (C₆)carbocycle that is mono-substituted in para position by (C₃)carbocycle or -(CF₃) or di-substituted in meta position by (C₃)carbocycle or -(CF₃); and
      (cc) substituted or non-substituted, aromatic or non-aromatic, preferably aromatic, (C₃₋₆)heterocycle having 1 to 3 heteroatoms each independently selected from N, O and S;
**R⁸** is selected from the group defined above for **R⁷**, the group further comprising
   (i) 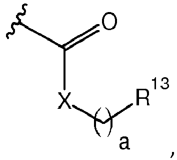 wherein **X** is N or C, **a** is an integer between 0 and 15, preferably between 0 and 10, more preferably between 0 and 5, most preferably is 0 or 1, and **R¹³** is selected from the group consisting of
      (aa) hydrogen, hydroxyl, F, Cl and Br;
      (bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
      (cc) substituted or non-substituted (C₃₋₁₀)carbocycle, preferably (C₃₋₆)cylcoalkyl, (C₇-C₁₀)carbo- or heterobicycle and (C₃₋₆)heterocycle having 1 to 3 heteroatoms each independently selected from N, O and S,
         more preferably substituted or non-substituted (C₇)heterobicycle having 2 heteroatoms selected from N and S, most preferably substituted or non-substituted indazolyl, benzimidazolyl and benzodioxolyl, preferably indazolyl and benzodioxolyl connected via position (5) or (6), more preferably via position (5) of the indazolyl and benzodioxolyl or position (6) of the benzimidazolyl,
         most preferably substituted or non-substituted aromatic (C₆)carbocycle, preferably (C₆)carbocycle that is mono- or di-substituted in meta position by (C₃)-carbocycle or -(CF₃) or mono-substituted in para position by (C₃)-carbocycle or -(CF₃); and
      (dd) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
      with the proviso that **R⁸** is not 1,2,4-triazolyl if **X** is N, **Y** is C and the ring of Formula (II) is aromatic,
      and wherein, if position (5) of the ring of Formula (II) is sp³-hybridized, **R⁸** is preferably (R)- or (S)-configured, more preferably (R)-configured;
**R¹⁰** is absent or selected from the group consisting of
   (i) hydrogen;
   (ii) methyl; and
   (iii) cyclopropyl or phenyl that is mono-substituted in para position by a substituent selected from the group consisting of H, Cl, F, Br, methyl, -(CF₃) and cyclopropyl;
   wherein one or more of **R² R³, R⁴, R⁵**, **R⁶**, **R⁷**, **R⁸**, **R⁹**, **R¹⁰** and **R¹¹** are either directly attached to the rings of Formulas (I) or (II) or are attached to a linker between **R²**, **R³, R⁴, R⁵**, **R⁶**, **R⁷**, **R⁸**, **R⁹**, **R¹⁰** and/or **R¹¹** and the rings of Formulas (I) or (II), wherein the linker is selected from the group consisting of linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether, linear or branched, substituted or non-substituted (C₁₋₁₀)alkyl, (C₂₋₁₀)alkenyl and (C₂₋₁₀)alkynyl;
   and pharmaceutically acceptable salts or solvates thereof;
for use in the medical treatment of CNS- and neurodegenerative diseases.

It was found that the herein-defined compounds inhibit major neuropathological features of CNS- and neurodegenerative diseases such as but not limited to AD, for example,
(I) Abeta plaque formation, Tau hyperphosphorylation, neuronal degeneration and neuronal loss in Tg2576 AD mice as a model of familial AD (FAD)
(II) Tau hyperphosphorylation in the chronic unpredictable mild stress (CUMS) model of sporadic AD, ageing and depression, and
(III) Tau hyperphosphorylation in the Tg-TauP301L transgenic model of tauopathy.
For more detail reference is made to the Examples and Figures further below.

The term medical treatment of CNS- and neurodegenerative diseases, as used herein, means prevention/prophylaxis and/or treatment of any disease or disorder associated with a malfunction of the peripheral and/or central nervous system.

In a preferred embodiment, the compound for use in the present invention is a compound, wherein
in Formula (I), a double bond is present between positions (3) and (4), or between positions (2) and (3) and between positions (4) and (5), or no double bond is present in the ring; and in Formula (II), no double bond is present in the ring or the ring is aromatic; and/or
**a** is 0 or 1; and/or
**R¹** is selected from the group consisting of
   (i) hydrogen;
   (ii) linear or branched, substituted or non-substituted (C₁₋₅)alkyl, more preferably methyl, ethyl and propyl, most preferably methyl;
   (iii) substituted or non-substituted cyclopropyl and phenyl, preferably substituted phenyl, more preferably phenyl that is mono-substituted in para position by a substituent selected from the group consisting of H, Cl, F, Br, methyl, -(CF₃) and cyclopropyl; and
   (iv) substituted or non-substituted, preferably non-substituted indazolyl, benzimidazolyl and benzodioxolyl connected via position (5) or (6), preferably via position (6) of the indazolyl and benzodioxolyl or position (5) of the benzimidazolyl.

In a further preferred embodiment, the compound for use according to the present invention is a compound, wherein
**R²** is selected from the group consisting of
   (i) hydrogen or oxo;
   (ii) linear or branched, substituted or non-substituted (C₁₋₅)alkyl, more preferably methyl, ethyl and propyl, most preferably methyl; and
   (iii) substituted or non-substituted indazolyl, benzimidazolyl and benzodioxolyl, preferably indazolyl, benzimidazolyl and benzodioxolyl connected via position (5) or (6) of the indazolyl, benzodioxolyl and benzimidazolyl; and/or
**R³** is selected from the group consisting of
   (i) hydrogen;
   (ii) linear or branched, substituted or non-substituted (C₁₋₅)alkyl, more preferably methyl, ethyl and propyl, most preferably methyl;
   (iii) 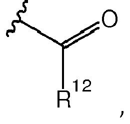 wherein **R¹²** is selected from the group consisting of
      (aa) N; and
      (bb) substituted or non-substituted cyclopropyl and phenyl, preferably phenyl that is mono-substituted in para position by cyclopropyl or -(CF₃) or di-substituted in meta position by cyclopropyl or -(CF₃) in each meta position; and
   (iv) 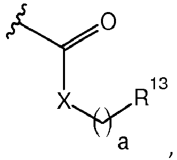 wherein **X** is N, **a** is 1 and **R¹³** is selected from the group consisting of substituted or non-substituted, preferably non-substituted indazolyl, benzimidazolyl and benzodioxolyl connected via position (6) or (5) of indazolyl, benzimidazolyl and benzodioxolyl, preferably via position (5) of the indazolyl and benzodioxolyl or position (6) of the benzimidazolyl;
   and/or
**R⁴** is selected from the group consisting of
   (i) hydrogen and hydroxyl;
   (ii) linear or branched, substituted or non-substituted (C₁₋₅)alkyl, more preferably methyl, ethyl and propyl, most preferably methyl;
   (iii) 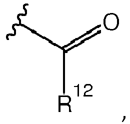 wherein **R¹²** is selected from the group consisting of
      (aa) N; and
      (bb) substituted or non-substituted cyclopropyl and phenyl, preferably phenyl that is mono-substituted in para or meta position by cyclopropyl or -(CF₃), or di-substituted in meta position by cyclopropyl or -(CF₃) in each meta position; and
   (iv) 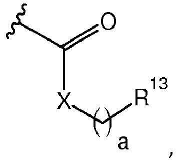 wherein **X** is N, **a** is 1 and **R¹³** is phenyl that is mono- or di-substituted in each meta position by cyclopropyl or -(CF₃) or mono-substituted in para position by cyclopropyl or -(CF₃);
   wherein, if positions (2), (3) and/or (4) of the ring of Formula (I) are sp³-hybridized **R²** and **R⁴** and/or **R³** and **R⁴** are preferably in cis or trans configuration to each other, more preferably in trans configuration, preferably, **R²** is (R)- or (S)-, **R³** is (R)- or (S)- and/or **R⁴** is (R)- or (S)-configured, more preferably, **R²** is (R)-, **R³** is (R)- and/or **R⁴** is (R)-configured, or **R²** is (S)-, **R³** is (S)- and/or **R⁴** is (S)-configured; and/or
**R⁵** is selected from the group consisting of
   (i) hydrogen;
   (ii) linear or branched, substituted or non-substituted (C₁₋₅)alkyl, more preferably methyl, ethyl and propyl, most preferably methyl;
   (iii) substituted or non-substituted cyclopropyl and phenyl, preferably substituted phenyl, more preferably phenyl that is mono-substituted in para position by a substituent selected from the group consisting of H, Cl, F, Br, methyl, -(CF₃) and cyclopropyl;
   (iv) cyclopenta-2,4-dien-1-yl; and
   (v) substituted or non-substituted, preferably non-substituted imidazolyl and pyrazolyl connected via the imidazolyl-/pyrazolyl-position-(1)-nitrogen to the ring of Formula (I);
   wherein, if position (5) of the ring of Formula (I) is sp³-hybridized, **R⁵** is preferably (R)- or (S)-configured, more preferably (R)-configured; and/or
**R⁶** and **R¹¹** are independently selected from the group consisting of substituted or non-substituted, preferably non-substituted indazolyl, benzimidazolyl and benzodioxolyl connected via position (6) or (5), preferably via position (6) of the indazolyl and benzodioxolyl or position (5) of the benzimidazolyl;
   wherein **R⁶** is not present if **Y** is S; and/or
   wherein **R¹¹** is absent if the ring of Formula (II) has a double bond between positions (4) and (5) or between positions (3) and (4) of the ring of Formula (II); and/or
**R⁷** and **R⁸** are independently selected from the group consisting of
   (i) hydrogen;
   (ii) linear or branched, substituted or non-substituted (C₁₋₅)alkyl, more preferably methyl, ethyl and propyl, most preferably methyl; and
   (iii) 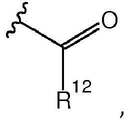 wherein **R¹²** is selected from the group consisting of
      (aa) N; and
      (bb) substituted or non-substituted cyclopropyl and phenyl, preferably phenyl that is mono-substituted in para position by cyclopropyl or -(CF₃), or di-substituted in meta position by cyclopropyl or -(CF₃) in each meta position;
      wherein, for **R⁸**, the group further comprises 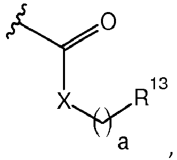 wherein **X** is N, **a** is 1 and **R¹³** is selected from the group consisting of substituted or non-substituted, preferably non-substituted indazolyl, benzimidazolyl and benzodioxolyl connected via position (6) or (5), preferably via position (5) of the indazolyl and benzodioxolyl or position (6) of the benzimidazolyl; and
      wherein, if position (5) of the ring of Formula (II) is sp³-hybridized, **R⁸** is preferably (R)- or (S)-configured, more preferably (R)-configured; and/or
**R⁹** is selected from the group consisting of
   (i) hydrogen, methyl and cyclopropyl; and
   (ii) substituted or non-substituted phenyl, preferably phenyl that is substituted in para position by a substituent selected from the group consisting of H, Cl, F, Br, methyl, - (CF₃) and cyclopropyl; and wherein, if position (3) of the ring of Formula (II) is sp³-hybridized, **R⁹** is preferably (R)- or (S)- configured, more preferably (S)-configured; and/or
**R¹⁰** is absent or selected from the group consisting of
   (i) hydrogen;
   (ii) methyl; and
   (iii) cyclopropyl and phenyl that is mono-substituted in para position by a substituent selected from the group consisting of H, Cl, F, Br, methyl, -(CF₃) and cyclopropyl,
   wherein **R¹⁰** is absent if **X** is C and/or if the ring of Formula (II) has a double bond between positions (1) and (2) or between positions (2) and (3) of the ring of Formula (II).

In a further preferred embodiment, the compound for use according to the present invention is a compound of Formula (I), wherein a double bond is located between positions (3) and (4) (Formula Ia) 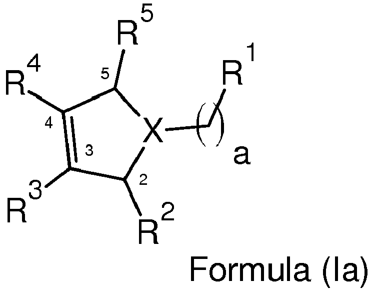 wherein X is N and a is 0, and wherein
**R¹** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R²** is oxo; and/or
**R³** is selected from the group consisting of
   (i) hydrogen;
   (ii) methyl; and
   (iii) 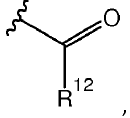 wherein **R¹²** is selected from the group consisting of
      (aa) N; and
      (bb) cyclopropyl and phenyl that is mono-substituted in para position by cyclopropyl or -(CF₃), or di-substituted in meta position by cyclopropyl or - (CF₃) in each meta position; and/or
**R⁴** is hydroxyl; and/or
**R⁵** is selected from the group consisting of
   (i) hydrogen;
   (ii) methyl;
   (iii) cyclopropyl and phenyl that is mono-substituted in para position by a substituent selected from the group consisting of H, Cl, F, Br, methyl, -(CF₃) and cyclopropyl;
   (iv) cyclopenta-2,4-dien-1-yl; and
   (v) imidazolyl and pyrazolyl connected via the imidazolyl-/pyrazolyl-position-(1)-nitrogen to the ring of Formula (I);
   wherein **R⁵** is preferably (R)- or (S)-configured, more preferably (R)-configured.

In a further preferred embodiment, the compound for use according to the present invention is a compound of Formula (I), wherein two double bonds are located between positions (2) and (3) and between positions (4) and (5), respectively (Formula Ib) 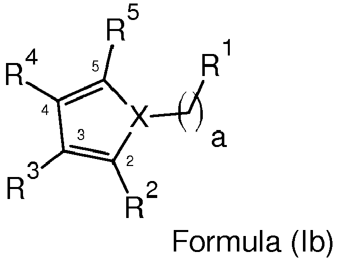 wherein **X** is N or C, preferably N, **a** is 0 or 1, preferably **a** is 0 if **X** is C, and wherein
**R¹** is selected from the group consisting of indazolyl, benzimidazolyl and benzodioxolyl connected via position (5) or (6), preferably via position (6) of the indazolyl and benzodioxolyl or position (5) of the benzimidazolyl; and/or
**R²** is selected from the group consisting of
   (i) hydrogen; and
   (ii) methyl; and/or
**R³** is selected from the group consisting of
   (i) hydrogen;
   (ii) methyl; and
   (iii) 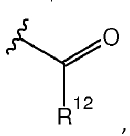 wherein **R¹²** is selected from the group consisting of
      (aa) N; and
      (bb) cyclopropyl and phenyl that is mono-substituted in para position by cyclopropyl or -(CF₃), or di-substituted in meta position by cyclopropyl or - (CF₃) in each meta position; and/or
**R⁴** is hydrogen; and/or
**R⁵** is selected from the group consisting of
   (i) hydrogen;
   (ii) methyl; and
   (iii) cyclopropyl and phenyl that is mono-substituted in para position by a substituent selected from the group consisting of H, Cl, F, Br, methyl, -(CF₃) and cyclopropyl.

In a further preferred embodiment, the compound for use according to the present invention is a compound of Formula (I), wherein the bonds in the five-membered ring of Formula (I) are fully saturated (Formula Ic) 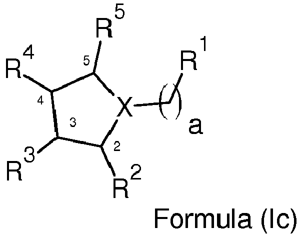 wherein **X** is N, **a** is 0 and wherein
**R¹** is selected from the group consisting of
   (i) hydrogen;
   (ii) methyl; and
   (iii) cyclopropyl and phenyl that is mono-substituted in para position by a substituent selected from the group consisting of H, Cl, F, Br, methyl, -(CF₃) and cyclopropyl; and/or
**R²** is selected from the group consisting of
   (i) hydrogen; and
   (ii) indazolyl, benzimidazolyl and benzodioxolyl, preferably indazolyl, benzimidazolyl and benzodioxolyl connected via position (5) or (6), more preferably via position (5) of the indazolyl and benzodioxolyl or position (6) of the benzimidazolyl if **R³** is not methyl, most preferably via position (6) of the indazolyl and benzodioxolyl or position (5) of the benzimidazolyl if **R³** is methyl; and/or
**R³** is selected from the group consisting of
   (i) hydrogen or methyl; and
   (ii) 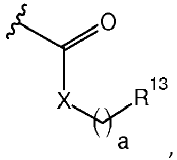 wherein **X** is N, **a** is 1 and **R¹³** is selected from the group consisting of indazolyl, benzimidazolyl and benzodioxolyl connected via position (6) or (5) of indazolyl, benzimidazolyl and benzodioxolyl, preferably via position (5) of the indazolyl and benzodioxolyl or position (6) of the benzimidazolyl; and/or
**R⁴** is selected from the group consisting of
   (i) hydrogen;
   (ii) methyl;
   (iii) 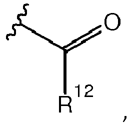 wherein **R¹²** is selected from the group consisting of
      (aa) N; and
      (bb) cyclopropyl and phenyl that is mono-substituted in para position by cyclopropyl or -(CF₃), or di-substituted in meta position by cyclopropyl or - (CF₃) in each meta position; and
   (iv) 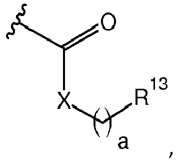 wherein **X** is N, **a** is 1 and **R¹³** is phenyl that is mono-substituted in meta position by cyclopropyl or -(CF₃), or di-substituted in each meta position by cyclopropyl or -(CF₃), or mono-substituted in para position by cyclopropyl or -(CF₃);
   wherein, **R²** and **R⁴** and/or **R³** and **R⁴** are preferably in a trans configuration to each other, preferably, **R²** is (S)-, **R³** is (S)- and **R⁴** is (R)-configured; and/or
**R⁵** is hydrogen.

In a further preferred embodiment, the compound for use according to the present invention is a compound of Formula (II), wherein the bonds within the ring of Formula (II) are fully saturated (Formula IIa) 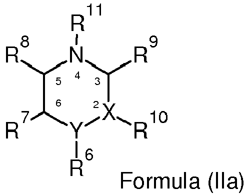 wherein **X** is C, **Y** is S and wherein
**R⁶** is not present; and/or
**R⁷** is selected from the group consisting of
   (i) hydrogen;
   (ii) methyl; and
   (iii) 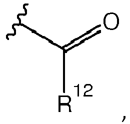 wherein **R¹²** is selected from the group consisting of
      (aa) N; and
      (bb) cyclopropyl or phenyl mono-substituted in para position by cyclopropyl or - (CF₃) or di-substituted in meta position by cyclopropyl or -(CF₃) in each meta position; and/or
**R⁸** is selected from the group consisting of
   (i) hydrogen and methyl; and
   (ii) 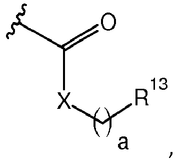 wherein **X** is N, **a** is 1 and **R¹³** is selected from the group consisting of indazolyl, benzimidazolyl and benzodioxolyl connected via position (6) or (5), preferably via position (5) of the indazolyl and benzodioxolyl or position (6) of the benzimidazolyl;
   wherein **R⁸** is preferably (R)- or (S)-configured, more preferably (R)-configured; and/or
**R⁹** is selected from the group consisting of
   (i) hydrogen, methyl and cyclopropyl; and
   (ii) phenyl that is mono-substituted in para position by a substituent selected from the group consisting of H, Cl, F, Br, methyl, -(CF₃) and cyclopropyl;
   wherein **R⁹** is preferably (R)- or (S)-configured, more preferably (S)-configured; and/or
**R¹⁰** is hydrogen; and/or
**R¹¹** is hydrogen or selected from the group consisting of indazolyl, benzimidazolyl and benzodioxolyl connected via position (6) or (5), preferably via position (6) of the indazolyl and benzodioxolyl or position (5) of the benzimidazolyl.

In a further preferred embodiment, the compound for use according to the present invention is a compound of Formula (II), wherein the ring of Formula (II) is aromatic (Formula IIb) 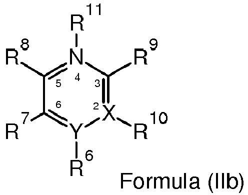 wherein **X** is N, **Y** is C and wherein
**R⁶** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R⁷** and **R⁸** are independently selected from the group consisting of
   (i) hydrogen;
   (ii) methyl; and
   (iii) 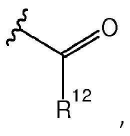 wherein **R¹²** is selected from the group consisting of
      (aa) N; and
      (bb) cyclopropyl and phenyl that is mono-substituted in para position by cyclopropyl or -(CF₃), or di-substituted in meta position by cyclopropyl or - (CF₃) in each meta position;
         and/or
**R⁹** is hydrogen; and/or
**R¹⁰** and/or **R¹¹** are absent.

In a further preferred embodiment, the compound for use according to the present invention is a compound of Formula (II), wherein two double bonds are located between positions (1) and (6) and between positions (4) and (5), respectively (Formula IIc) 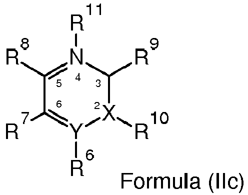 wherein **X** is N, **Y** is C and wherein
**R⁶** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R⁷** and **R⁸** are independently selected from the group consisting of hydrogen and methyl; and/or
**R⁹** is hydrogen; and/or
**R¹⁰** is selected from the group consisting of
   (i) hydrogen;
   (ii) methyl; and
   (iii) cyclopropyl and phenyl that is mono-substituted in para position by a substituent selected from the group consisting of H, Cl, F, Br, methyl, -(CF₃) and cyclopropyl; and/or
**R¹¹** is absent.

In a further preferred embodiment, the compound for use according to the present invention is a compound, wherein
**R¹** is selected from the group consisting of hydrogen, methyl, 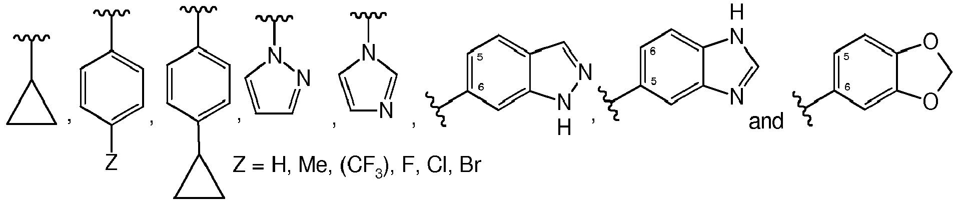 and/or
**R²** is selected from the group consisting of hydrogen, oxo, methyl, 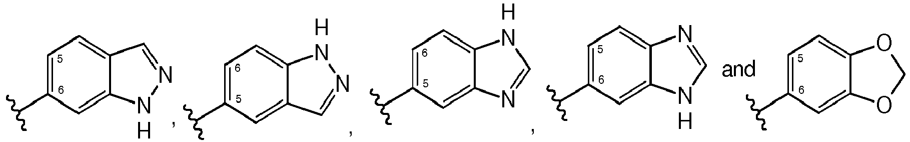 and/or
**R³** is selected from the group consisting of hydrogen, methyl, 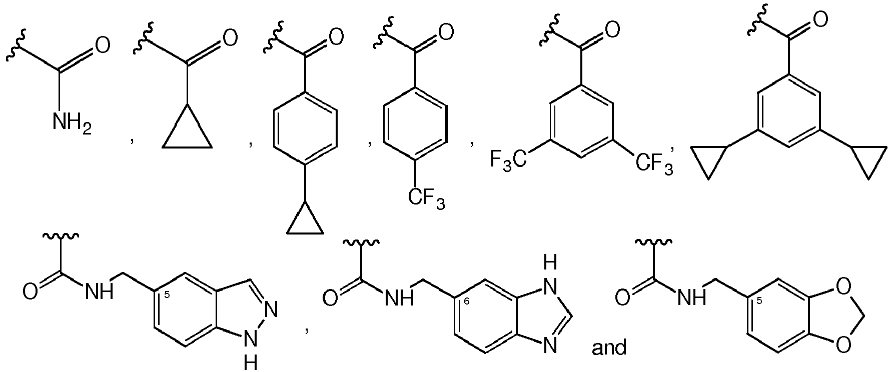 and/or
**R⁴** is selected from the group consisting of hydrogen, hydroxyl, methyl, 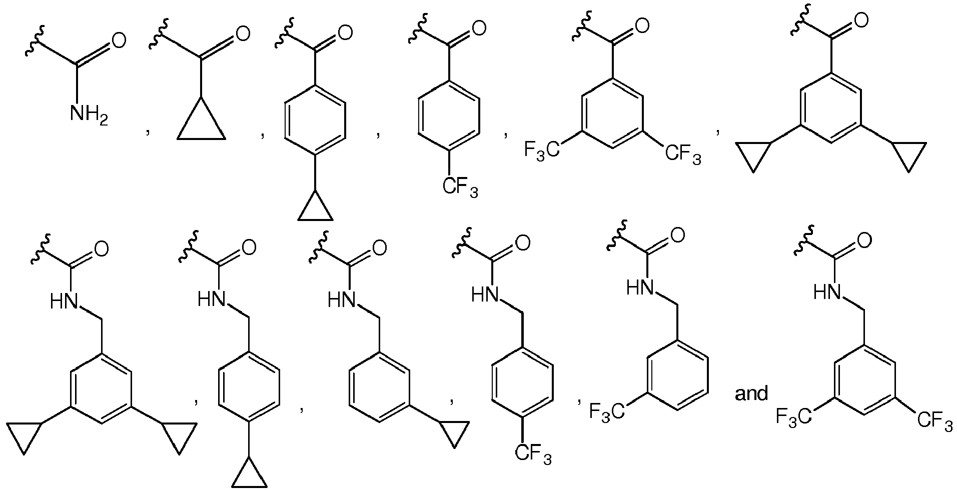 wherein, **R⁴** and/or **R³** and **R⁴** are preferably in cis or trans configuration to each other, more preferably in trans configuration, preferably, **R²** is (R)- or (S)-, **R³** is (R)- or (S)- and/or **R⁴** is (R)- or (S)-configured, more preferably, **R²** is (R)-, **R³** is (R)- and/or **R⁴** is (R)-configured, or **R²** is or (S)-, **R³** is (S)- and/or **R⁴** is (S)-configured, and/or
   **R⁵** and **R⁹** are selected from the group consisting of hydrogen, methyl, cyclopenta-2,4-dien-1-yl, 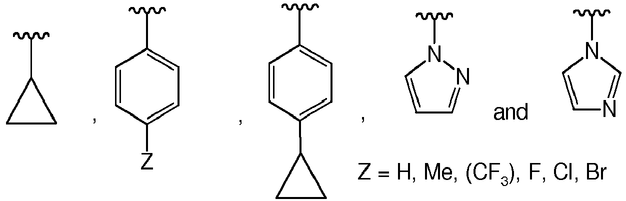 wherein **R⁹** is preferably not cyclopenta-2,4-dien-1-yl,
   wherein, if position (5) of the ring of Formula (I) is sp³-hybridized, **R⁵** is preferably (R)- or (S)-configured, more preferably (R)-configured, and
   wherein **R⁹** is preferably (R)- or (S)-configured, more preferably (R)-configured, and/or
**R⁶** is not present if **Y** is S, and if **Y** is C, **R⁶** is selected from the group consisting of 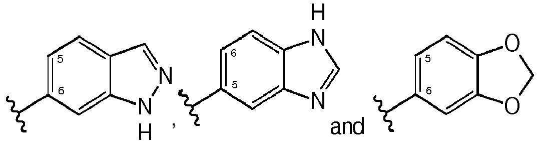 and/or
**R⁷** and **R⁸** are selected from the group consisting of hydrogen, methyl, 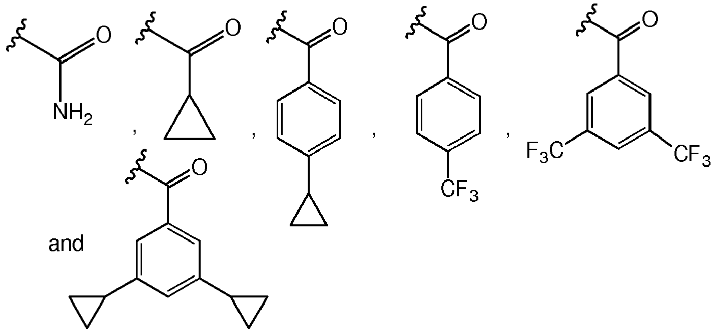 wherein **R⁸** is additionally selected from the group consisting of 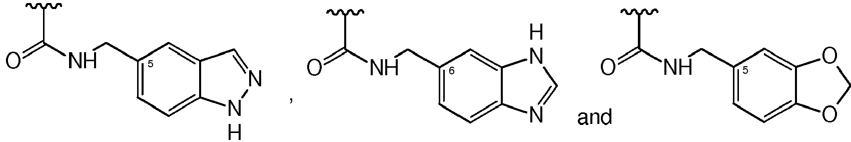 and wherein **R⁸** is preferably (R)- or (S)-configured, more preferably (R)-configured; and/or **R¹⁰** is absent or selected from the group consisting of hydrogen, methyl, 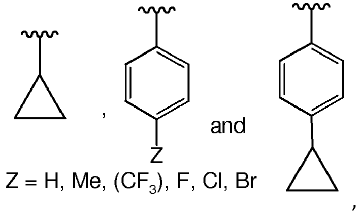 wherein **R¹⁰** is absent if **X** is C and/or if the ring of Formula (II) has a double bond between positions (1) and (2) or between positions (2) and (3) of the ring of Formula (II), and/or
**R¹¹** is absent or selected from the group consisting of hydrogen, 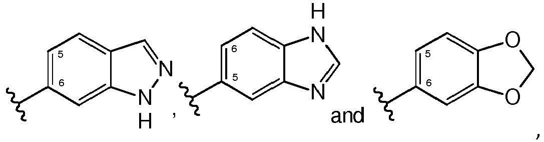 wherein **R¹¹** is absent if the ring of Formula (II) has a double bond between positions (4) and (5) or between positions (3) and (4) of the ring of Formula (II).

In a further preferred embodiment, the compound for use according to the present invention is a compound selected from the group consisting of:
**(i)** a first residue selected from the group consisting of
   1-(1,3-benzodioxol-5-yl)-3-hydroxy-5-oxo-2-methyl-2H-pyrrol-4-yl,
   1-(1,3-benzodioxol-5-yl)-2-cyclopropyl-3-hydroxy-5-oxo-2H-pyrrol-4-yl,
   1-(1,3-benzodioxol-5-yl)-3-hydroxy-5-oxo-2H-pyrrol-4-yl,
   1-(1,3-benzodioxol-5-yl)-2-(cyclopenta-2,4-dien-1-yl)-5-oxo-3-hydroxy-2H-pyrrol-4-yl,
   1-(1,3-benzodioxol-5-yl)-3-hydroxy-5-oxo-2-(pyrazol-1-yl)-2H-pyrrol-4-yl,
   1-(1,3-benzodioxol-5-yl)-3-hydroxy-5-oxo-2-(imidazol-1-yl)-2H-pyrrol-4-yl,
   1-(1,3-benzodioxol-5-yl)-3-hydroxy-5-oxo-2-phenyl-2H-pyrrol-4-yl,
   1-(1,3-benzodioxol-5-yl)-3-hydroxy-5-oxo-2-(p-tolyl)-2H-pyrrol-4-yl,
   1-(1,3-benzodioxol-5-yl)-2-(4-chlorophenyl)-3-hydroxy-5-oxo-2H-pyrrol-4-yl,
   1-(1,3-benzodioxol-5-yl)-2-(4-fluorophenyl)-3-hydroxy-5-oxo-2H-pyrrol-4-yl,
   1-(1,3-benzodioxol-5-yl)-2-(4-bromophenyl)-3-hydroxy-5-oxo-2H-pyrrol-4-yl,
   1-(1,3-benzodioxol-5-yl)-2-(4-cyclopropylphenyl)-3-hydroxy-5-oxo-2H-pyrrol-4-yl,
   1-(1,3-benzodioxol-5-yl)-3-hydroxy-5-oxo-2-[4-(trifluoromethyl)phenyl]-2H-pyrrol-4-yl,
   3-hydroxy-1-(1H-indazol-6-yl)-5-oxo-2-[4-(trifluoromethyl)phenyl]-2H-pyrrol-4-yl,
   3-hydroxy-1-(1H-indazol-6-yl)-5-oxo-2-phenyl-2H-pyrrol-4-yl,
   3-hydroxy-1-(1H-indazol-6-yl)-5-oxo-2-(p-tolyl)-2H-pyrrol-4-yl,
   2-(4-chlorophenyl)-3-hydroxy-1-(1H-indazol-6-yl)-5-oxo-2H-pyrrol-4-yl,
   2-(4-fluorophenyl)-3-hydroxy-1-(1H-indazol-6-yl)-5-oxo-2H-pyrrol-4-yl,
   2-(4-bromophenyl)-3-hydroxy-1-(1H-indazol-6-yl)-5-oxo-2H-pyrrol-4-yl,
   2-(4-cyclopropylphenyl)-3-hydroxy-1-(1H-indazol-6-yl)-5-oxo-2H-pyrrol-4-yl,
   2-cyclopropyl-3-hydroxy-1-(1H-indazol-6-yl)-5-oxo-2H-pyrrol-4-yl,
   3-hydroxy-1-(1H-indazol-6-yl)-5-oxo-2-methyl-2H-pyrrol-4-yl,
   3-hydroxy-1-(1H-indazol-6-yl)-5-oxo-2H-pyrrol-4-yl,
   3-hydroxy-1-(1H-indazol-6-yl)-5-oxo-2-(pyrazol-1-yl)-2H-pyrrol-4-yl,
   2-(cyclopenta-2,4-dien-1-yl)-3-hydroxy-1-(1H-indazo1-6-yl)-5-oxo-2H-pyrrol-4-yl,
   3-hydroxy-2-(imidazol-1-yl)-1-(1H-indazo1-6-yl)-5-oxo-2H-pyrrol-4-yl,
   1-(1H-benzimidazol-5-yl)-3-hydroxy-5-oxo-2-dimethyl-2H-pyrrol-4-yl,
   1-(1H-benzimidazol-5-yl)-2-cyclopropyl-3-hydroxy-5-oxo-2H-pyrrol-4-yl,
   1-(1H-benzimidazol-5-yl)-3-hydroxy-5-oxo-2-(pyrazol-1-yl)-2H-pyrrol-4-yl,
   1-(1H-benzimidazol-5-yl)-3-hydroxy-5-oxo-2H-pyrrol-4-yl,
   1-(1H-benzimidazol-5-yl)-3-hydroxy-5-oxo-2-(imidazol-1-yl)-2H-pyrrol-4-yl,
   1-(1H-benzimidazol-5-yl)-2-(cyclopenta-2,4-dien-1-yl)-3-hydroxy-5-oxo-2H-pyrrol-4-yl,
   1-(1H-benzimidazol-5-yl)-2-(4-fluorophenyl)-3-hydroxy-5-oxo-2H-pyrrol-4-yl,
   1-(1H-benzimidazol-5-yl)-3-hydroxy-5-oxo-2-[4-(trifluoromethyl)phenyl]-2H-pyrrol-4-yl,
   1-(1H-benzimidazol-5-yl)-3-hydroxy-5-oxo-2-phenyl-2H-pyrrol-4-yl,
   1-(1H-benzimidazol-5-yl)-3-hydroxy-5-oxo-2-(p-tolyl)-2H-pyrrol-4-yl,
   1-(1H-benzimidazol-5-yl)-2-(4-chlorophenyl)-3-hydroxy-5-oxo-2H-pyrrol-4-yl,
   1-(1H-benzimidazol-5-yl)-2-(4-bromophenyl)-3-hydroxy-5-oxo-2H-pyrrol-4-yl,
   1-(1H-benzimidazol-5-yl)-2-(4-cyclopropylphenyl)-3-hydroxy-5-oxo-2H-pyrrol-4-yl,
   wherein the numbering of the 2H-pyrrole ring is as follows: 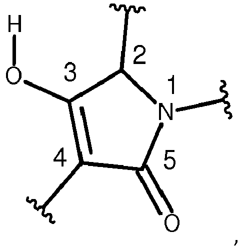
   1-(1,3-benzodioxol-5-ylmethyl)-2-methyl-5-(p-tolyl)pyrrol-3-yl,
   1-(1,3-benzodioxol-5-ylmethyl)-5-(4-chlorophenyl)-2-methyl-pyrrol-3-yl,
   1-(1,3-benzodioxol-5-ylmethyl)-5-(4-fluorophenyl)-2-methyl-pyrrol-3-yl,
   1-(1,3-benzodioxol-5-ylmethyl)-2-methyl-5-[4-(trifluoromethyl)phenyl]pyrrol-3-yl,
   1-(1,3-benzodioxol-5-ylmethyl)-5-(4-bromophenyl)-2-methyl-pyrrol-3-yl,
   1-(1,3-benzodioxol-5-ylmethyl)-5-(4-cyclopropylphenyl)-2-methyl-pyrrol-3-yl,
   1-(1,3-benzodioxol-5-ylmethyl)-2-methyl-5-phenyl-pyrrol-3-yl,
   1-(1,3-benzodioxol-5-ylmethyl)-5-phenyl-pyrrol-3-yl,
   1-(1,3-benzodioxol-5-ylmethyl)-5-(p-tolyl)pyrrol-3-yl,
   1-(1,3-benzodioxol-5-ylmethyl)-5-(4-fluorophenyl)pyrrol-3-yl,
   1-(1,3-benzodioxol-5-ylmethyl)-5-[4-(trifluoromethyl)phenyl]pyrrol-3-yl,
   1-(1,3-benzodioxol-5-ylmethyl)-5-(4-chlorophenyl)pyrrol-3-yl,
   1-(1,3-benzodioxol-5-ylmethyl)-5-(4-bromophenyl)pyrrol-3-yl,
   1-(1,3-benzodioxol-5-ylmethyl)-5-(4-cyclopropylphenyl)pyrrol-3-yl,
   1-(1,3-benzodioxol-5-ylmethyl)-2,5-dimethyl-pyrrol-3-yl,
   1-(1,3-benzodioxol-5-ylmethyl)-2-methyl-pyrrol-3-yl,
   1-(1,3-benzodioxo1-5-ylmethyl)-5-cyclopropyl-2-methyl-pyrro1-3-yl, 1-(1,3-benzodioxol-5-ylmethyl)pyrrol-3-yl,
   1-(1,3-benzodioxol-5-ylmethyl)-5-methyl-pyrrol-3-yl,
   1-(1,3-benzodioxol-5-ylmethyl)-5-cyclopropyl-pyrrol-3-yl,
   1-(1H-indazol-6-ylmethyl)-2-methyl-5-phenyl-pyrrol-3-yl,
   1-(1H-indazol-6-ylmethyl)-2-methyl-5-(p-tolyl)pyrrol-3-yl,
   5-(4-chlorophenyl)-1-(1H-indazol-6-ylmethyl)-2-methyl-pyrrol-3-yl,
   5-(4-fluorophenyl)-1-(1H-indazol-6-ylmethyl)-2-methyl-pyrrol-3-yl,
   1-(1H-indazol-6-ylmethyl)-2-methyl-5-[4-(trifluoromethyl)phenyl]pyrrol-3-yl,
   5-(4-bromophenyl)-1-(1H-indazol-6-ylmethyl)-2-methyl-pyrrol-3-yl,
   5-(4-cyclopropylphenyl)-1-(1H-indazol-6-ylmethyl)-2-methyl-pyrrol-3-yl,
   1-(1H-indazol-6-ylmethyl)-5-phenyl-pyrrol-3-yl,
   1-(1H-indazol-6-ylmethyl)-5-(p-tolyl)pyrrol-3-yl,
   5-(4-fluorophenyl)-1-(1H-indazol-6-ylmethyl)pyrrol-3-yl,
   1-(1H-indazol-6-ylmethyl)-5-[4-(trifluoromethyl)phenyl]pyrrol-3-yl,
   5-(4-chlorophenyl)-1-(1H-indazol-6-ylmethyl)pyrrol-3-yl,
   5-(4-bromophenyl)-1-(1H-indazo1-6-ylmethyl)pyrrol-3-yl,
   5-(4-cyclopropylphenyl)-1-(1H-indazol-6-ylmethyl)pyrrol-3-yl,
   5-cyclopropyl-1-(1H-indazol-6-ylmethyl)-2-methyl-pyrrol-3-yl,
   1-(1H-indazol-6-ylmethyl)-2-methyl-pyrrol-3-yl,
   1-(1H-indazol-6-ylmethyl)-2,5-dimethyl-pyrrol-3-yl,
   1-(1H-indazol-6-ylmethyl)pyrrol-3-yl,
   5-cyclopropyl-1-(1H-indazol-6-ylmethyl)pyrrol-3-yl,
   1-(1H-indazol-6-ylmethyl)-5-methyl-pyrrol-3-yl,
   1-(1H-benzimidazol-5-ylmethyl)-2-methyl-5-phenyl-pyrrol-3-yl,
   1-(1H-benzimidazol-5-ylmethyl)-2-methyl-5-(p-tolyl)pyrrol-3-yl,
   1-(1H-benzimidazol-5-ylmethyl)-2-methyl-5-(p-tolyl)pyrrol-3-yl,
   1-(1H-benzimidazol-5-ylmethyl)-5-(4-fluorophenyl)-2-methyl-pyrrol-3-yl,
   1-(1H-benzimidazol-5-ylmethyl)-5-(4-cyclopropylphenyl)-2-methyl-pyrrol-3-yl,
   1-(1H-benzimidazol-5-ylmethyl)-5-(4-bromophenyl)-2-methyl-pyrrol-3-yl,
   1-(1H-benzimidazol-5-ylmethyl)-2-methyl-5-[4-(trifluoromethyl)phenyl]pyrrol-3-yl,
   1-(1H-benzimidazol-5-ylmethyl)-5-phenyl-pyrrol-3-yl,
   1-(1H-benzimidazol-5-ylmethyl)-5-(p-tolyl)pyrrol-3-yl,
   1-(1H-benzimidazol-5-ylmethyl)-5-[4-(trifluoromethyl)phenyl]pyrrol-3-yl,
   1-(1H-benzimidazol-5-ylmethyl)-5-(4-chlorophenyl)pyrrol-3-yl,
   1-(1H-benzimidazol-5-ylmethyl)-5-(4-fluorophenyl)pyrrol-3-yl,
   1-(1H-benzimidazol-5-ylmethyl)-5-(4-bromophenyl)pyrrol-3-yl,
   1-(1H-benzimidazol-5-ylmethyl)-5-(4-cyclopropylphenyl)pyrrol-3-yl,
   1-(1H-benzimidazol-5-ylmethyl)-2,5-dimethyl-pyrrol-3-yl,
   1-(1H-benzimidazol-5-ylmethyl)-2-methyl-pyrrol-3-yl,
   1-(1H-benzimidazol-5-ylmethyl)-5-cyclopropyl-2-methyl-pyrrol-3-yl,
   1-(1H-benzimidazol-5-ylmethyl)-5-methyl-pyrrol-3-yl,
   1-(1H-benzimidazol-5-ylmethyl)pyrrol-3-yl,
   1-(1H-benzimidazol-5-ylmethyl)-5-cyclopropyl-pyrrol-3-yl,
   1-(1,3-benzodioxol-5-yl)-5-(p-tolyl)pyrrol-3-yl,
   1-(1,3-benzodioxol-5-yl)-5-phenyl-pyrrol-3-yl,
   1-(1,3-benzodioxol-5-yl)-5-(4-fluorophenyl)pyrrol-3-yl,
   1-(1,3-benzodioxol-5-yl)-5-[4-(trifluoromethyl)phenyl]pyrrol-3-yl,
   1-(1,3-benzodioxol-5-yl)-5-(4-bromophenyl)pyrrol-3-yl,
   1-(1,3-benzodioxol-5-yl)-5-(4-chlorophenyl)pyrrol-3-yl,
   1-(1,3-benzodioxol-5-yl)-5-(4-cyclopropylphenyl)pyrrol-3-yl,
   1-(1,3-benzodioxol-5-yl)-2-methyl-5-(p-tolyl)pyrrol-3-yl,
   1-(1,3-benzodioxol-5-yl)-2-methyl-5-phenyl-pyrrol-3-yl,
   1-(1,3-benzodioxol-5-yl)-5-(4-fluorophenyl)-2-methyl-pyrrol-3-yl,
   1-(1,3-benzodioxol-5-yl)-2-methyl-5-[4-(trifluoromethyl)phenyllpyrrol-3-yl,
   1-(1,3-benzodioxol-5-yl)-5-(4-chlorophenyl)-2-methyl-pyrrol-3-yl,
   1-(1,3-benzodioxol-5-yl)-5-(4-bromophenyl)-2-methyl-pyrrol-3-yl,
   1-(1,3-benzodioxol-5-yl)-5-(4-cyclopropylphenyl)-2-methyl-pyrrol-3-yl,
   1-(1,3-benzodioxol-5-yl)-2,5-dimethyl-pyrrol-3-yl,
   1-(1,3-benzodioxol-5-yl)-2-methyl-pyrrol-3-yl,
   1-(1,3-benzodioxol-5-yl)-5-cyclopropyl-2-methyl-pyrrol-3-yl,
   1-(1,3-benzodioxol-5-yl)-5-methyl-pyrrol-3-yl,
   1-(1,3-benzodioxol-5-yl)pyrrol-3-yl,
   1-(1,3-benzodioxol-5-yl)-5-cyclopropyl-pyrrol-3-yl,
   1-(1H-indazol-6-yl)-5-(p-tolyl) pyrrol-3-yl,
   5-(4-chlorophenyl)-l-(1H-indazo1-6-yl)pyrrol-3-yl,
   5-(4-bromophenyl)-1-(1H-indazol-6-yl)pyrrol-3-yl,
   5-(4-fluorophenyl)-1-(1H-indazol-6-yl)pyrrol-3-yl,
   1-(1H-indazol-6-yl)-5-phenyl-pyrrol-3-yl,
   5-(4-cyclopropylphenyl)-1-(1H-indazol-6-yl)pyrrol-3-yl,
   1-(1H-indazol-6-yl)-5-[4-(trifluoromethyl)phenyl]pyrrol-3-yl,
   1-(1H-indazol-6-yl)-2-methyl-5-(p-tolyl)pyrrol-3-yl,
   5-(4-chlorophenyl)-1-(1H-indazol-6-yl)-2-methyl-pyrrol-3-yl,
   5-(4-bromophenyl)-1-(1H-indazo1-6-yl)-2-methyl-pyrrol-3-yl,
   5-(4-fluorophenyl)-1-(1H-indazol-6-yl)-2-methyl-pyrrol-3-yl,
   1-(1H-indazol-6-yl)-2-methyl-5-phenyl-pyrrol-3-yl,
   5-(4-cyclopropylphenyl)-1-(1H-indazol-6-yl)-2-methyl-pyrrol-3-yl,
   1-(1H-indazol-6-yl)-2-methyl-5-[4-(trifluoromethyl)phenyl]pyrrol-3-yl,
   1-(1H-indazol-6-yl)-2,5-dimethyl-pyrrol-3-yl,
   1-(1H-indazol-6-yl)-2-methyl-pyrrol-3-yl,
   5-cyclopropyl-1-(1H-indazol-6-yl)-2-methyl-pyrrol-3-yl,
   1-(1H-indazol-6-yl)-5-methyl-pyrrol-3-yl,
   1-(1H-indazol-6-yl)pyrrol-3-yl,
   5-cyclopropyl-1-(1H-indazol-6-yl)pyrrol-3-yl,
   1-(1H-benzimidazol-5-yl)-5-(p-tolyl)pyrrol-3-yl,
   1-(1H-benzimidazoI-5-yl)-5-(4-chlorophenyl)pyrrol-3-yl,
   1-(1H-benzimidazol-5-yl)-5-(4-bromophenyl)pyrrol-3-yl,
   1-(1H-benzimidazol-5-yl)-5-(4-fluorophenyl)pyrrol-3-yl,
   1-(1H-benzimidazol-5-yl)-5-phenyl-pyrrol-3-yl,
   1-(1H-benzimidazol-5-yl)-5-(4-cyclopropylphenyl)pyrrol-3-yl,
   1-(1H-benzimidazol-5-yl)-5-[4-(trifluoromethyl)phenyl]pyrrol-3-yl,
   1-(1H-benzimidazol-5-yl)-2-methyl-5-(p-tolyl)pyrrol-3-yl,
   1-(1H-benzimidazol-5-yl)-5-(4-chlorophenyl)-2-methyl-pyrrol-3-yl,
   1-(1H-benzimidazol-5-yl)-5-(4-bromophenyl)-2-methyl-pyrrol-3-yl,
   1-(1H-benzimidazol-5-yl)-5-(4-fluorophenyl)-2-methyl-pyrrol-3-yl,
   1-(1H-benzimidazol-5-yl)-2-methyl-5-phenyl-pyrrol-3-yl,
   1-(1H-benzimidazol-5-yl)-5-(4-cyclopropylphenyl)-2-methyl-pyrrol-3-yl,
   1-(1H-benzimidazol-5-yl)-2-methyl-5-[4-(trifluoromethyl)phenyl]pyrrol-3-yl,
   1-(1H-benzimidazol-5-yl)-2,5-dimethyl-pyrrol-3-yl,
   1-(1H-benzimidazol-5-yl)-2-methyl-pyrrol-3-yl,
   1-(1H-benzimidazol-5-yl)-5-cyclopropyl-2-methyl-pyrrol-3-yl,
   1-(1H-benzimidazol-5-yl)-5-methyl-pyrrol-3-yl,
   1-(1H-benzimidazol-5-yl)pyrrol-3-yl,
   1-(1H-benzimidazol-5-yl)-5-cyclopropyl-pyrrol-3-yl,
   wherein the numbering of the pyrrole ring is as follows: 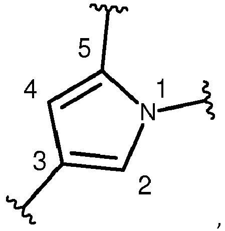
   (3R)-3-(1,3-benzodioxol-5-ylmethylcarbamoyl)-5-phenyl-thiomorpholin-2-yl,
   (3R)-3-(1,3-benzodioxol-5-ylmethylcarbamoyl)-5-(p-tolyl)thiomorpholin-2-yl,
   (3R)-3-(1,3-benzodioxol-5-ylmethylcarbamoyl)-5-(4-fluorophenyl)thiomorpholin-2-yl,
   (3R)-3-(1,3-benzodioxol-5-ylmethylcarbamoyl)-5-(4-bromophenyl)thiomorpholin-2-yl,
   (3R)-3-(1,3-benzodioxol-5-ylmethylcarbamoyl)-5-(4-chlorophenyl)thiomorpholin-2-yl,
   (3R)-3-(1,3-benzodioxol-5-ylmethylcarbamoyl)-5-(4-cyclopropylphenyl)thiomorpholin-2-yl,
   (3R)-3-(1,3-benzodioxol-5-ylmethylcarbamoyl)-5-[4-(trifluoromethyl)phenyl]thio-morpholin-2-yl,
   (3R)-3-(1H-indazol-5-ylmethylcarbamoyl)-5-phenyl-thiomorpholin-2-yl,
   (3R)-3-(1H-indazol-5-ylmethylcarbamoyl)-5-(p-tolyl)thiomorpholin-2-yl,
   (3R)-5-(4-fluorophenyl)-3-(1H-indazol-5-ylmethylcarbamoyl)thiomorpholin-2-yl,
   (3R)-5-(4-bromophenyl)-3-(1H-indazol-5-ylmethylcarbamoyl)thiomorpholin-2-yl,
   (3R)-5-(4-chlorophenyl)-3-(1H-indazol-5-ylmethylcarbamoyl)thiomorpholin-2-yl,
   (3R)-5-(4-cyclopropylphenyl)-3-(1H-indazol-5-ylmethylcarbamoyl)thiomorpholin-2-yl,
   (3R)-3-(1H-indazol-5-ylmethylcarbamoyl)-5-[4-(trifluoromethyl)phenyl]thiomorpholin-2-yl,
   (3R)-3-(3H-benzimidazol-5-ylmethylcarbamoyl)-5-phenyl-thiomorpholin-2-yl,
   (3R)-3-(3H-benzimidazol-5-ylmethylcarbamoyl)-5-(p-tolyl)thiomorpholin-2-yl,
   (3R)-3-(3H-benzimidazol-5-ylmethylcarbamoyl)-5-(4-fluorophenyl)thiomorpholin-2-yl,
   (3R)-3-(3H-benzimidazol-5-ylmethylcarbamoyl)-5-[4-(trifluoromethyl)phenyl]thio-morpholin-2-yl,
   (3R)-3-(3H-benzimidazol-5-ylmethylcarbamoyl)-5-(4-bromophenyl)thiomorpholin-2-yl,
   (3R)-3-(3H-benzimidazol-5-ylmethylcarbamoyl)-5-(4-chlorophenyl)thiomorpholin-2-yl,
   (3R)-3-(3H-benzimidazol-5-ylmethylcarbamoyl)-5-(4-cyclopropylphenyl)thio-morpholin-2-yl,
   4-(1,3-benzodioxol-5-yl)-5-(4-fluorophenyl)thiomorpholin-2-yl,
   4-(1,3-benzodioxol-5-yl)-5-(4-bromophenyl)thiomorpholin-2-yl,
   4-(1,3-benzodioxol-5-yl)-5-(4-chlorophenyl)thiomorpholin-2-yl,
   4-(1,3-benzodioxol-5-yl)-5-methyl-thiomorpholin-2-yl,
   4-(1,3-benzodioxol-5-yl)-5-cyclopropyl-thiomorpholin-2-yl,
   4-(1,3-benzodioxol-5-yl)-5-(4-cyclopropylphenyl)thiomorpholin-2-yl,
   4-(1,3-benzodioxol-5-yl)-5-(p-tolyl)thiomorpholin-2-yl,
   4-(1,3-benzodioxol-5-yl)-5-phenyl-thiomorpholin-2-yl,
   4-(1,3-benzodioxol-5-yl)-5-[4-(trifluoromethyl)phenyl]thiomorpholin-2-yl,
   4-(1,3-benzodioxol-5-yl)thiomorpholin-2-yl,
   4-(1,3-benzodioxol-5-yl)-5-(4-fluorophenyl)-3-methyl-thiomorpholin-2-yl,
   4-(1,3-benzodioxol-5-yl)-5-(4-bromophenyl)-3-methyl-thiomorpholin-2-yl,
   4-(1,3-benzodioxol-5-yl)-5-(4-chlorophenyl)-3-methyl-thiomorpholin-2-yl,
   4-(1,3-benzodioxol-5-yl)-3,5-dimethyl-thiomorpholin-2-yl,
   4-(1,3-benzodioxol-5-yl)-5-cyclopropyl-3-methyl-thiomorpholin-2-yl,
   4-(1,3-benzodioxol-5-yl)-5-(4-cyclopropylphenyl)-3-methyl-thiomorpholin-2-yl,
   4-(1,3-benzodioxol-5-yl)-3-methyl-5-(p-tolyl)thiomorpholin-2-yl,
   4-(1,3-benzodioxol-5-yl)-3-methyl-5-phenyl-thiomorpholin-2-yl,
   4-(1,3-benzodioxol-5-yl)-3-methyl-5-[4-(trifluoromethyl)phenyl]thiomorpholin-2-yl,
   4-(1,3-benzodioxol-5-yl)-3-methyl-thiomorpholin-2-yl,
   5-(4-fluorophenyl)-4-(1H-indazol-6-yl)thiomorpholin-2-yl,
   5-(4-bromophenyl)-4-(1H-indazol-6-yl)thiomorpholin-2-yl,
   5-(4-chlorophenyl)-4-(1H-indazol-6-yl)thiomorpholin-2-yl,
   4-(1H-indazol-6-yl)-5-methyl-thiomorpholin-2-yl,
   5-cyclopropyl-4-(1H-indazol-6-yl)thiomorpholin-2-yl,
   5-(4-cyclopropylphenyl)-4-(1H-indazol-6-yl)thiomorpholin-2-yl,
   4-(1H-indazol-6-yl)-5-(p-tolyl)thiomorpholin-2-yl,
   4-(1H-indazol-6-yl)-5-phenyl-thiomorpholin-2-yl,
   4-(1H-indazol-6-yl)-5-[4-(trifluoromethyl)phenyl]thiomorpholin-2-yl,
   4-(1H-indazol-6-yl)thiomorpholin-2-yl,
   5-(4-fluorophenyl)-4-(1H-indazol-6-yl)-3-methyl-thiomorpholin-2-yl,
   5-(4-bromophenyl)-4-(1H-indazol-6-yl)-3-methyl-thiomorpholin-2-yl,
   5-(4-chlorophenyl)-4-(1H-indazol-6-yl)-3-methyl-thiomorpholin-2-yl,
   4-(1H-indazol-6-yl)-3,5-dimethyl-thiomorpholin-2-yl,
   5-(4-cyclopropylphenyl)-4-(1H-indazol-6-yl)-3-methyl-thiomorpholin-2-yl,
   4-(1H-indazol-6-yl)-3-methyl-5-(p-tolyl)thiomorpholin-2-yl,
   4-(1H-indazol-6-yl)-3-methyl-5-phenyl-thiomorpholin-2-yl,
   5-cyclopropyl-4-(1H-indazol-6-yl)-3-methyl-thiomorpholin-2-yl,
   4-(1H-indazol-6-yl)-3-methyl-thiomorpholin-2-yl,
   4-(1H-indazol-6-yl)-3-methyl-5-[4-(trifluoromethyl)phenyl]thiomorpholin-2-yl,
   4-(1H-benzimidazol-5-yl)-5-(4-fluorophenyl)thiomorpholin-2-yl,
   4-(1H-benzimidazol-5-yl)-5-(4-bromophenyl)thiomorpholin-2-yl,
   4-(1H-benzimidazol-5-yl)-5-(4-chlorophenyl)thiomorpholin-2-yl,
   4-(1H-benzimidazol-5-yl)-5-methyl-thiomorpholin-2-yl,
   4-(1H-benzimidazol-5-yl)-5-cyclopropyl-thiomorpholin-2-yl,
   4-(1H-benzimidazol-5-yl)-5-(4-cyclopropylphenyl)thiomorpholin-2-yl,
   4-(1H-benzimidazol-5-yl)-5-(p-tolyl)thiomorpholin-2-yl,
   4-(1H-benzimidazol-5-yl)-5-phenyl-thiomorpholin-2-yl,
   4-(1H-benzimidazol-5-yl)-5-[4-(trifluoromethyl)phenyl]thiomorpholin-2-yl,
   4-(1H-benzimidazol-5-yl)thiomorpholin-2-yl,
   4-(1H-benzimidazol-5-yl)-5-(4-fluorophenyl)-3-methyl-thiomorpholin-2-yl,
   4-(1H-benzimidazol-5-yl)-5-(4-bromophenyl)-3-methyl-thiomorpholin-2-yl,
   4-(1H-benzimidazol-5-yl)-5-(4-chlorophenyl)-3-methyl-thiomorpholin-2-yl,
   4-(1H-benzimidazol-5-yl)-3,5-dimethyl-thiomorpholin-2-yl,
   4-(1H-benzimidazol-5-yl)-5-cyclopropyl-3-methyl-thiomorpholin-2-yl,
   4-(1H-benzimidazol-5-yl)-5-(4-cyclopropylphenyl)-3-methyl-thiomorpholin-2-yl,
   4-(1H-benzimidazol-5-yl)-3-methyl-5-(p-tolyl)thiomorpholin-2-yl,
   4-(1H-benzimidazol-5-yl)-3-methyl-5-phenyl-thiomorpholin-2-yl,
   4-(1H-benzimidazol-5-yl)-3-methyl-5-[4-(trifluoromethyl)phenyl]thiomorpholin-2-yl,
   4-(1H-benzimidazol-5-yl)-3-methyl-thiomorpholin-2-yl,
   wherein the numbering of the thiomorpholine ring is as follow: 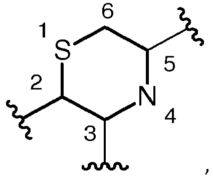 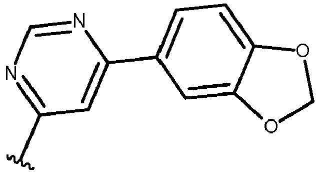
   6-(1,3-benzodioxol-5-yl)pyrimidin-4-yl, 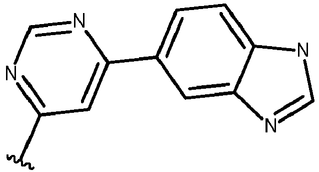
   6-(1H-benzimidazol-5-yl)pyrimidin-4-yl, 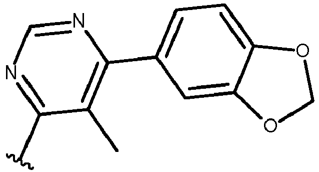
   6-(1,3-benzodioxol-5-yl)-5-methyl-pyrimidin-4-yl, 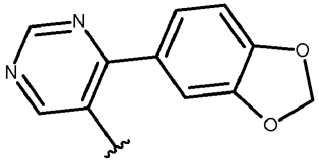
   4-(1,3-benzodioxol-5-yl)pyrimidin-5-yl, 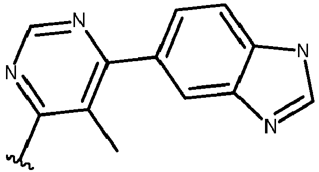
   6-(1H-benzimidazol-5-yl)-5-methyl-pyrimidin-4-yl, 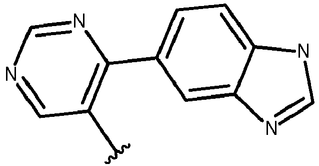
   4-(1H-benzimidazol-5-yl)pyrimidin-5-yl, 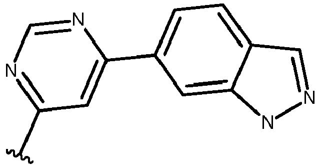
   6-(1H-indazol-6-yl)pyrimidin-4-yl, 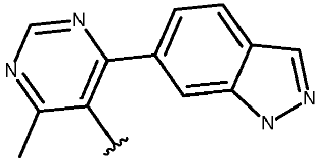
   6-(1H-indazol-6-yl)-4-methyl-pyrimidin-5-yl, and 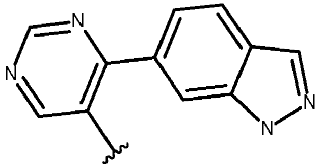
   6-(1H-indazol-6-yl)pyrimidin-5-yl;
   covalently bound to a second residue selected from the group consisting of hydrogen, methyl, 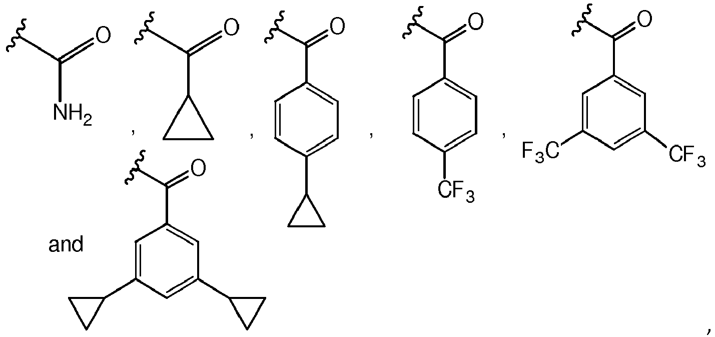 and
**(ii)** a first residue selected from the group consisting of
   6-(1,3-benzodioxol-5-yl)-2H-pyrimidin-1-yl,
   6-(1,3-benzodioxol-5-yl)-4-methyl-2H-pyrimidin-1-yl,
   6-(1H-benzimidazol-5-yl)-2H-pyrimidin-1-yl,
   6-(1H-benzimidazol-5-yl)-4-methyl-2H-pyrimidin-1-yl,
   6-(1,3-benzodioxol-5-yl)-4,5-dimethyl-2H-pyrimidin-1-yl,
   6-(1,3-benzodioxol-5-yl)-5-methyl-2H-pyrimidin-1-yl,
   6-(1H-benzimidazol-5-yl)-4,5-dimethyl-2H-pyrimidin-1-yl,
   6-(1H-indazol-6-yl)-2H-pyrimidin-1-yl,
   6-(1H-indazol-6-yl)-4-methyl-2H-pyrimidin-1-yl,
   6-(1H-benzimidazol-5-yl)-5-methyl-2H-pyrimidin-1-yl,
   6-(1H-indazol-6-yl)-4,5-dimethyl-2H-pyrimidin-1-yl,
   6-(1H-indazol-6-yl)-5-methyl-2H-pyrimidin-1-yl,
   wherein the 2H-pyrimidine ring is numbered as follows: 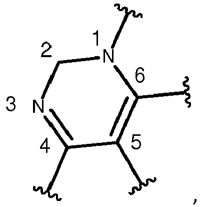
   (4R)-3-(1,3-benzodioxol-5-ylmethylcarbamoyl)-4-[[3-(trifluoromethyl)phenyl]methylcarbamoyl]pyrrolidin-1-yl,
   (4R)-3-(1,3-benzodioxol-5-ylmethylcarbamoyl)-4-[[3,5-bis(trifluoromethyl)phenyl]-methylcarbamoyl]pyrrolidin-1-yl,
   (4R)-3-(1,3-benzodioxol-5-ylmethylcarbamoyl)-4-[[4-(trifluoromethyl)phenyl]methylcarbamoyl]pyrrolidin-1-yl,
   (4R)-3-(1,3-benzodioxol-5-ylmethylcarbamoyl)-4-[[3-(cyclopropylphenyl)phenyl]-methylcarbamoyl]pyrrolidin-1-yl,
   (4R)-3-(1,3-benzodioxol-5-ylmethylcarbamoyl)-4-[[3,5-bis(cyclopropylphenyl)phenyl]-methylcarbamoyl]pyrrolidin-1-yl,
   (4R)-3-(1,3-benzodioxol-5-ylmethylcarbamoyl)-4-[[4-(cyclopropylphenyl)phenyl]-methylcarbamoyl]pyrrolidin-1-yl,
   (4R)-3-(1H-indazol-5-ylmethylcarbamoyl)-4-[[3-(trifluoromethyl)phenyl]methylcarbamoyl]pyrrolidin-1-yl,
   (4R)-3-(1H-indazol-5-ylmethylcarbamoyl)-4-[[3,5-bis(trifluoromethyl)phenyl]methylcarbamoyl]pyrrolidin-1-yl,
   (4R)-3-(1H-indazol-5-ylmethylcarbamoyl)-4-[[4-(trifluoromethyl)phenyl]methylcarbamoyl]pyrrolidin-1-yl,
   (4R)-3-(1H-indazol-5-ylmethylcarbamoyl)-4-[[3-(cyclopropyl)phenyl]methylcarbamoyl]pyrrolidin-1-yl,
   (4R)-3-(1H-indazol-5-ylmethylcarbamoyl)-4-[[3,5-bis(cyclopropyl)phenyl]methylcarbamoyl]pyrrolidin-1-yl,
   (4R)-3-(1H-indazol-5-ylmethylcarbamoyl)-4-[[4-(cyclopropyl)phenyl]methylcarbamoyl]pyrrolidin-1-yl,
   (4R)-3-(3H-benzimidazol-5-ylmethylcarbamoyl)-4-[[3-(trifluoromethyl)phenyl]methylcarbamoyl]pyrrolidin-1-yl,
   (4R)-3-(3H-benzimidazol-5-ylmethylcarbamoyl)-4-[[3,5-bis(trifluoromethyl)phenyl]-methylcarbamoyl]pyrrolidin-1-yl,
   (4R)-3-(3H-benzimidazol-5-ylmethylcarbamoyl)-4-[[4-(trifluoromethyl)phenyl]methylcarbamoyl]pyrrolidin-1-yl,
   (4R)-3-(3H-benzimidazol-5-ylmethylcarbamoyl)-4-[[3-(cyclopropyl)phenyl]methylcarbamoyl]pyrrolidin-1-yl,
   (4R)-3-(3H-benzimidazol-5-ylmethylcarbamoyl)-4-[[3,5-bis(cyclopropyl)phenyl]methylcarbamoyl]pyrrolidin-1-yl,
   (4R)-3-(3H-benzimidazol-5-ylmethylcarbamoyl)-4-[[4-(cyclopropyl)phenyl]methylcarbamoyl]pyrrolidin-1-yl,
   (4R)-2-(1,3-benzodioxol-5-yl)-4-[[3-(trifluoromethyl)phenyl]methyl-carbamoyl]pyrrolidin-1-yl, (4R)-2-(1,3-benzodioxol-5-yl)-4-[[3,5-bis(trifluoromethyl)phenyl]methylcarbamoyl]-pyrrolidin-1-yl,
   (4R)-2-(1,3-benzodioxol-5-yl)-4-[[4-(trifluoromethyl)phenyl]methyl-carbamoyl]pyrrolidin-1-yl,
   (4R)-2-(1,3-benzodioxol-5-yl)-4-methyl-pyrrolidin-1-yl,
   (4R)-2-(1,3-benzodioxol-5-yl)-4-[[3-(cyclopropyl)phenyl]methylcarbamoyl]pyrrolidin-1-yl,
   (4R)-2-(1,3-benzodioxol-5-yl)-4-[[3,5-bis(cyclopropyl)phenyl]methyl-carbamoyl]pyrrolidin-1-yl,
   (4R)-2-(1,3-benzodioxol-5-yl)-4-[[4-(cyclopropyl)phenyl]methylcarbamoyl]pyrrolidin-1-yl,
   (4R)-2-(1H-indazol-5-yl)-4-[[3-(trifluoromethyl)phenyl]methyl-carbamoyl]pyrrolidin-1-yl,
   (4R)-2-(1H-indazol-5-yl)-4-[[3,5-bis(trifluoromethyl)phenyl]methyl-carbamoyl]pyrrolidin-1-yl,
   (4R)-2-(1H-indazol-5-yl)-4-[[4-(trifluoromethyl)phenyl]methyl-carbamoyl]pyrrolidin-1-yl,
   (4R)-2-(1H-indazol-5-yl)-3-methyl-pyrrolidin-1-yl,
   (4R)-2-(1H-indazol-5-yl)-4-[[3-(cyclopropyl)phenyl]methylcarbamoyl]pyrrolidin-1-yl,
   (4R)-2-(1H-indazol-5-yl)-4-[[3,5-bis(cyclopropyl)phenyl]methyl-carbamoyl]pyrrolidin-1-yl,
   (4R)-2-(1H-indazol-5-yl)-4-[[4-(cyclopropyl)phenyl]methylcarbamoyl]pyrrolidin-1-yl,
   (4R)-2-(3H-benzimidazol-5-yl)-4-[[3-(trifluoromethyl)phenyl]methyl-carbamoyl]pyrrolidin-1-yl,
   (4R)-2-(3H-benzimidazol-5-yl)-4-[[3,5-bis(trifluoromethyl)phenyl]methylcarbamoyl]-pyrrolidin-1-yl,
   (4R)-2-(3H-benzimidazol-5-yl)-4-[[4-(trifluoromethyl)phenyl]methyl-carbamoyl]pyrrolidin-1-yl,
   (4R)-2-(3H-benzimidazol-5-yl)-3-methyl-pyrrolidin-1-yl,
   (4R)-2-(3H-benzimidazol-5-yl)-4-[[3-(cyclopropyl)phenyl]methylcarbamoyl]pyrrolidin-1-yl,
   (4R)-2-(3H-benzimidazol-5-yl)-4-[[3,5-bis(cyclopropyl)phenyl]methyl-carbamoyl]pyrrolidin-1-yl,
   (4R)-2-(3H-benzimidazol-5-yl)-4-[[4-(cyclopropyl)phenyl]methylcarbamoyl]pyrrolidin-1-yl,
   (4R)-2-(1,3-benzodioxol-5-yl)-3-methyl-4-[[3-(trifluoromethyl)phenyl]methylcarbamoyl]pyrrolidin-1-yl,
   (4R)-2-(1,3-benzodioxol-5-yl)-3-methyl-4-[[3,5-bis(trifluoromethyl)phenyl]methylcarbamoyl]pyrrolidin-1-yl,
   (4R)-2-(1,3-benzodioxol-5-yl)-3-methyl-4-[[4-(trifluoromethyl)phenyl]methylcarbamoyl]pyrrolidin-1-yl],
   (4R)-2-(1,3-benzodioxol-5-yl)-3-methyl-pyrrolidin-1-yl,
   (4R)-2-(1,3-benzodioxol-5-yl)-3-methyl-4-[[3-(cyclopropyl) phenyl]methyl-carbamoyl]pyrrolidin-1-yl,
   (4R)-2-(1,3-benzodioxol-5-yl)-3-methyl-4-[[3,5-bis(cyclopropyl) phenyl]methylcarbamoyl]pyrrolidin-1-yl,
   (4R)-2-(1,3-benzodioxol-5-yl)-3-methyl-4-[[4-(cyclopropyl)phenyl]methyl-carbamoyl]pyrrolidin-1-yl,
   (4R)-2-(1H-indazol-6-yl)-3-methyl-4-[[3-(trifluoromethyl)phenyl]methyl-carbamoyl]pyrrolidin-1-yl,
   (4R)-2-(1H-indazol-6-yl)-3-methyl-4-[[3,5-bis(trifluoromethyl)phenyl]methylcarbamoyl]pyrrolidin-1-yl,
   (4R)-2-(1H-indazol-6-yl)-3-methyl-4-[[4-(trifluoromethyl)phenyl]methyl-carbamoyl]pyrrolidin-1-yl,
   (4R)-2-(1H-indazol-6-yl)-3-methyl-pyrrolidin-1-yl
   (4R)-2-(1H-indazol-6-yl)-3-methyl-4-[[3-(cyclopropyl)phenyl]methyl-carbamoyl]pyrrolidin-1-yl,
   (4R)-2-(1H-indazol-6-yl)-3-methyl-4-[[3,5-bis(cyclopropyl)phenyl]methyl-carbamoyl]pyrrolidin-1-yl,
   (4R)-2-(1H-indazol-6-yl)-3-methyl-4-[[4-(cyclopropyl)phenyl]methyl-carbamoyl]pyrrolidin-1-yl,
   (4R)-2-(1H-benzimidazol-5-yl)-4-[[3-(trifluoromethyl)phenyl]methyl-carbamoyl]pyrrolidin-1-yl,
   (4R)-2-(1H-benzimidazol-5-yl)-4-[[3,5-bis(trifluoromethyl)phenyl]methyl-carbamoyl]pyrrolidin-1-yl,
   (4R)-2-(1H-benzimidazol-5-yl)-4-[[4-(trifluoromethyl)phenyl]methyl-carbamoyl]pyrrolidin-1-yl, (4R)-2-(1H-benzimidazol-5-yl)-3-methyl-pyrrolidin-1-yl,
   (4R)-2-(1H-benzimidazol-5-yl)-4-[[3-(cyclopropyl)phenyl]methylcarbamoyl]pyrrolidin-1-yl,
   (4R)-2-(1H-benzimidazol-5-yl)-4-[[3,5-bis(cyclopropyl)phenyl]methyl-carbamoyl]pyrrolidin-1-yl, and
   (4R)-2-(1H-benzimidazol-5-yl)-4-[[4-(cyclopropyl)phenyl]methylcarbamoyl]pyrrolidin-1-yl, wherein the pyrrolidine ring is numbered as follows: 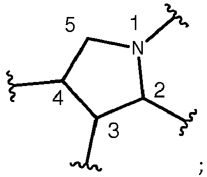
   covalently bound to a second residue selected from the group consisting of hydrogen, methyl, 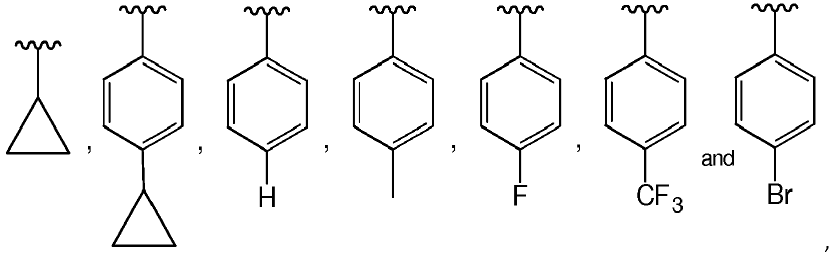
   wherein the first residue is covalently bound to the second residue at the -yl position of the first residue;
   preferably a compound selected from the group consisting of 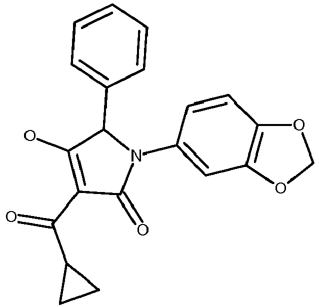
   1-(1,3-benzodioxol-5-yl)-4-(cyclopropanecarbonyl)-3-hydroxy-2-phenyl-2H-pyrrol-5-one; 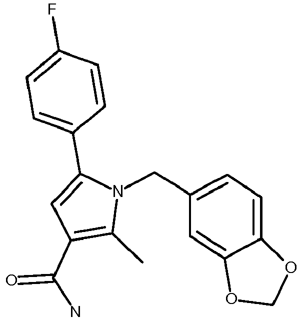
   1-(1,3-benzodioxol-5-ylmethyl)-5-(4-fluorophenyl)-2-methyl-pyrrole-3-carboxamide; 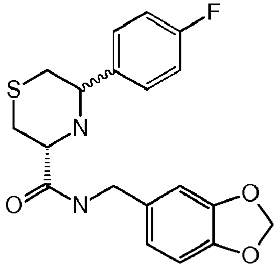
   (3R)-N-(1,3-benzodioxol-5-ylmethyl)-5-(4-fluorophenyl)thiomorpholine-3-carboxamide; 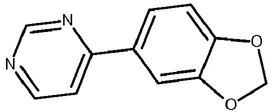
   4-(1,3-benzodioxol-5-yl)pyrimidine; 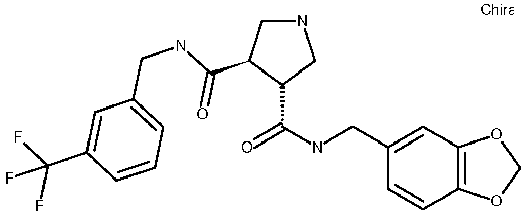
   (4R)-N3-(1,3-benzodioxol-5-ylmethyl)-N4-[[3-(trifluoromethyl)phenyl]methyl]pyrrolidine-3,4-dicarboxamide; 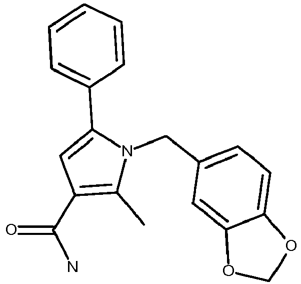
   1-(1,3-benzodioxol-5-ylmethyl)-2-methyl-5-phenyl-pyrrole-3-carboxamide; 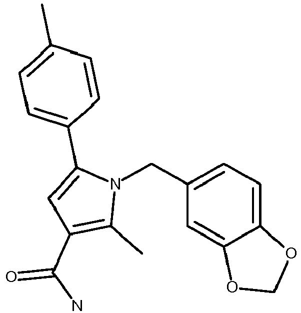
   1-(1,3-benzodioxol-5-ylmethyl)-2-methyl-5-(p-tolyl)pyrrole-3-carboxamide; and 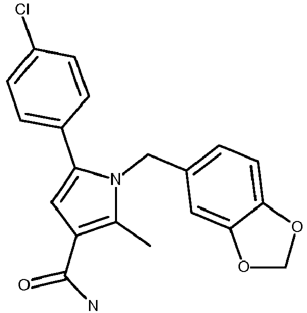
   1-(1,3-benzodioxol-5-ylmethyl)-5-(4-chlorophenyl)-2-methyl-pyrrole-3-carboxamide.

In a further aspect, the present invention is directed to a compound selected from the group consisting of 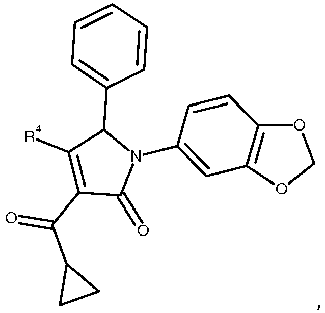 wherein **R⁴** is selected from the group consisting of hydroxyl, -O-R¹⁴, and -O-C(=O)-**R¹⁴**, wherein **R¹⁴** is selected from the group consisting of
(aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
(bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in para position by (C₃)carbocycle or-(CF₃) or di-substituted in meta position by (C₃)carbocycle or -(CF₃); and
(cc) substituted or non-substituted aromatic or non-aromatic, preferably aromatic, (C₃₋₆)heterocycle having 1 to 3 heteroatoms each independently selected from N, O and S;
preferably a compound selected from the group consisting of 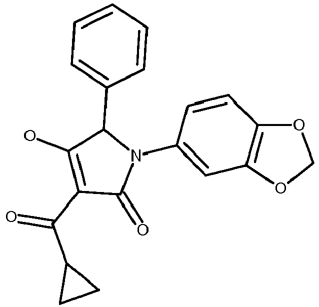
1-(1,3-benzodioxol-5-yl)-4-(cyclopropanecarbonyl)-3-hydroxy-2-phenyl-2H-pyrrol-5-one; 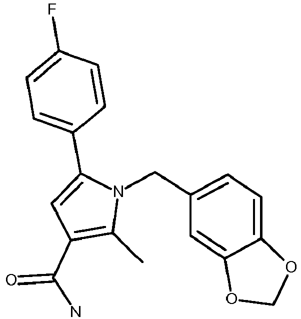
1-(1,3-benzodioxol-5-ylmethyl)-5-(4-fluorophenyl)-2-methyl-pyrrole-3-carboxamide; 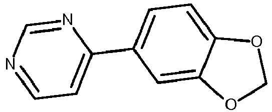
4-(1,3-benzodioxol-5-yl)pyrimidine; and 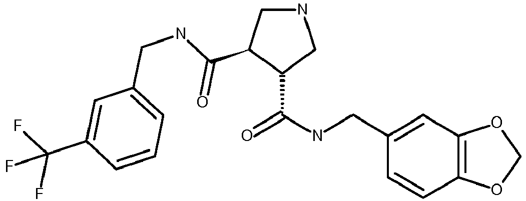
(4R)-N3-(1,3-benzodioxol-5-ylmethyl)-N4-[[3-(trifluoromethyl)phenyl]methyl]pyrrolidine-3,4-dicarboxamide for use according to the present invention.

The compounds described herein are generally named by using the nomenclature that was computed based on the structural drawings by the software ACD/Chemsketch 2015 provided by Advanced Chemistry Development, Inc., Canada and BIOVIA Draw 2016 provided by BIOVIA, USA. For each molecule described herein, the description provides a structural formula that unambiguously numbers the residues of the rings of Formula I and II for the purposes of nomenclature. It is further noted that the structural formulae are binding and not the computed chemical names; in other words, if the name and the structural formula contradict each other, the structural formula prevails.

For compounds having asymmetric centers, it is understood that, unless otherwise specified, all of the optical isomers and mixtures thereof are encompassed. Each stereogenic carbon may be in the (R)- or (S)- configuration or a combination of configurations if not indicated differently. Also, compounds with two or more asymmetric elements can be present as mixtures of diastereomers. Furthermore, the compounds of the present invention preferably have a diastereomeric purity of at least 50%, preferably at least 60%, 70%, 80%, 85%, more preferably at least 90%, 95%, 96%, 97%, most preferably at least 98%, 99% or 100%. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms.

For example, the compound depicted as follows: 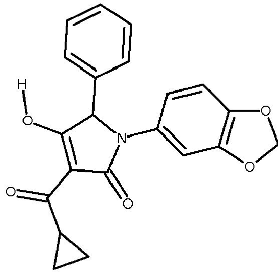 encompasses the tautomeric form:

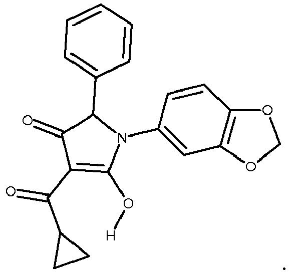

Recited compounds are further intended to encompass compounds in which one or more atoms are replaced with an isotope, i.e., an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, preferably at least 90%, 95%, or 98%. Some embodiments of the compounds have an enantiomeric excess of at least 99%. It will be apparent that single enantiomers (optically active forms) can be obtained by asymmetric synthesis, synthesis from optically pure precursors, biosynthesis, e.g. using modified CYP102 (CYP BM-3) or by resolution of the racemates, e.g. enzymatic resolution or resolution by conventional methods such as crystallization in the presence of a resolving agent, or chromatography, using, for example, a chiral HPLC column.

As used herein, a "substituent" or "residue" or "R", refers to a molecular moiety that is covalently bound to an atom within a molecule of interest. For example, a "substituent", "R" or "residue" may be a moiety such as a halogen, alkyl group, haloalkyl group or any other substituent described herein that is covalently bonded to an atom, preferably a carbon or nitrogen atom, that that forms part of a molecule of interest. The term "substituted" as used herein, means that any one or more hydrogens on the designated atom is replaced with a selection from the indicated substituents, provided that the designated atom's normal valence is not exceeded, and that the substitution results in a stable compound, i.e., a compound that can be isolated and characterized using conventional means. For example, substitution can be in the form of an oxygen bound to any other chemical atom than carbon, e.g. hydroxyl group, or an oxygen anion. When a substituent is oxo, i.e., =O, then 2 hydrogens on the atom are replaced. An oxo group that is a substituent of an aromatic carbon atom results in a conversion of -CH- to -C(=O)- and a loss of aromaticity. For example, a pyridyl group substituted by oxo is a pyridone.

The term "heteroatom" as used herein shall be understood to mean atoms other than carbon and hydrogen such as and preferably O, N, S and P.

If a first compound, a substituent or a residue ends, e.g., in the name "-3-yl", this ending indicates that the first compound, substituent or residue is covalently bound to a second compound, substituent or residue at the atom number 3 position of the first compound. Of course, this definition holds true for any given integer before the "-yl" terminus of the compound's, substituent's or residue's name. For example, if 1-(1,3-benzodioxol-5-ylmethyl)pyrrol-3-yl is selected as a first residue to be covalently bound to the second residue 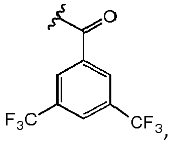 the following compound is formed:

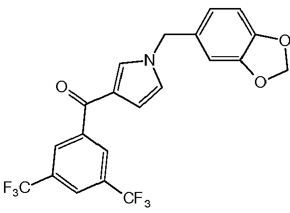

In the context of the present invention it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. "(C₁₋₁₀)alkyl" denotes a hydrocarbon residue containing from 1 to 10 carbon atoms, e.g. a methyl, ethyl, propyl, iso-propyl, n-butyl, iso-butyl, sec-butyl, tert-butyl, n-pentyl, iso-pentyl, n-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), iso-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, preferably 1, 2, 3, 4, 5, or 6 double bond(s).

The expression "alkynyl" refers to at least partially unsaturated, substituted or non-substituted straight-chain or branched hydrocarbon groups that contain the number of carbon items indicated, e.g. "(C₂₋₁₀)alkynyl" denotes a hydrocarbon residue containing from 2 to 10 carbon atoms, for example an ethinyl, propinyl, butinyl, acetylenyl, or propargyl group. Preferably, alkynyl groups have one or two (especially preferably one) triple bond(s).

Furthermore, the terms "alkyl", "alkenyl" and "alkynyl" refer to groups in which one or more hydrogen atom(s) have been replaced, e.g. by a halogen atom, preferably F or Cl, such as, for example, a 2,2,2-trichloroethyl or a trifluoromethyl group.

The term "carbocycle" shall be understood to mean a substituted or non-substituted aliphatic hydrocarbon cycle containing the number of carbon items indicated, e.g. "(C₃₋₁₀)carbocycle" or from 3 to 20, preferably from 3 to 12 carbon atoms, more preferably 5 or 6 carbon atoms. These carbocycles may be either aromatic or non-aromatic systems. The non-aromatic ring systems may be mono- or polyunsaturated.

The term "carbobicycle" refers to a carbocycle as defined above comprising more than 1 ring, preferably two rings. Preferred carbocycles and carbobicycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, benzocycloheptenyl, spiro[4,5]decanyl, norbornyl, decalinyl, bicyclo[4.3.0]nonyl, tetraline, or cyclopentylcyclohexyl. The carbo- and/or carbobicyclic residue may be bound to the remaining structure of the complete molecule by any atom of the cycle, which results in a stable structure

The term "carbocycle" shall also include "cycloalkyl" which is to be understood to mean aliphatic hydrocarbon-containing rings preferably having from 3 to 12 carbon atoms. These non-aromatic ring systems may be mono- or polyunsaturated, i.e. the term encompasses cycloalkenyl and cycloalkynyl.

The term "heterocycle" refers to a stable substituted or non-substituted, aromatic or non-aromatic, preferably 3 to 20 membered, more preferably 3-12 membered, most preferably 5 or 6 membered, monocyclic, heteroatom-containing cycle. Each heterocycle consists of carbon atoms and one or more, preferably 1 to 4, more preferably 1 to 3 heteroatoms preferably chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

The term "heterobicycle" refers to a heterocycle as defined above comprising more than 1 ring, preferably two rings.

The hetero- and/or heterobicyclic residue may be bound to the remaining structure of the complete molecule by any atom of the cycle, which results in a stable structure. Exemplary heterocycles and heterobicycles include, but are not limited to pyrrolidinyl, pyrrolinyl, morpholinyl, thiomorpholinyl, thiomorpholinyl sulfoxide, thiomorpholinyl sulfone, dioxalanyl, piperidinyl, piperazinyl, tetrahydrofuranyl, 1-oxo-λ4-thiomorpholinyl, 13-oxa-11-aza-tricyclo[7.3.1.0-2,7]-tridecy-2,4,6-triene, tetrahydropyranyl, 2-oxo-2H-pyranyl, tetrahydrofuranyl, 1,3-dioxolanone, 1,3-dioxanone, 1,4-dioxanyl, 8-oxa-3-aza-bicyclo[3.2.1]octanyl, 2-oxa-5-aza-bicyclo[2.2.1]heptanyl, 2-thia-5-aza-bicyclo[2.2.1]heptanyl, piperidinonyl, tetrahydro-pyrimidonyl, pentamethylene sulphide, pentamethylene sulfoxide, pentamethylene sulfone, tetramethylene sulphide, tetramethylene sulfoxide and tetramethylene sulfone, indazolyl, benzimidazolyl, benzodioxolyl, imidazolyl, 1,3-benzodioxolyl and pyrazolyl.

The expressions "alkyl/alkenyl/alkynyl ether" refer to a saturated or non-saturated, straight-chain or branched hydrocarbon group that contains the number of carbon items indicated. For example, "(C₁₋₁₀)alkyl ether" denotes a hydrocarbon residue containing from 1 to 10 carbon atoms, and any suitable number of oxygen atoms that will result in an ether structure. Alkyl/alkenyl/alkynyl ether groups as used herein shall be understood to mean any linear or branched, substituted or non-substituted alkyl/alkenyl/alkynyl chain comprising an oxygen atom either as an ether motif, i.e. an oxygen bound by two carbons. The ether residue can be attached to the Formulas provided in the present invention either via the carbon atom or via the oxygen atom of the ether residue.

The "substituent" or "residue" or "**R**" as used herein, preferably **R², R³, R⁴, R⁵**, **R⁶**, **R⁷**, **R⁸**, **R⁹**, **R¹⁰** and/or **R¹¹** can be attached directly to the Formulas provided in the present invention or by means of a linker. Said linker can also be in the form of polyethyleneglycol. The term polyethyleneglycol as used herein refers to a chain of substituted or non-substituted ethylene oxide monomers.

As used herein, the terms "nitrogen" or "N" and "sulphur" or "S" include any oxidized form of nitrogen and sulphur and the quaternized form of any basic nitrogen as long as the resulting compound is chemically stable. For example, for an -S-C₁₋₆ alkyl radical shall be understood to include -S(O)-C₁₋₆alkyl and -S(O)₂-C₁₋₆alkyl.

A residue connected via a given position to a second compound of interest is to be understood as a residue that is covalently bound to the second compound at the atom position indicated. For example, indazolyl connected via position (5) of the indazolyl denotes the following residue: 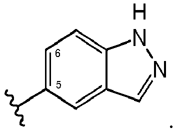 In this case, the numbering starts - as customary in the art - on the 1H-nitrogen. However, it is noted that some nomenclature may provide a different starting point for the numbering. For example, a 1H-benzimidazol-6-yl residue is identical to a 3H-benzimidazol-5-yl residue, as is understood by the skilled person.

As used herein, a wording defining the limits of a range of length such as, e. g., "from 1 to 5" or "(C₁₋₅)" means any integer from 1 to 5, i. e. 1, 2, 3, 4 and 5. In other words, any range defined by two integers explicitly mentioned is meant to comprise and disclose any integer defining said limits and any integer comprised in said range.

By way of example, the term "mono- or di-substituted in meta position or mono-substituted in para position", as used herein, means that a compound is either substituted by at least one given substituent in para position to the position where the compound is attached to another compound or residue, or substituted in two of its meta positions by at least one substituent. For example, the term "di-substituted in meta position by (C₃)carbocycle or -(CF₃)" denotes that a compound is substituted by one (C₃)carbocycle or -(CF₃) in each meta position or by a (C₃)carbocycle in one meta position and by -(CF₃) in the other meta position. Preferably, the term denotes that a compound is substituted by one (C₃)carbocycle in each meta position or by one -(CF₃) in each meta position, i.e. is substituted in both meta positions by the same substituent. As denoted above for the para position, the meta position denotes the position meta to the position where the compound is attached to another compound or residue.

As an example, the term "phenyl, preferably mono-substituted in para or meta position by cyclopropyl or -(CF₃), or di-substituted in meta position by cyclopropyl or -(CF₃) in each meta position" preferably denotes the following structures:

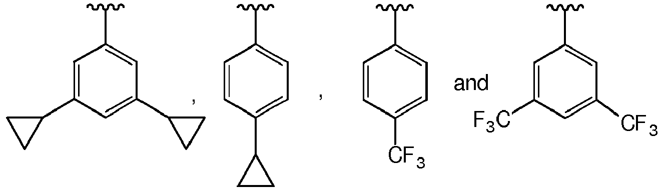

The residues **R¹, R⁵** and/or **R⁹** for use in the present invention are preferably phenyl that is mono-substituted in para position by a group consisting of Cl, F, Br, substituted or non-substituted methyl, preferably -(CF₃), ethyl, propyl and cyclopropyl. The residues **R¹²** and **R¹³** for use in the present invention are preferably (C₆)carbocycle, more preferably phenyl that is mono-substituted in para position by a (C₃)carbocycle, preferably cyclopropyl, or -(CF₃), or di-substituted in meta position by (C₃)carbocycle, preferably cyclopropyl, or (-CF₃). It is further preferred that **R¹, R⁵**, **R⁹**, **R¹²** and/or **R¹³** are phenyl that is mono-, di- or tri-substituted in ortho, meta and/or para position by a group consisting of Cl, F, Br, substituted or non-substituted methyl, preferably - (CF₃), ethyl, propyl and cyclopropyl. The di- or tri-substituted phenyl representing **R¹, R⁵**, **R⁹**, **R¹²** and/or **R¹³** can be di- or tri-substituted phenyl that is substituted with the same substituent in the respective ortho, meta and para position or by different substituents in the respective ortho, meta and/or para position, wherein the substituents are selected from the group consisting of Cl, F, Br, substituted or non-substituted methyl, preferably -(CF₃), ethyl, propyl and cyclopropyl. Each combination and number of substituents selected from the group consisting of Cl, F, Br, substituted or non-substituted methyl, preferably -(CF₃), ethyl, propyl and cyclopropyl in ortho, meta and/or para position of the phenyl representing **R¹**, **R⁵**, **R⁹**, **R¹²** and/or **R¹³** is explicitly disclosed herein.

The scope of the present invention includes those analogs of the compounds as described above and in the claims that, e.g. for reasons of metabolic stability, feature the exchange of one or more carbon-bonded hydrogens, preferably one or more aromatic carbon-bonded hydrogens, with halogen atoms such as F, Cl, or Br, preferably F. For example, Compound-1 can feature one or more halogen atoms, preferably F, instead of the aromatic carbon-bonded hydrogens in the phenyl ring or instead of the aromatic or non-aromatic carbon-bonded hydrogens in the 1,3-benzodioxol-5-yl-moiety. Also, for example, Compound-4 can feature one or more halogen atoms, preferably F, instead of the aromatic carbon-bonded hydrogens in the pyrimidine ring or instead of the aromatic or non-aromatic carbon-bonded hydrogens in the benzodioxole moiety.

In a preferred embodiment, the present invention is directed to a herein-described compound for use as described herein, wherein the compound inhibits the PHF (paired helical filament) Tau hyperphosphorylation, preferably also inhibits phosphorylation of the serine/arginine-rich splicing factor 1 (SRSF1, ASF-1, SF2) by a kinase, preferably by the G-protein-coupled receptor kinase 2 (GRK2, ADRBK1), more preferably also inhibits the formation of Abeta peptides and Abeta plaques, more preferably also inhibits neurodegeneration and/or neuronal loss, preferably hippocampal neuronal loss.

The herein observed neuroprotective activity of the GRK2-inhibitory compounds could involve inhibition of mitochondrial dysfunction, which is supported by a previous study, which demonstrates that the active GRK2 induces mitochondrial dysfunction (Sato et al., J. Mol. Cell. Cardiol 89, 360-364 (2015)). In addition, inhibition of GRK2-mediated activating SRSF1 phosphorylation, which promotes aberrant prelamin A (LMNA) mRNA splicing, enhanced ageing and mitochondrial dysfunction (Harthouri et al., EMBO Mol. Med. 9, 1294-1313 (2017); Gonzalo et al.,Ageing Res. Rev. 33, 18-29 (2017)), could also contribute to inhibition of neurodegeneration, which is triggered and aggravated by mitochondrial dysfunction (Lin and Beal, Nature 443, 787-795 (2006)).

In another aspect, the present invention is directed to a pharmaceutical composition, comprising as active substance a compound for use as described herein or a pharmaceutically acceptable derivative thereof, optionally combined with excipients and/or carriers.

The invention includes pharmaceutically acceptable salts or solvates of the compounds of Formula (I) and (II) of the present invention. A "pharmaceutically acceptable salt or solvate" refers to any pharmaceutically acceptable salt, solvate or ester or any other compound which, upon administration to a patient, is capable of providing (directly or indirectly) a compound of the invention, or a pharmacologically active metabolite or pharmacologically active residue thereof. A pharmacologically active metabolite shall be understood to mean any compound of the invention capable of being metabolized enzymatically or chemically. This includes, for example, hydroxylated or oxidized derivative compounds of the present invention.

Pharmaceutically acceptable salts include those derived from pharmaceutically acceptable inorganic and organic acids and bases. Examples of suitable acids include hydrochloric, hydrobromic, sulphuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal (e.g. magnesium), ammonium and N-(C₁-C₄alkyl)₄⁺ salts.

In addition, the scope of the invention also encompasses prodrugs of compounds of the present invention. Prodrugs include those compounds that, upon simple chemical transformation, are modified to produce compounds of the invention. Simple chemical transformations include hydrolysis, oxidation and reduction. Specifically, when a prodrug is administered to a patient, the prodrug may be transformed into a compound disclosed hereinabove, thereby imparting the desired pharmacological effect.

In a preferred embodiment, the compounds for use in the present invention are for use in the treatment of CNS- and neurodegenerative diseases selected from the group consisting of dementia-associated CNS- and neurodegenerative disorders, preferably schizophrenia with dementia, depression-associated CNS- and neurodegenerative disorders, preferably depression and depression-related symptoms (anhedonia, anorexia), brain injury, preferably traumatic brain injury, cerebrovascular disease-induced neurodegeneration, preferably ischemic stroke-induced neurodegeneration, hypertension-induced neurodegeneration, atherosclerosis-induced neurodegeneration, amyloid angiopathy-induced neurodegeneration, preferably small-vessel cerebrovascular disease, motor neuron disease, ALS (amyotrophic lateral sclerosis), multiple sclerosis, familial and sporadic forms of Alzheimer's Disease, vascular dementia, Morbus Parkinson, chromosome-17-linked Morbus Parkinson, frontotemporal dementia, Korsakoff's psychosis, Lewy Body diseases, progressive supranuclear palsy, corticobasal degeneration, Pick's disease, Huntington's disease, thalamic degeneration, prion-associated diseases, preferably Creutzfeld-Jacob disease, HIV-associated dementia, diabetes-induced neuropathy, neurodegenerative symptoms of ageing, preferably loss of appetite or greying of hair, cognitive-related disorders, mild cognitive impairment, age-associated memory impairment, age-associated cognitive decline, vascular cognitive impairment, central and peripheral neuronal symptoms of atherosclerosis and ischemia, stress-related CNS- and neurodegenerative disorders, attention deficit disorders, attention deficit hyperactivity disorders, memory disturbances in children, and progeria infantilis;

Preferably, the compound or pharmaceutical composition as described herein is for use in the treatment of animals or humans, more preferably mammalians, most preferably humans.

All the above-mentioned diseases and disorders are associated with CNS- and/or neurodegenerative symptoms. For example, prolonged stress and depression are both associated with CNS- and neurodegeneration. The same, stress and ageing can lead to CNS- and/or neurodegenerative symptoms such as age/stress-associated memory impairment, age/stress-associated cognitive decline, age/stress-related loss of appetite and also age/stress-associated greying of hair. In the experimental section below it is demonstrated in a rodent model that oral administration of the compounds disclosed herein actually increased appetite and retarded greying of hair in a representative rodent model for stress and ageing.

As commonly used, the term treatment encompasses the actual therapeutic treatment of an existing disease as well as the prophylactic, i.e. preventive treatment of a future disease.

In a preferred embodiment, the present invention relates to compounds for the therapeutic and/or prophylactic treatment of CNS- and neurodegenerative diseases selected from the group consisting of
i. therapeutic treatment of CNS- and neurodegenerative disease-associated dementia;
ii. therapeutic treatment of CNS- and neurodegenerative disease-associated depression;
iii. therapeutic treatment of brain injury, preferably traumatic brain injuries;
iv. therapeutic and prophylactic treatment of cerebrovascular diseases-induced neurodegeneration (i.e. ischemic stroke-induced neurodegeneration, hypertension-induced neurodegeneration, atherosclerosis-induced neurodegeneration, amyloid angiopathy-induced neurodegeneration) and preferably small-vessel cerebrovascular disease;
v. therapeutic treatment of motor neuron disease;
vi. therapeutic treatment of ALS;
vii. therapeutic treatment of multiple sclerosis;
viii. therapeutic and prophylactic treatment of familial and sporadic forms of Alzheimer's Disease;
ix. therapeutic treatment of CNS- and neurodegenerative disease-associated vascular dementia;
x. therapeutic treatment of CNS- and neurodegenerative disease-associated frontotemporal dementia;
xi. therapeutic and prophylactic treatment of Morbus Parkinson, preferably chromosome-17-linked Morbus Parkinson;
xii. therapeutic and prophylactic treatment of symptoms of depression and depression-related symptoms, preferably anhedonia and anorexia;
xiii. therapeutic treatment of CNS- and neurodegenerative disease-associated schizophrenia with dementia;
xiv. therapeutic treatment of Korsakoff's psychosis;
xv. therapeutic treatment of Lewy Body diseases;
xvi. therapeutic treatment of progressive supranuclear palsy;
xvii. therapeutic treatment of corticobasal degeneration;
xviii. therapeutic treatment of Pick's disease;
xix. therapeutic treatment of Huntington's disease;
xx. therapeutic treatment of CNS- and neurodegenerative disease-associated thalamic degeneration;
xxi. therapeutic treatment of prion diseases, preferably Creutzfeld-Jacob disease;
xxii. therapeutic treatment of HIV-associated dementia;
xxiii. therapeutic and prophylactic treatment of diabetes-induced neuropathy;
xxiv. therapeutic and prophylactic treatment of CNS- and/or neurodegeneration-associated symptoms such as age/stress-associated memory impairment, age/stress-associated cognitive decline, age/stress-related loss of appetite, and age/stress-associated greying of hair;
xxv. therapeutic and prophylactic treatment of cognitive-related disorder;
xxvi. therapeutic and prophylactic treatment of mild cognitive impairment;
xxvii. therapeutic and prophylactic treatment of vascular cognitive impairment; therapeutic and prophylactic treatment of central and peripheral symptoms of atherosclerosis and ischemia;
xxviii.therapeutic and prophylactic treatment of perivascular disease;
xxix. prophylaxis against renal dysfunction and renal failure;
xxx. therapeutic and prophylactic treatment of stress-related disorders;
xxxi. therapeutic and prophylactic treatment of attention deficit disorders;
xxxii. therapeutic and prophylactic treatment of attention deficit hyperactivity disorders;
xxxiii.therapeutic and prophylactic treatment of memory disturbances in children;
and wherein the compound or pharmaceutical composition is for use in the treatment of animals or humans, preferably mammalians, more preferably humans.

In a more preferred embodiment, the present invention relates to compounds for use in the treatment of CNS- and neurodegenerative diseases selected from the group consisting of
(i) therapeutic and prophylactic treatment of familial and sporadic forms of Alzheimer's Disease;
(ii) therapeutic and prophylactic treatment of tauopathies;
(iii) therapeutic and prophylactic treatment of Morbus Parkinson;
(iv) therapeutic and prophylactic treatment of diabetes-induced neuropathy, preferably diabetes type 2; and
(v) therapeutic and prophylactic treatment of dementias associated with neurodegeneration.

For therapeutic use, the compounds described herein may be administered in any conventional dosage form in any conventional manner. Routes of administration include, but are not limited to oral administration, intravenous, intramuscular and subcutaneous injections. The preferred modes of administration are oral, intravenous or subcutaneous.

The compounds may be administered alone or in combination with adjuvants that enhance stability of the compounds, facilitate administration of pharmaceutical compositions containing them in certain embodiments, provide increased dissolution or dispersion, increase activity, provide adjunct therapy (e.g. with approved drugs for the treatment of AD, or inhibitors of the ACE or the AT1 receptor) and the like, including other active ingredients.

The herein-described compounds may be physically combined with conventional therapeutics or other adjuvants into a single pharmaceutical composition. Reference in this regard may be made to Cappola et al.: U.S. patent application no. 09/902,822, PCT/US 01/21860 und US provisional application no. 60/313,527, each incorporated by reference herein in their entirety. Advantageously, the compounds may then be administered together in a single dosage form. In some embodiments, the pharmaceutical compositions comprising such combinations of compounds contain at least about 5 %, but more preferably at least about 20 %, of a compound of the present invention (w/w). The optimum percentage (w/w) of a compound of the invention may vary and is within the purview of those skilled in the art. Alternatively, the compounds may be administered separately (either serially or in parallel). Separate dosing allows for greater flexibility in the dosing regime.

As mentioned above, dosage forms of the compounds described herein include pharmaceutically acceptable carriers and adjuvants known to those of ordinary skill in the art. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from 1-300 mg/dose for a 70 kg patient. Although one dose per day may be sufficient, up to 5 doses per day may be given. For oral doses, up to 2000 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician.

In another aspect, the present invention is directed to a method for the therapeutic or prophylactic treatment of a patient suffering or likely of suffering from a CNS- or neurodegenerative disease, preferably a mammalian patient, more preferably a human patient, the method comprising the step of administering a therapeutically or prophylactically effective amount of a compound or pharmaceutical composition for use according to any one of claims 1 to 14 to the patient in need of such treatment..

The following Figures and Examples serve to illustrate the invention and are not intended to limit the scope of the invention as described in the appended claims.

### Figures

**Fig. 1****.** Compound-1 and Compound-4 retard Abeta plaque formation in Tg2576 mice
**Fig. 1A** shows the chemical formulas of Compound-1: 1-(1,3-benzodioxol-5-yl)-4-(cyclopropanecarbonyl)-3-hydroxy-2-phenyl-2H-pyrrol-5-one; and Compound-4: 4-(1,3-benzodioxol-5-yl)pyrimidine;
**Figs. 1B and 1C** illustrate the immunohistological assessment of Abeta plaque load in hippocampal and frontal cortex areas from 18 months-old Tg2576 mice treated with Compound-1 and Compound-4 for 6 months compared to untreated Tg2576 mice. The upper panels show images from 4 mice/group (**B**), and the lower panel (**C**) shows quantitative evaluation of plaque area (±s.d., n=8; ***, p<0.001; Tukey's test).
**Fig. 2****.** Compound-1 and Compound-4 retard hippocampal neuronal loss and Tau hyperphosphorylation in Tg2576 mice subjected to CUMS (chronic unpredictable mild stress)
**Figs. 2A and 2B** show the quantification of neuronal cell bodies by direct binding assay with [¹²⁵I]-labeled anti-NeuN antibody (**A**) and hyperphosphorylated Tau with [125I]-labeled AT8 antibody (**B**) in hippocampi isolated from 15 months-old Tg2576 mice subjected to the CUMS (chronic unpredictable mild stress) protocol for three months and treated with Compound-1 and Compound-4 compared to untreated stressed Tg2576 controls (±s.d., n=8; ***, p<0.001; Tukey's test).
**Fig. 3****.** Compound-1 and Compound-4 retard hippocampal Tau hyperphosphorylation in a rat model with symptoms of sporadic AD
**Figs. 3A and 3B** demonstrate hippocampal Tau hyperphosphorylation as determined in an immunoblot with anti-PHF antibody (AT8) in a rat model with symptoms of sporadic AD triggered in 16 months-old rats by the CUMS protocol for 4 weeks. Hippocampi were evaluated from stressed rats treated with Compound-1 and Compound-4 relative to untreated stressed controls (n=5/- group). The upper panel shows immunoblot detection (**A**), and the lower panel (**B**) shows quantitative data evaluation (±s.d., n=5, **, p=0.0014 vs. untreated control rats subjected to CUMS; Tukey's test).
**Fig. 4****.** Compound-1 and Compound-4 retard Tau hyperphosphorylation in the Tg-TauP301L model of tauopathy
**Fig. 4A** illustrates the immunohistological detection of hippocampal Tau hyperphosphorylation as performed with anti-PHF antibody (AT8) on hippocampal sections of 12 months-old Tg-Tau-P301L mice after treatment for 6 months with Compound-1 and Compound-4 compared to untreated Tg-TauP301L controls; bar: 40 microm.
**Fig. 4B** is a bar graph showing the quantitative determination of hippocampal Tau hyperphosphorylation in 12 months-old Tg-TauP301L mice after treatment for 6 months with Compound-1 and Compound-4 compared to untreated Tg-TauP301L controls was performed by direct binding assay with [125I]-labeled AT8 antibody (±s.d., n=8, ***, p<0.001; Tukey's test).

### Examples

### Materials and Methods

### Compound synthesis

Compounds for use in the present invention can and were synthesized by routine adaption of standardized protocols, for example, were synthesized by EMC microcollections GmbH, Tuebingen, Germany and ChiroBlock GmbH, Wolfen, Germany. The synthesis of such compounds was performed in a small scale by solid phase chemical synthesis methods, which were adapted from established protocols (For "Compound-1" (1-(1,3-benzodioxol-5-yl)-4-(cyclopropane-carbonyl)-3-hydroxy-2-phenyl-2H-pyrrol-5-one): Poncet J, et al., J. Chem. Soc. Perkin Trans I., 611-616 (1990); for "Compound-2" (1-(1,3-benzodioxol-5-ylmethyl)-5-(4-fluorophenyl)-2-methyl-pyrrole-3-carboxamide), "Compound-22" (1-(1,3-benzodioxol-5-ylmethyl)-2-methyl-5-phenyl-pyrrole-3-carboxamide), "Compound-23"(1-(1,3-benzodioxol-5-ylmethyl)-2-methyl-5-(p-tolyl)-pyrrole-3-carboxamide), and "Compound-24" (1-(1,3-benzodioxol-5-ylmethyl)-5-(4-chlorophenyl)-2-methyl-pyrrole-3-carboxamide): Trautwein AW, et al., Bioorg. Med. Chem. Lett. 8, 2381-2384 (1998); for Compound-3: Sakai K, et al., Chem. Pharm. Bull. 29(6) 1554-1560 (1981); for "Compound-4": Coombs TC, et al., Bioorg. Med. Chem. Lett. 23, 3654-3661 (2013); and for "Compound-5" ((4R)-N3-(1,3-benzodioxol-5-ylmethyl)-N4-[[3-(trifluoromethyl)phenyl]-methyl]-pyrrolidine-3,4-dicarboxamide): Baber JC, et al., Bioorg. Med. Chem. 20, 3565-3574 (2012)). In addition, Compound-1 and Compound-4 were synthesized in a larger scale as detailed below.

### Synthesis of Compound-1

Synthesis of Compound-1 (1-(1,3-Benzodioxol-5-yl)-4-(cyclopropanecarbonyl)-3-hydroxy-2-phenyl-2H-pyrrol-5-one) was performed by a 6-step chemical reaction process (ChiroBlock GmbH, Wolfen, Germany). Step-1 encompassed the synthesis of methyl 2-(1,3-benzodioxol-5-ylamino)-2-phenyl-acetate. A mixture of methyl 2-oxo-2-phenyl-acetate (96 g, 584 mmol, 4.0 equivalents), 1,3-benzodioxol-5-amine (20 g, 146 mmol, 1.0 equivalents), and Na₂SO₄ in cyclohexane (800 ml) was refluxed under N₂ for 21 h. 5 % Pd/C (7.8 g) was added, and the obtained suspension was hydrogenated at 20 bar and 20 °C for 48 h. The resulting heterogeneous mixture was diluted with EtOAc (ca. 800 ml) and filtered through Celite. The filtrate was concentrated in vacuo (40 °C, 100 mbar) to yield a brown oil (135 g) that was purified by flash chromatography (silica gel, ethyl acetate - petroleum ether 12:88 to 30:70) to yield target 3, which was an off-white solid (18.46 g; purity 95 %, yield 44 %).

Step-2 was the synthesis of S-tert-butyl ethanethioate. A solution of pyridine (87.0 g, 1.1 mol, 1.1 equivalents) in chloroform (800 ml) was cooled in an ice bath and treated with acetyl chloride (86.4 g, 1.1. mol, 1.1 equivalents), with the reaction temperature not exceeding 11 °C. To the resulting orange suspension, 2-methylpropane-2-thiol (90.2 g, 1.0 mol, 1.0 equivalents) was dropwise added over 40 min., and the mixture was stirred for 48 h and subsequently quenched with water (500 ml). The phases were separated and the aqueous phase was extracted with chloroform (400 ml). The combined organic extracts were washed with 400 ml each of water, 10 % H₂SO₄, sat. NaHCO₃, and water being subsequently dried over Na₂SO₄. The obtained chloroformic solution was subjected to fractional distillation, which afforded target S-tert-butyl ethanethioate as a clear liquid (55.8 g, purity 95 %, yield 45 %).

In Step-3 the synthesis of S-(2-pyridyl) cyclopropanecarbothioate was performed. Cyclopropanecarbonyl chloride (23.5 g, 225 mmol, 1.0 equiv.) was dropwise added to solution of pyridine-2-thiol (25.0 g, 225 mmol, 1.0 equiv.) in THF (250 ml) at 20 °C. The mixture was stirred for 10 min, filtered, and the filter cake was washed with 1:4 Et₂O/petrol ether (250 ml). The thus obtained solid was dissolved in water (250 ml) and treated with NaHCO₃ (19 g, 225 mmol, 1.0 equiv.), and the aqueous solution was extracted with 2*250 ml EtOAc. The combined organic fractions were dried over Na₂SO₄ and concentrated in vacuo to afford S-(2-pyridyl) cyclopropanecarbothioate as a yellow oil (37 g, purity 95 %; yield 92 %).

Step-4 was the synthesis of S-tert-butyl 3-cyclopropyl-3-oxo-propanethioate. A 2-L 3-neck round-bottom flask was charged with HMDS (83.3 g, 516 mmol, 2.5 equiv) and freshly distilled THF (800 ml). The obtained mixture was cooled in an acetone/dry ice bath, and 1.6 M nBuLi in hexanes (323 ml, 516 mmol, 2.5 equiv.) was dropwise added while keeping the temperature below -50 °C. Subsequently, the obtained mixture was sequentially treated with solutions of S-(2-pyridyl) cyclopropanecarbothioate (37.0 g, 206 mmol, 1.0 equiv.) and S-tert-butyl ethanethioate (23.4 g, 214 mmol, 1.04 equiv.). The obtained solution was stirred for 1 h at -30 °C, and the reaction was quenched (under TLC process control) by 1 N H₂SO₄ (800 ml). The resulting suspension was extracted with EtOAc (3*900 ml), and the organic fractions combined, washed with brine (2 L), dried over Na₂SO₄, and concentrated in vacuo. The crude product was purified by flash chromatography (silica gel, ethyl acetate - petroleum ether 25:75) to yield target S-tert-butyl 3-cyclopropyl-3-oxo-propanethioate as a brown oil (29.5 g, purity 83 %, yield: 59 %).

In Step-5, the synthesis of Methyl 2-[1,3-benzodioxol-5-yl-(3-cyclopropyl-3-oxo-propanoyl)amino]-2-phenyl-acetate was performed. A 1-L round-bottom flask was charged with Methyl 2-(1,3-benzodioxol-5-ylamino)-2-phenyl-acetate (18.5 g, 61 mmol, 1.0 equiv.), S-tert-butyl 3-cyclopropyl-3-oxo-propanethioate (15.9 g, 66 mmol, 1.073 equiv.), CF₃COOAg 814.6 g, 66 mmol, 1.073 equiv.), and distilled THF (400 ml), and the obtained mixture was stirred at 20 °C for 36 h (the process was controlled by TLC). The dark-brown reaction mixture was concentrated in vacuo and purified by flash chromatography (silica gel, ethyl acetate -petroleum ether 25:75 to 50:50) to yield target Methyl 2-[1,3-benzodioxol-5-yl-(3-cyclopropyl-3-oxo-propanoyl)amino]-2-phenyl-acetate as a brown oil (21.0 g, purity: 90 %, yield: 78 %).

The final Step-6 yielded the final target 1-(1,3-Benzodioxol-5-yl)-4-(cyclopropanecarbonyl)-3-hydroxy-2-phenyl-2H-pyrrol-5-one (Compound-1). A 500 ml round-bottom flask was charged with Methyl 2-[1,3-benzodioxol-5-yl-(3-cyclopropyl-3-oxo-propanoyl)amino]-2-phenyl-acetate (20.0 g; 45.5 mmol, 1.0 equiv.), CsF (6.9 g, 45.5. mmol, 1.0 equiv.), and DMF (140 ml), and the obtained mixture was stirred at 60 °C for 20 h (the process was controlled by TLC). The dark-brown reaction mixture was concentrated in vacuo and the residue was treated with 1N H₂SO₄ (400 ml). The obtained mixture was extracted with EtOAc (500 ml), and the organic phase was washed with brine (2*300 ml), dried over Na₂SO₄, and concentrated in vacuo to afford crude 1-(1,3-Benzodioxol-5-yl)-4-(cyclopropanecarbonyl)-3-hydroxy-2-phenyl-2H-pyrrol-5-one as a brown solid (19 g). The above solid was washed on filter with EtOAc until becoming colorless, affording target Compound-1 (1-(1,3-Benzodioxol-5-yl)-4-(cyclopropanecarbonyl)-3-hydroxy-2-phenyl-2H-pyrrol-5-one) as an off-white solid (5.0 g, purity: 98 %, yield: 30 %).

### Synthesis of Compound-4

Compound-4 (4-(1,3-Benzodioxol-5-yl)pyrimidine) was synthesized by the following procedure (ChiroBlock GmbH, Wolfen, Germany). A 250 ml round-bottom flask was loaded with 1-(1,3-Benzodioxol-5-yl)ethanone (10.0g, 60.9 mmol, 1.0 equivalent), (EtO)₃CH (27 g, 183 mmol, 3.0 equivalents), ZnCl₂ (0.83 g, 6.1 mmol, 0.1 equivalent), NH4CH3COO (0.4 g, 122 mmol, 2.0 equivalents) and toluene (120 ml), and the obtained mixture was stirred at reflux for 48 g and subsequently at 20 °C for 48 h (the process was controlled by TLC). The reaction mixture was quenched with saturated NaHCO₃ (400 ml) and extracted with chloroform (400 ml). The organic phase was dried over Na2SO4 and concentrated in vacuo, and the resulting crude product was purified by flash chromatography (silica gel, MeOH-CHCl₃ (0:100 to 5:95) to yield target Compound-4 (4-(1,3-Benzodioxol-5-yl)pyrimidine) as an off-white solid (3.0 g, purity 97 %; yield 25

### Animal models

For a genetic model of familial AD (FAD), Tg2576 mice (Taconic Biosciences, Rensselaer, NY, USA) were used with neuron-specific overexpression of human APPSwe, i.e the Swedish mutation of APP695 isolated from a Swedish family with FAD featuring the double mutation K670N/M671L (Hsiao et al., Science 274, 99-103 (1996)). As a genetic model of tauopathy, the Tg-TauP301L mice (Model 2508, Taconic Biosciences, Rensselaer, NY, USA) with neuron-specific expression of the most common FTDP-17 (frontotemporal dementia and parkinsonism linked to chromosome 17) mutation (Lewis et al., Nature Genetics 25, 402-405 (2000)) were used. To enhance neurodegeneration and neuronal loss, the CUMS (chronic unpredictable mild stress) protocol was performed with male 12 months-old Tg2576 mice for 3 months essentially as described (AbdAlla et al., J. Biol. Chem. 284, 6554-6565 (2009); AbdAlla et al., J. Biol. Chem. 284, 6566-6574 (2009)).

As a model for reproducing major features of sporadic AD and depression, 15 months-old male rats were aged according to the CUMS protocol for 4 weeks. The following stimuli were administered each week in a random order: two periods (7h and 17 h) of 45 ° cage tilt; soaked cage for 17 h; food deprivation (24h) and water deprivation (12h), twice a week; paired housing (17 h); overnight illumination during the dark phase, twice a week; noise (85 dB) in the room for 5h, twice a week; flashing light (60 flashes/min) for 6h, three times a week (AbdAlla et al., Biomed. Res. Int. 2015:917156 (2015); El-faramawy et al., Pharmacol. Biochem. Behav. 91, 339-344 (2009)). The sucrose preference test (2 % sucrose in water) was done immediately after a period of food and water deprivation. After four weeks of stress, more than 90 % of untreated stressed rats showed signs of anhedonia, which was documented by a decreased sucrose consumption in the sucrose preference test (≤ 50 % compared to non-stressed age-matched control group and/or the stressed group treated with compound-1 and compound-4).

As indicated, representative compounds (Compound-1 and Compound-4; 5 mg/kg body weight/d) were added to drinking water. Treatment of the Tg2576 model was performed for three and six months starting at an age of 12 months. Treatment of the Tg-TauP301L model was started at an age of 6 months and continued until 12 months. Aged 15 months-old rats were treated during the CUMS protocol. All mice/rats were kept on a light/dark cycle of 12 h light/12h dark, had free access to food and water (unless the CUMS protocol required a restriction) and were fed a standard rodent chow. At the end of the observation period, mice or rats were anesthetized with tribromoethanol (250 mg/kg; i.p.) or urethane (1 g/kg, i.p.), perfused intracardially with sterile PBS, and brains were isolated, and processed for histology or biochemical analyses. For protein extraction, hippocampi were dissected and immediately frozen in liquid nitrogen. All animal experiments were performed in accordance with NIH guidelines and approved by the local committees on animal experiments (Univ. Zurich and MRC Cairo).

### Antibodies

The following antibodies were used for immunoblotting and/or immunohistology: Abeta plaques were stained with monoclonal mouse antibody BAM-10 (crossreactive with residues 1-12 of the Abeta peptide, Sigma-Aldrich, St. Louis, MO, USA); PHF-Tau was detected with monoclonal AT8 antibody (MN1020; Thermo Fisher Scientific, Waltham, MA, USA); mouse monoclonal anti-NeuN antibody was raised against the neuron-specific protein NeuN (MAB377, clone A60, EMD Millipore, Merck KGaA, Darmstadt, Germany).

### Immunohistochemistry

For Abeta plaque load quantification by immunohistochemistry, paraffin-embedded brain sections (or cryosections) (8 microm, 10-15 sections/brain taken at 30-50 microm intervals) were prepared from brains isolated from 18-month-old Tg2576 mice (Taconic Biosciences, Rensselaer, NY, USA) treated for six months without and with Compound-1 and Compound-4 (5 mg/kg body weight/day in drinking water). After antigen retrieval by microwave heating for 30 min in antigen retrieval buffer (10 mM sodium citrate, pH 6.0 supplemented with 0.05 % Tween-20), sections were washed with PBS, and endogenous peroxidases were inactivated by incubation for 5 min in 3 % H2O2 solution. After washing with PBS, brain sections were incubated for 30 min in blocking buffer (5 % bovine serum albumin, BSA, 005 % Tween-20 in PBS). Thereafter, sections were incubated for 1 h with monoclonal BAM-10 antibody, which cross-reacts with residues 1-12 of the Abeta peptide (Sigma Aldrich, St. Louis, MO, USA), diluted 1:200 in blocking buffer. Unbound antibody was removed by three washing steps for 5 min each with washing buffer (0.05 % Tween-20 in PBS). After incubation with a secondary antibody-peroxidase conjugate (goat anti-mouse) diluted 1:500 in blocking buffer and washing steps, bound antibody was visualized by an enzyme substrate reaction with DAB (3,3'-diaminobenzidine tetrahydrochloride) as substrate applied by the DAB Enhanced liquid substrate system (Sigma Aldrich, St. Louis, MO, USA). By oxidation of DAB with the secondary antibody-coupled peroxidase, Abeta plaques were visualized by a brown precipitate. The substrate reaction was stopped by incubation with tap water. Histological sections were mounted in Poly-mount Xylene (Polysciences Inc., Warrington, PA, USA), and imaged with a DMI6000 microscope and a DFC420 camera (Leica Microsystems GmbH, Wetzlar, Germany). Plaque burden was analyzed by computerized quantitative image analysis, which quantifies brain areas (hippocampus and brain cortex) covered with Abeta AD plaques.

Similarly, hyper-phosphorylated Tau was detected with AT8 antibody on paraffin-embedded brain sections from 12 months-old Tg-TauP301L mice (Model 2508, Taconic Biosciences, Rensselaer, NY, USA) without and with treatment with Compound-1 and Compound-4 for 6 months.

### Immunoblot detection of proteins and biochemical analyses

For immunoblot detection of PHF-Tau in the hippocampus of aged 16 month-old rats subjected to the CUMS protocol for 4 weeks, hippocampi were dissected out from isolated brains on ice, pulverized under liquid nitrogen, and proteins were extracted with guanidine-hydrochloride (6.25 M guanidine hydrochloride in 50 mM Tris, pH 8.0 supplemented with 1 x protease inhibitors and 1x phosphatase inhibitors) for 30 min at 4°C. Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % beta-mercaptoethanol), and 6 M urea for 90 min at room temperature. Proteins were stored at a concentration of 0.5-1 mg/ml at -70 °C for further use. After separation of proteins by 8 M urea-containing SDS-PAGE (7.5 % polyacrylamide gel) and electrophoretic protein transfer to PVDF membranes in a tank transfer cell (Mini Trans-Blot cell, Bio-Rad GmbH, München, Germany), immunoblot detection of hyperphosphorylated PHF-tau was performed with monoclonal anti-PHF antibody (AT8, MN1020; Thermo Fisher Scientific, Waltham, MA, USA). Bound antibody was visualized with F(ab)₂ fragments of enzyme-(peroxidase-)-coupled secondary antibodies (Dianova GmbH, Hamburg, Germany) pre-absorbed to mouse serum proteins, and followed by enhanced chemiluminescent detection (ECL Plus or ECL Prime, Amersham, GE Healthcare Life Sciences, Glattbrugg, Switzerland). For quantitative analysis, quantitative immunoblot evaluation was performed. To control for equal protein loading, the total content of hippocampal Gnb was determined.

For quantitative analysis of SDS-insoluble hippocampal contents of Abeta1-40 and Abeta1-42, hippocampi were dissected out from brains isolated on ice from 18-month-old Tg2576 mice without and with treatment for 6 months with compound-1 and compound-4. Isolated hippocampi were pulverized under liquid nitrogen, and SDS-insoluble Abeta peptides were extracted by serial extraction in 14 microL/mg wet weight of Tris buffer (50 mM Tris, 200 nM NaCl, 2 mM EDTA, pH 7.2, supplemented with 1x protease inhibitors/1x phosphatase inhibitors), followed by extraction with Triton X-100-containing buffer (Tris extraction buffer with 0.1 % Triton X-100), and followed by extraction with 2 % SDS. The remaining pellet was extracted with formic acid (70 % formic acid in Tris buffer supplemented with 1x protease inhibitors/1x phosphatase inhibitors). The resulting formic-acid extract was neutralized with 1 M Tris buffer, pH 11, and used for quantitative determination of Abeta1-40 and Abeta1-42 by sandwich ELISA relative to a standard curve according to the protocol of the manufacturer (KHB3481 and KHB3441, Thermo Fisher Scientific, Waltham, MA, USA).

Neuronal cell loss and hyperphosphorylated PHF Tau were determined in hippocampi of 15 months-old Tg2576 mice, which were treated without or with compound-1 and compound-4 for three months during the neurodegeneration-enhancing CUMS (chronic unpredictable mild stress) protocol. Neuronal cell loss was determined with crude homogenates of dissected hippocampi by direct binding assay with the neuron-specific [125I]-labeled anti-NeuN antibody (MAB377, clone A60, EMD Millipore, Merck KGaA, Darmstadt, Germany). Similarly, the neuronal cell loss-causing PHF tau hyperphosphorylation was quantified with [125I]-labeled AT8 antibody. To determine neuronal cell bodies in the hippocampi of Tg2576 AD mice subjected to CUMS, hippocampi were dissected out from brains on ice, crude hippocampal homogenates were prepared (0.5 mg protein/ml PBS supplemented with 5 % BSA and 1x protease inhibitors and 1x phosphatase inhibitors), and incubated with [125I]-labelled anti-NeuN antibody (final concentration 5 x 10-8 M; 1 microCi/point) in the absence and presence of a 10-fold molar excess of unlabelled antibody (to determine non-specific binding) for 1 h at 4 °C followed by three washing steps to remove unbound antibody. Similarly, the content of hyperphosphorylated PHF-tau was determined with [125I]-labelled AT8 antibody (AbdAlla et al., J. Biol. Chem. 284, 6554-6565 (2009)). The binding assays were performed in triplicates, and specific hippocampal-bound radioactivity indicative of the content of neuronal cell bodies and hyperphosphorylated PHF tau, respectively, was determined in a gamma-counter.

### Examples

### Example 1a: General method for the identification of GRK2-inhibitory compounds, which inhibit Abeta plaque formation in the Tg2576 model of Alzheimer's disease

In the context of the present invention, the following method is disclosed for the identification of compounds, which inhibit Abeta plaque formation *in vivo,* in an AD disease model, preferably the Tg2576 AD mouse, comprising the steps of
(i) treating Tg2576 AD mice without and with the compound of interest in drinking water,
(ii) determination of Abeta plaque load in hippocampal and brain cortical areas by immunohistology with an Abeta-specific antibody,
(iii) identifying the compound of interest as an inhibitor, which inhibits the accumulation of senile AD plaques of insoluble Abeta compared to the untreated control.

It is preferred that the method for the identification of inhibitors as described above is a method wherein
(a) in step (i), the treatment is performed for 3-6 months, preferably 6 months starting at an age between 3-12 months, preferably 12 months with an orally bioavailable compound in drinking water at a dose of 1-1000 mg/kg/d, preferably 5-10 mg/kg/d; and/or
(b) the treatment in step (i) is performed with a compound, which inhibits the GRK2-mediated phosphorylation of SRSF1; and/or
(c) in step (ii) Abeta plaque load is quantified by immunohistology on paraffin sections (or cryosections) with an antibody against Abeta; and/or
(d) the identification of an inhibitor of Abeta plaque formation in step (iii) is performed by quantitative image analysis relative to the untreated control.

### Example 1b: Compounds for use in the present invention retard Abeta plaque formation in Tg2576 AD mice

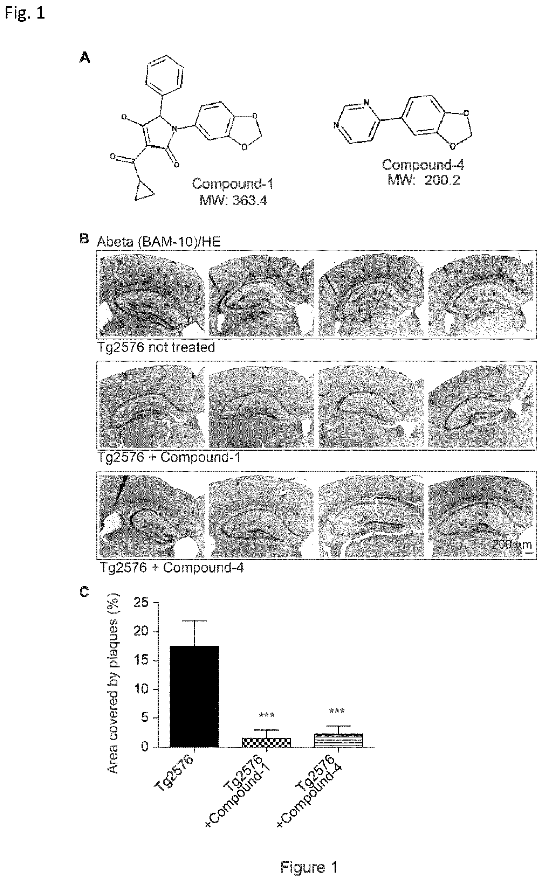

Based on their previously demonstrated utility to prevent cardiovascular disease-induced ageing, the inventors investigated the compounds of their previous and presently unpublished patent application (PCT/EP2018/050504) and determined, whether these compounds could prevent neuropathological symptoms of Alzheimer's disease (AD), which is a typical ageing-associated disease. Compound-1 and Compound-4 (see Fig. 1A for structure, also disclosed in PCT/EP2018/050504) were tested.

For Abeta plaque load quantification by immunohistology, paraffin-embedded brain sections (or cryosections) (8 microm, 10-15 sections/brain taken at 30-50 microm intervals) were prepared from brains isolated from 18-month-old Tg2576 mice (Taconic Biosciences, Rensselaer, NY, USA) treated for six months without and with Compound-1 and Compound-4 (5 mg/kg/d in drinking water). After antigen retrieval by microwave heating for 30 min in antigen retrieval buffer (10 mM sodium citrate, pH 6.0, supplemented with 0.05 % Tween-20), sections were washed with PBS, and endogenous peroxidases were inactivated by incubation for 5 min in 3 % H2O2 solution. After washing with PBS, brain sections were incubated for 30 min in blocking buffer (5 % bovine serum albumin, BSA, 005 % Tween-20 in PBS). Thereafter, sections were incubated for 1 h with monoclonal BAM-10 antibody, which cross-reacts with residues 1-12 of the Abeta peptide (Sigma Aldrich, St. Louis, MO, USA), diluted 1:200 in blocking buffer. Unbound antibody was removed by three washing steps for 5 min each with washing buffer (0.05 % Tween-20 in PBS). After incubation with a secondary antibody-peroxidase conjugate (goat anti-mouse) diluted 1:500 in blocking buffer and washing steps, bound antibody was visualized by an enzyme substrate reaction with DAB (3,3'-diaminobenzidine tetrahydrochloride) as substrate applied by the DAB Enhanced liquid substrate system (Sigma Aldrich, St. Louis, MO, USA). By oxidation of DAB with the secondary antibody-coupled peroxidase, Abeta plaques were visualized by a brown precipitate. The substrate reaction was stopped by incubation with tap water. Histological sections were mounted in Poly-mount Xylene (Polysciences Inc., Warrington, PA, USA), and imaged with a DMI6000 microscope and a DFC420 camera (Leica Microsystems GmbH, Wetzlar, Germany). Plaque burden was analysed by computerized quantitative image analysis, which quantifies brain areas (hippocampus and brain cortex) covered with Abeta AD plaques.

The immunohistological evaluation of Abeta plaque load showed that treatment with Compound-1 and Compound-4 for 6 months significantly retarded the accumulation of Abeta plaques in the hippocampus and frontal cortex of Tg2576 mice compared to untreated Tg2576 controls (Figs. 1B, C).

### Example 2a: Identification of GRK2-inhibitory compounds, which inhibit the hippocampal neuronal cell loss in Tg2576 AD mice subjected to the neurodegeneration-enhancing CUMS protocol

In the context of the present invention the following method is disclosed for the identification of compounds, which inhibit the hippocampal neuronal cell loss *in vivo,* in an AD model, preferably the Tg2576 AD mouse model, preferably subjected to the neurodegeneration-enhancing CUMS protocol, comprising the steps of
(i) subjecting aged 12-month-old Tg2576 AD mice to the neurodegeneration-enhancing CUMS (chronic unpredictable mild stress) protocol,
(ii) treating Tg2576 AD mice during the CUMS protocol without and with the compound of interest in drinking water,
(iii) determining the content of neuronal cell bodies in the hippocampus by direct binding assay with an anti-NeuN antibody, and
(iv) identifying the compound of interest as an inhibitor, which prevents hippocampal neuronal loss compared to the untreated control.

It is preferred that the method for the identification of inhibitors as described above is a method wherein
(a) in step (i), aged Tg2576 AD mice, preferably 12 month of age, are subjected to the CUMS protocol for 1-3 months, preferably 3 months; and/or
(b) in step ((ii), the treatment of Tg2576 mice is performed during the CUMS protocol for 1-3 months, preferably for 3 months starting at an age of 12 months with an orally bioavailable compound in drinking water at a dose of 1-1000 mg/kg/d, preferably 5-10 mg/kg/d; and/or
(c) the treatment in step (ii) is performed with a compound, which inhibits the GRK2-mediated phosphorylation of SRSF1; and/or
(d) in step (iii) the hippocampal neuronal cell bodies and the neuronal cell loss-causing PHF tau hyperphosphorylation are quantified by direct binding assay with [125I]-labeled anti-NeuN antibody and [125I]-labeled anti-PHF antibody.

### Example 2b: Compounds for use in the present invention retard hippocampal neuronal loss and Tau hyperphosphorylation in Tg2576 AD mice subjected to CUMS

The sole inhibition of Abeta plaque formation is probably not sufficient to retard neurodegeneration in AD patients (Kulshreshtha and Piplani, Neurol. Sci. 37, 1403-1435, 2016). In order to enhance the process of neurodegeneration in Tg2576 mice, the mice were subjected to environmental stress, which is known to aggravate symptoms of dementia and neurodegeneration in animal models and patients (AbdAlla et al., 2,3 AbdAlla et al., J. Biol. Chem. 284, 6554-6565 (2009); AbdAlla et al., J. Biol. Chem. 284, 6566-6574, 2009; Peavy et al., Biol. Psychiatry 62, 472-478 (2007); Wilson et al., Neuroepidemiology 27, 143-163 2006). Environmental stress was imposed by the chronic unpredictable mild stress (CUMS) protocol (AbdAlla et al., Biomed. Res. Int. 2015:917156, 2015; El-faramawy et al., Pharmacol. Biochem. Behav. 91, 339-344 (2009)). In addition to Tau hyperphosphorylation, environmental stress enhances neurodegeneration and induces hippocampal neuronal loss in Tg2576 mice (AbdAlla et al., J. Biol. Chem. 284, 6554-6565 (2009); AbdAlla et al., J. Biol. Chem. 284, 6566-6574, 2009).

### Detailed method

Neuronal cell loss and hyperphosphorylated PHF Tau were determined in hippocampi of 15 months-old Tg2576 mice, which were treated without or with compound-1 and compound-4 for three months during the neurodegeneration-enhancing CUMS (chronic unpredictable mild stress) protocol. Neuronal cell loss was determined with crude homogenates of dissected hippocampi by direct binding assay with the neuron-specific [125I]-labeled anti-NeuN antibody (MAB-377, clone A60, EMD Millipore, Merck KGaA, Darmstadt, Germany). Similarly, the neuronal cell loss-causing PHF tau hyperphosphorylation was quantified with [125I]-labeled AT8 antibody. To determine neuronal cell bodies in the hippocampi of Tg2576 AD mice subjected to CUMS, hippocampi were dissected out from brains on ice, crude hippocampal homogenates were prepared (0.5 mg protein/ml PBS supplemented with 5 % BSA and 1x protease inhibitors and 1x phosphatase inhibitors), and incubated with [125I]-labelled anti-NeuN antibody (final concentration 5 x 10-8 M; 1 microCi/point) in the absence and presence of a 10-fold molar excess of unlabelled antibody (to determine non-specific binding) for 1 h at 4 °C followed by three washing steps to remove unbound antibody. Similarly, the content of hyperphosphorylated PHF-tau was determined with [125I]-labelled AT8 antibody (AbdAlla et al., J. Biol. Chem. 284, 6554-6565 (2009)). The binding assays were performed in triplicates, and specific hippocampal-bound radioactivity indicative of the content of neuronal cell bodies and hyperphosphorylated PHF tau, respectively, was determined in a gamma-counter.

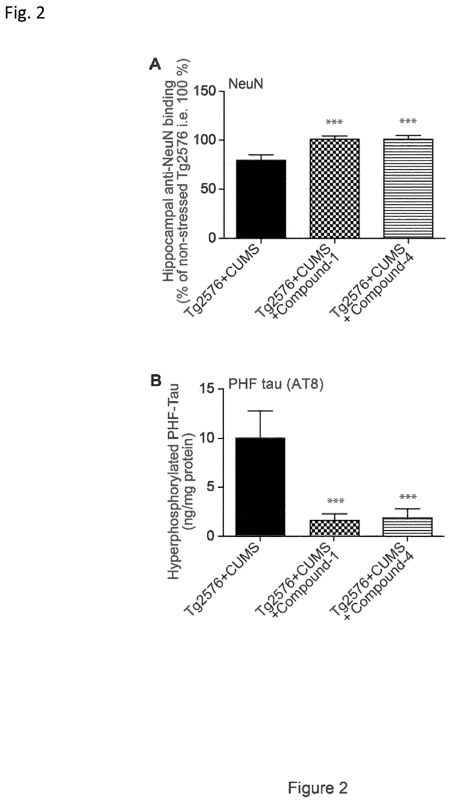

Treatment with representative Compound-1 and Compound-4 for three months significantly retarded the loss of hippocampal neurons as determined by anti-NeuN antibody binding as an indicator of neuronal loss (Fig. 2A). Concomitantly, treatment with illustrative Compound-1 and Compound-4 led to a significantly decreased hippocampal content of hyperphosphorylated PHF Tau in stressed Tg2576 mice (Fig. 2B). Thus, Compound-1 and Compound-4 retard hippocampal neuronal loss and tau hyperphosphorylation in AD mice.

### Example 3: Compounds for use in the present invention retard hippocampal Tau hyperphosphorylation in a rat model with symptoms of sporadic AD, ageing and depression

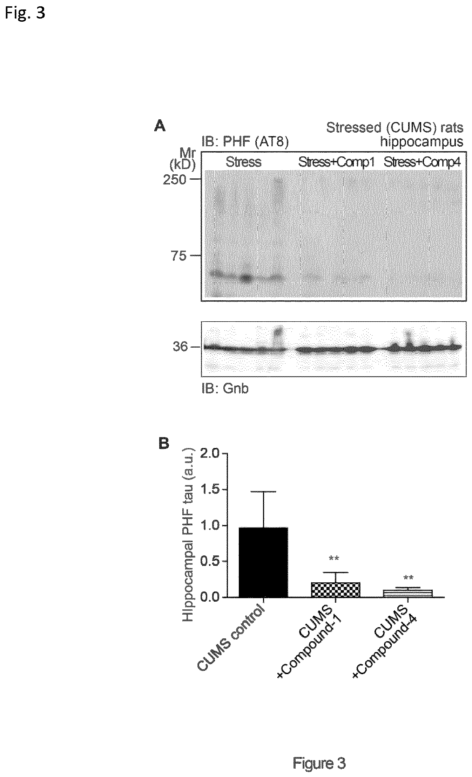

The Tg2576 mouse is a well-established genetic model of familial AD, which reproduces the gene mutation-induced generation of aggregation-prone Abeta. But the predominant late-onset sporadic AD is caused by multiple brain-insulting factors including, e.g. age, vascular and metabolic diseases, and psychiatric illnesses, which account for stress-related psychiatric syndromes. In view of the recent failure of several Abeta-targeting clinical trials (Doody et al., N. Engl. J. Med. 370, 311-321 (2014); Salloway, N. Engl. J. Med. 370, 322-333 (2014)), there is an urgent need to identify and target other (non-genetic) factors of neurodegeneration. The chronic unpredictable mild stress (CUMS) model reproduces psychological, psychosocial and physical stress as psychiatric risk factors of neurodegeneration and ageing. The CUMS protocol induces typical neuropathological features of AD such as Abeta generation and Tau hyperphosphorylation in concert with other AD markers (Briones et al., Br. J. Pharmacol. 165, 897-907 (2012)). Moreover, the sensitivity of this model increases with age, which is the best-established risk factor for AD (AbdAlla et al., Biomed. Res. Int. 2015:917156 (2015); Briones et al., Br. J. Pharmacol. 165, 897-907 (2012); El-faramawy et al., Pharmacol. Biochem. Behav. 91, 339-344 (2009)). Because the CUMS model reproduces major features of sporadic AD, the treatment effect of compounds for use in the present invention as illustrated by Compound-1 and Compound-4 was also investigated in this non-genetic model of neurodegeneration. The treatment effects were evaluated in aged 16 months-old rats subjected to the CUMS protocol for four weeks, which is sufficient to trigger symptoms of neurodegeneration (AbdAlla et al., Biomed. Res. Int. 2015:917156 (2015)). The hippocampal content of hyperphosphorylated PHF Tau as a marker of neurodegeneration was determined in immunoblot (Fig. 3A). Treatment of aged rats subjected to chronic mild stress for 4 weeks significantly retarded hippocampal Tau hyperphosphorylation as detected with PHF-specific AT8 antibody (Figure 3A,B). Thus, illustrative Compound-1 and Compound-4 both retard PHF Tau hyperphosphorylation in a rat model of neurodegeneration, which reproduces major symptoms of sporadic AD.

### Example 4: Treatment with compounds for use in the present invention retards PHF Tau hyperphosphorylation in the Tg-TauP301L model of tauopathy

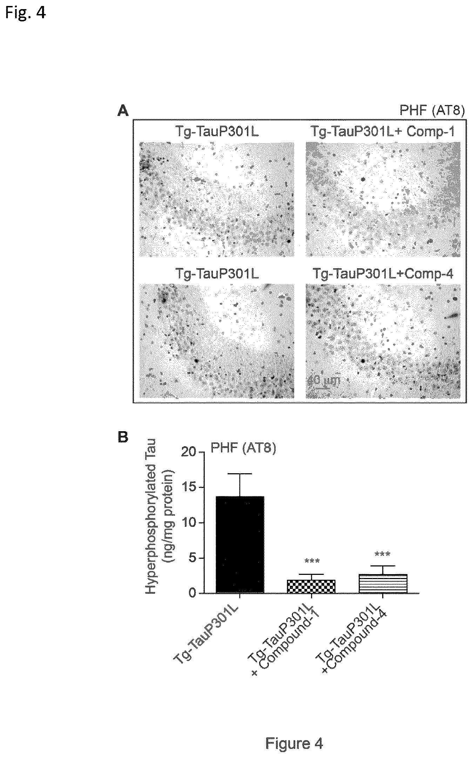

Furthermore, the compounds for use in the present invention were investigated in a genetic model of tauopathy and Tau dysfunction, i.e. Tg-TauP301L mice with neuron-specific expression of the most common FTDP-17 (frontotemporal dementia and parkinsonism linked to chromosome 17) mutation (Lewis et al., Nature Genetics 25, 402-405 (2000)). Untreated 12 months-old Tg-TauP301L mice showed prominent PHF Tau hyperphosphorylation in axons of the hippocampal CA3 area (Fig. 4A). Tau hyperphosphorylation was largely absent in age-matched Tg-TauP301L mice treated for 6 months with illustrative Compound-1 and Compound-4 (Fig. 4A). Quantitative evaluation showed that treatment with illustrative Compound-1 and Compound-4 led in both cases to a significantly decreased hippocampal content of hyperphosphorylated PHF-Tau (Fig. 4B).

### Example 5: Identification of GRK2-inhibitory compounds, which inhibit PHF tau hyperphosphorylation in the Tg-TauP301L model of tauopathy

In the context of the present invention, the following method is disclosed for the identification of compounds, which inhibit the neuronal formation of hyperphosphorylated PHF tau in vivo, in a disease model of tauopathy, preferably the Tg-TauP301L mouse model comprising the steps of
(i) treating Tg-TauP301L mice without and with the compound of interest in drinking water,
(ii) determining the content of hyperphosphorylated PHF tau in brain and hippocampal areas by immunohistology with a PHF-specific antibody,
(iii) identifying the compound of interest as an inhibitor, which inhibits the formation of hyperphosphorylated PHF-Tau compared to the untreated control.

It is preferred that the method for the identification as described above is a method wherein
(a) in step (i), the treatment is performed for 3-6 months, preferably 6 months starting at an age of 3-6 months, preferably 6 months with an orally bioavailable compound in drinking water at a dose of 1-1000 mg/kg/d, preferably 5-10 mg/kg/d; and/or
(b) the treatment in step (i) is performed with a compound, which inhibits the GRK2-mediated phosphorylation of SRSF1; and/or
(c) in step (ii), the content of hyperphosporylated PHF tau is quantified by immunohistology on paraffin sections (or cryosections) with an antibody against hyperphosphorylated PHF tau; and/or
(d) the identification of an inhibitor of hyperphosphorylated PHF tau formation in step (iii) is performed by quantitative image analysis.

### Detailed method

For detection of hyper-phosphorylated PHF Tau by immunohistology, paraffin-embedded brain sections (or cryosections) (8 microm, 10-15 sections/brain taken at 30-50 microm intervals) were prepared from brains isolated from 12-month-old Tg-TauP301L taupathy model mice (Model 2508; Taconic Biosciences, Rensselaer, NY, USA) without and with 6 months of treatment with Compound-1 and Compound-4 (5 mg/kg body-weight/day in drinking water). After antigen retrieval by microwave heating for 30 min in antigen retrieval buffer (10 mM sodium citrate, pH 6.0, supplemented with 0.05 % Tween-20), histological sections were washed with PBS, and endogenous peroxidases were inactivated by incubation for 5 min in 3 % H2O2 solution. After washing with PBS, brain sections were incubated for 30 min in blocking buffer (5 % bovine serum albumin, BSA, 0.05 % Tween-20 in PBS). Thereafter, sections were incubated for 1 h with monoclonal AT8 antibody diluted 1:200 in blocking buffer, which detects the PHF-form of hyperphosphorylated tau (Sigma Aldrich, St. Louis, MO, USA). Unbound antibody was removed by three washing steps for 5 min each with washing buffer (0.05 % Tween-20 in PBS). After incubation with a secondary antibody-peroxidase conjugate (goat anti-mouse) diluted 1:500 in blocking buffer and washing steps, bound antibody was visualized by an enzyme substrate reaction with the DAB (3,3'-diaminobenzidine tetrahydrochloride) as substrate applied by the DAB Enhanced liquid substrate system (Sigma Aldrich, St. Louis, MO, USA). By oxidation of DAB with the seconddary antibody-coupled peroxidase, hyperphosphorylated PHF tau was visualized by a brown precipitate. The substrate reaction was stopped by incubation with tap water. Histological sections were mounted in Poly-mount-Xylene (Polysciences Inc., Warrington, PA, USA), and imaged with a DMI6000 microscope and a DFC420 camera (Leica Microsystems GmbH, Wetzlar, Germany). The content of hyperphosphorylated PHF tau was analysed by computerized quantitative image analysis, which quantifies areas stained positive for PHF-tau.

### Example 6: Identification of GRK2-inhibitory compounds, which inhibit PHF tau hyperphosphorylation in a rat model of depression with symptoms of early sporadic AD

In the context of the present invention, the following method is disclosed for the identification of compounds, which inhibit the neuronal accumulation of hyperphosphorylated PHF tau *in vivo,* in a disease model of depression with symptoms of early sporadic AD, preferably the chronic unpredictable mild stress model (CUMS) comprising the steps of
(i) treating aged rats subjected to the CUMS protocol without and with the compound of interest in drinking water,
(ii) determining the content of hyperphosphorylated PHF tau in the hippocampus by immunblot detection with a PHF-specific antibody,
(iii) identifying the compound of interest as an inhibitor, which inhibits the formation of hyperphosphorylated PHF-Tau compared to the untreated control.

It is preferred that the method for the identification of compounds as described above is a method wherein
(a) in step (i), the treatment is performed for 1-3 months, preferably 1 month during the CUMS protocol starting at an age of 13-18 months, preferably 15 months with an orally bioavailable compound in drinking water at a dose of 1-1000 mg/kg/d, preferably 5-10 mg/kg/d; and/or
(b) the treatment in step (i) is performed with a compound, which inhibits the GRK2-mediated phosphorylation of SRSF1; and/or
(c) in step (ii), the content of hyperphosporylated PHF tau is quantified by immunoblot detection in hippocampal lysates with an antibody against hyperphosphorylated PHF tau.

### Detailed method

For immunoblot detection of PHF-Tau in the hippocampus of aged 16 month-old rats subjected to the CUMS protocol for 4 weeks, hippocampi were dissected out from isolated brains on ice, pulverized under liquid nitrogen, and proteins were extracted with guanidine-hydrochloride (6.25 M guanidine hydrochloride in 50 mM Tris, pH 8.0 supplemented with 1x protease inhibitors and 1x phosphatase inhibitors) for 30 min at 4°C. Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % beta-mercaptoethanol), and 6 M urea for 90 min at room temperature. Proteins were stored at a concentration of 0.5-1 mg/ml at -70 °C for further use. After separation of proteins by 8 M urea-containing SDS-PAGE (7.5 % polyacrylamide gel) and electrophoretic protein transfer to PVDF membranes in a tank transfer cell (Mini Trans-Blot cell, Bio-Rad GmbH, München, Germany), immunoblot detection of hyperphosphorylated PHF-tau was performed with monoclonal anti-PHF antibody (AT8, MN1020; Thermo Fisher Scientific, Waltham, MA, USA). Bound antibody was visualized with F(ab)₂ fragments of enzyme-(peroxidase-)-coupled secondary antibodies (Dianova GmbH, Hamburg, Germany) pre-absorbed to mouse serum proteins, and followed by enhanced chemiluminescent detection (ECL Plus or ECL Prime, Amersham, GE Healthcare Life Sciences, Glattbrugg, Switzerland). For quantitative analysis, quantitative immunoblot evaluation was performed. To control for equal protein loading, the total content of hippocampal Gnb was determined, which is the Gbeta subunit of heterotrimeric G-proteins.

### Example 7: Identification of GRK2-inhibitory compounds, which inhibit the hippocampal accumulation of SDS-insoluble Abeta peptides, Abetal-40 and Abeta1-42

In the context of the present invention, the following method is disclosed for the identification of compounds, which inhibit the hippocampal accumulation of SDS-insoluble Abeta peptides, Abeta1-40 and Abeta1-42, *in vivo,* in an AD disease model, preferably the Tg2576 AD mouse comprising the steps of
(i) treating Tg2576 AD mice without and with the compound of interest in drinking water
(ii) determination of the content of SDS-insoluble Abeta peptides in hippocampal and/or brain cortical areas by sandwich ELISA
(iii) identifying the compound of interest as an inhibitor, which inhibits the hippocampal accumulation of insoluble Abeta peptides compared to the untreated control.

It is preferred that the method for the identification of inhibitors as described above is a method wherein
(a) in step (i), the treatment is performed for 3-12 months, preferably 6 months starting at an age of 3-12 months, preferably 12 months with an orally bioavailable compound in drinking water at a dose of 1-1000 mg/kg/d, preferably 5-10 mg/kg/d; and/or
(b) the treatment in step (i) is performed with a compound, which inhibits the GRK2-mediated phosphorylation of SRSF1; and/or
(c) in step (ii) the hippocampal and/or brain content of SDS-insoluble Abeta1-40 and Abeta1-42 is quantified with a sandwich ELISA specific for Abeta1-40 and Abeta1-42.

### Detailed method

For quantitative analysis of SDS-insoluble hippocampal contents of Abeta1-40 and Abeta1-42, hippocampi were dissected out from brains isolated on ice from 18-month-old Tg2576 mice without and with treatment for 6 months with compound-1 and compound-4. Isolated hippocampi were pulverized under liquid nitrogen, and SDS-insoluble Abeta peptides were extracted by serial extraction in 14 microL/mg wet weight of Tris buffer (50 mM Tris, 200 mM NaCl, 2 mM EDTA, pH 7.2, supplemented with 1x protease inhibitors/1x phosphatase inhibitors), followed by extraction with Triton X-100-containing buffer (Tris extraction buffer with 0.1 % Triton X-100), and followed by extraction with 2 % SDS. The remaining pellet was extracted with formic acid (70 % formic acid in Tris buffer supplemented with 1x protease inhibitors/1x phosphatase inhibitors). The resulting formic-acid extract was neutralized with 1 M Tris buffer, pH 11, and used for quantitative determination of Abeta1-40 and Abeta1-42 by sandwich ELISA and with a standard curve according to the protocol of the manufacturer (KHB3481 and KHB3441, Thermo Fisher Scientific, Waltham, MA, USA).

### Example 8: Method for identification of compounds, which inhibit the GRK2-mediated phosphorylation assay of SRSF1

In the context of the present invention the following method is disclosed for the identification of inhibitors of the G-protein-coupled receptor kinase 2 (GRK2), preferably by determination of the GRK2-mediated phosphorylation of the serine/arginine-rich splicing factor 1 (SRSF1) comprising the steps of
(i) providing and incubating GRK2 and SRSF1 under physiological conditions suitable for the phosphorylation of SRSF1 in the presence and in the absence of a compound of interest;
(ii) determining the phosphorylation of SRSF1 in the presence and in the absence of the compound of interest;
(iii) identifying the compound of interest as an inhibitor or non-inhibitor based on the phosphorylation of SRSF1 in the presence of the compound of interest relative to the phosphorylation of SRSF1 in the absence of the compound of interest.

It is preferred that the method for the identification as described above is a method wherein
(a) in step (i), the incubation is performed in the presence of radioactively labelled ATP, preferably [gamma-³²P]ATP, preferably at about 25 to 37 °C for about 30 to 90 min; and/or
(b) the incubation of step (i) is stopped by dilution at temperatures below 30 °C, preferably at temperatures of about 0 to 10 °C; and/or
(c) the determination of the phosphorylation of SRSF1 in step (ii) is performed by (A) filtering the product of (i) through a filter, preferably a glass fiber filter; (B) washing the filter; and (C) determining the filter-bound radioactivity, preferably with a beta-counter.

### Detailed method

For the identification of small molecule inhibitors of the (GRK2)-mediated phosphorylation of (SRSF1), the phosphorylation assay was performed in a reaction buffer (e.g. 20 mM Tris, 2 mM EDTA, 5 mM MgCI2, 0.05 % BSA, pH 7.5,) supplemented with ATP, preferably about 50 microM, [gamma-32P]-ATP (e.g. 1x 10 6 DPM, specific activity of about 3000 Ci/mmol)) and about 300-500 nM of SRSF1. The reaction mixture was added to GRK2 (e.g. about 100 nM in reaction buffer, without or with increasing concentrations of the small molecule compound) to give a final reaction volume of, e.g. about 50 microL. After an incubation for e.g. about 30-60 min at about 30 °C, the phosphorylation was stopped by the addition of ice-cold reaction buffer, preferably about 5 volumes. The reaction mixture was immediately applied to filters, preferably glass fiber filters. After three washing steps, e.g. with about 5 ml of reaction buffer, filter-bound radioactivity was determined in a beta-counter.

### Conclusions on the experimental data

The above data show that compounds for use in the present invention, which also retard cardiovascular disease-induced ageing (presently unpublished PCT/EP2018/050504), retard the formation of Abeta plaques, Tau hyperphosphorylation and hippocampal neuronal loss as major hallmarks of AD and neurodegeneration in different animal models of neurodegenerative diseases and tauopathies, i.e. the Tg2576 genetic model of AD, the non-genetic CUMS model of neurodegeneration with features of sporadic AD, ageing and depression, and the Tg-TauP301L genetic model of tauopathy. Without wishing to be bound by theory, it is assumed that neuroprotective mechanisms induced by these compounds could involve inhibition of mitochondrial dysfunction caused by GRK2 (Sato et al., J. Mol. Cell. Cardiol 89, 360-364 (2015)), and retardation of SRSF1-induced symptoms of ageing (Harthouri et al., EMBO Mol. Med. 9, 1294-1313 (2017)).

## Claims

1. A compound according to Formula (I) or (II): 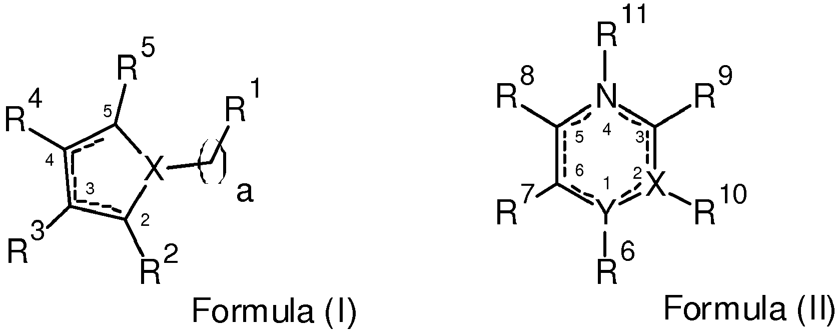 wherein:
the dotted lines between positions (2), (3), (4) and (5) in Formula (I) and between positions (1), (2), (3), (4), (5) and (6) in Formula (II) represent single bonds or double bonds between the respective positions;
**X** is selected from the group consisting of N and C;
**Y** is selected from the group consisting of S and C, with the proviso that when **Y** is C, **X** is N;
**a** is an integer between 0 and 15, preferably between 0 and 10, more preferably between 0 and 5, most preferably is 0 or 1;
**R¹** is selected from the group consisting of
(i) hydrogen, hydroxyl, F, Cl, Br and oxo, preferably if **X** is not N or if **X** is N and **a** is not 0, **R¹** is selected from the group consisting of hydroxyl, F, Cl, Br and oxo;
(ii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
(iii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
(iv) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a *para-*substituted phenyl that is substituted by a substituent selected from the group consisting of Cl, F, Br, substituted or non-substituted methyl, preferably -(CF₃), ethyl, propyl and cyclopropyl; and
(v) substituted or non-substituted (C₃₋₆)heterocycle and (C₇₋C₁₀)carbo- or heterobicycle having 1 to 3 heteroatoms each independently selected from N, O and S, preferably substituted or non-substituted (C₇)heterobicycle having 2 heteroatoms selected from N and S, more preferably substituted or non-substituted indazolyl, benzimidazolyl and benzodioxolyl, preferably indazolyl, benzimidazolyl and benzodioxolyl connected via position (5) or (6), more preferably via position (6) of the indazolyl or benzodioxolyl or position (5) of the benzimidazolyl;
**R²** is selected from the group consisting of
(i) hydrogen, hydroxyl, O-**R¹⁴**, -O-C(=O)-**R¹⁴**, F, Cl, Br and oxo wherein **R¹⁴** is selected from the group consisting of
(aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
(bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in *para* position by (C₃)carbocycle or -(CF₃) or di-substituted in *meta* position by (C₃)carbocycle or -(CF₃); and
(cc) substituted or non-substituted aromatic or non-aromatic, preferably aromatic, (C₃₋₆)heterocycle having 1 to 3 heteroatoms each independently selected from N, O and S;
(ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl, and (C₃₋₁₀)carbocycle, preferably (C₃₋₆)cylcoalkyl;
(iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether; and
(iv) substituted or non-substituted (C₃₋₆)heterocycle and (C₇-C₁₀)carbo- or heterobicycle having 1 to 3 heteroatoms each independently selected from N, O and S, preferably substituted or non-substituted (C₇)heterobicycle having 2 heteroatoms selected from N and S, more preferably substituted or non-substituted indazolyl, benzimidazolyl and benzodioxolyl, preferably indazolyl, benzimidazolyl and benzodioxolyl connected via position (5) or (6) of the indazolyl, benzodioxolyl or benzimidazolyl;
**R³** and **R⁴** are independently selected from the group consisting of
(i) hydrogen, hydroxyl, -O-**R¹⁴**, -O-C(=O)-**R¹⁴**, F, Cl, Br and oxo, wherein **R¹⁴** is selected from the group consisting of
(aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
(bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in *para* position by (C₃)carbocycle or -(CF₃) or di-substituted in *meta* position by (C₃)carbocycle or -(CF₃); and
(cc) substituted or non-substituted aromatic or non-aromatic, preferably aromatic, (C₃₋₆)heterocycle having 1 to 3 heteroatoms each independently selected from N, O and S;
(ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl and (C₃₋₁₀)carbocycle, preferably substituted or non-substituted (C₃₋₆)cylcoalkyl and (C₃₋₆)heterocycle having 1 to 3 heteroatoms each independently selected from N, O and S;
(iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
(iv) 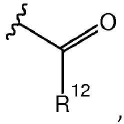 wherein **R¹²** is selected from the group consisting of
(aa) hydrogen, hydroxyl, substituted or non-substituted N, F, Cl and Br;
(bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
(cc) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in *para* position by (C₃)carbocycle or -(CF₃) or di-substituted in *meta* position by (C₃)carbocycle or -(CF₃); and
(dd) substituted or non-substituted aromatic or non-aromatic, preferably aromatic, (C₃₋₆)heterocycle having 1 to 3 heteroatoms each independently selected from N, O and S; and
(v) 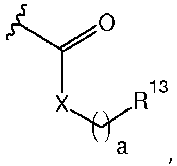 wherein X is N or C, a is an integer between 0 and 15, preferably between 0 and 10, more preferably between 0 and 5, most preferably is 0 or 1, and **R¹³** is selected from the group consisting of
(aa) hydrogen, hydroxyl, F, Cl and Br;
(bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
(cc) substituted or non-substituted (C₃₋₁₀)carbocycle, preferably (C₃₋₆)cylcoalkyl, (C₇-C₁₀)carbo- or heterobicycle and (C₃₋₆)heterocycle having 1 to 3 heteroatoms each independently selected from N, O and S,
more preferably, for **R³, R¹³** is (C₇)heterobicycle having 2 heteroatoms selected from N and S, most preferably substituted or non-substituted indazolyl, benzimidazolyl and benzodioxolyl, preferably indazolyl, benzimidazolyl and benzodioxolyl connected via position (5) or (6), more preferably via position (5) of the indazolyl and benzodioxolyl or position (6) of the benzimidazolyl, and most preferably, for **R⁴, R¹³** is substituted or non-substituted aromatic (C₆)carbocycle, preferably (C₆)carbocycle that is mono- or di-substituted in *meta* position by (C₃)-carbocycle or -(CF₃), or mono-substituted in *para* position by (C₃)-carbocycle or -(CF₃); and
(dd) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
wherein, if positions (2), (3) and/or (4) of the ring of Formula (I) are sp³-hybridized, **R²** and **R⁴** and/or **R³** and **R⁴** are preferably in *cis* or *trans* configuration to each other, more preferably in *trans* configuration, preferably, **R²** is (*R*)- or (*S*)-, **R³** is (*R*)- or (*S*)- and/or **R⁴** is (*R*)- or (*S*)-configured, more preferably, **R²** is (*R*)-, **R³** is (*R*)- and/or **R⁴** is (*R*)-configured, or **R²** is (*S*)-, **R³** is (*S*)- and/or **R⁴** is (*S*)-configured.
**R⁵** and **R⁹** are selected from the group consisting of
(i) hydrogen, hydroxyl, F, Cl, Br and oxo, with the proviso that **R⁹** is not oxo if **X** is N and **Y** is C;
(ii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
(iii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
(iv) substituted or non-substituted (C₃₋₁₀)carbocycle, preferably substituted or non-substituted (C₃)carbocycle, substituted or non-substituted aromatic (C₅₋₆)carbocycle, more preferably cyclopenta-2,4-dien-1-yl and aromatic (C₆)carbocycle, most preferably phenyl that is non-substituted or substituted in *para* position by a substituent selected from the group consisting of Cl, F, Br, substituted or non-substituted methyl, preferably -(CF₃), ethyl, propyl and cyclopropyl; and
(v) (C₃₋₆)heterocycle having 1 to 3 heteroatoms each independently selected from N, O and S, preferably substituted or non-substituted imidazolyl and pyrazolyl, more preferably imidazolyl and pyrazolyl connected via imidazolyl-/pyrazolyl-position-(1)-nitrogen to the rings of Formula (I);
wherein, if position (5) of the ring of Formula (I) is sp³-hybridized, **R⁵** is preferably (*S*)- or (*R*)-configured, more preferably (*R*)-configured;
and wherein, if position (3) of the ring of Formula (II) is sp³-hybridized, **R⁹** is preferably (*S*)- or (*R*)-configured, more preferably (*S*)-configured;
**R⁶** and **R¹¹** are independently selected from the group consisting of
(i) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
(ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
(iii) substituted or non-substituted carbocycle selected from the group consisting of (C₃₋₁₀)carbocycle, preferably (C₃)carbocycle and (C₅₋₆)carbocycle, more preferably aromatic (C₆)carbocycle, most preferably phenyl that is non-substituted or mono- or di-substituted in *meta* and *para* position by a substituent selected from the group consisting of Cl, F, Br, substituted or non-substituted methyl, ethyl, propyl and cyclopropyl; and
(iv) substituted or non-substituted (C₃₋₆)heterocycle and (C₇-C₁₀)carbo- or heterobicycle having 1 to 3 heteroatoms each independently selected from N, O and S, preferably substituted or non-substituted (C₇)heterobicycle having 2 heteroatoms selected from N and S, most preferably substituted or non-substituted indazolyl, benzimidazolyl and benzodioxolyl, preferably indazolyl, benzimidazolyl and benzodioxolyl connected via position (5) or (6), more preferably via position (6) of the indazolyl or benzodioxolyl or position (5) of the benzimidazolyl,
wherein **R⁶** is not present if **Y** is S; and/or
wherein **R¹¹** is absent if the ring of Formula (II) has a double bond between positions (4) and (5) or between positions (3) and (4) of the ring of Formula (II), and with the proviso that **R⁶** is not 1,2,4-triazolyl if **X** is N, **Y** is C and the ring of Formula (II) is aromatic;
**R⁷** is selected from the group consisting of
(i) hydrogen, hydroxyl, F, Cl, Br and oxo;
(ii) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂₋₁₀)alkynyl and(C₃-₁₀)carbocycle, preferably (C₃₋₆)cylcoalkyl and (C₃₋₆)heterocycle having 1 to 3 heteroatoms each independently selected from N, O and S;
(iii) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether; and
(iv) 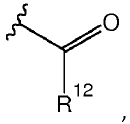 wherein **R¹²** is selected from the group consisting of
(aa) hydrogen, hydroxyl, substituted or non-substituted N, F, Cl and Br;
(bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, (C₂-₁₀)alkynyl and aromatic or non-aromatic (C₃₋₁₀)carbocycle, preferably (C₃₋₆)cylcoalkyl, more preferably (C₃)carbocycle, most preferably aromatic (C₆)carbocycle that is mono-substituted in *para* position by (C₃)carbocycle or -(CF₃) or di-substituted in *meta* position by (C₃)carbocycle or -(CF₃); and
(cc) substituted or non-substituted, aromatic or non-aromatic, preferably aromatic, (C₃₋₆)heterocycle having 1 to 3 heteroatoms each independently selected from N, O and S;
**R⁸** is selected from the group defined above for **R⁷,** the group further comprising 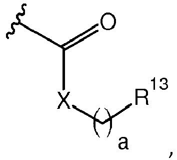 wherein **X** is N or C, **a** is an integer between 0 and 15, preferably between 0 and 10, more preferably between 0 and 5, most preferably is 0 or 1, and **R¹³** is selected from the group consisting of
(aa) hydrogen, hydroxyl, F, Cl and Br;
(bb) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl and (C₂₋₁₀)alkynyl;
(cc) substituted or non-substituted (C₃₋₁₀)carbocycle, preferably (C₃₋₆)cylcoalkyl, (C₇-C₁₀)carbo- or heterobicycle and (C₃₋₆)heterocycle having 1 to 3 heteroatoms each independently selected from N, O and S,
more preferably substituted or non-substituted (C₇)heterobicycle having 2 heteroatoms selected from N and S, most preferably substituted or non-substituted indazolyl, benzimidazolyl and benzodioxolyl, preferably indazolyl and benzodioxolyl connected via position (5) or (6), more preferably via position (5) of the indazolyl and benzodioxolyl or position (6) of the benzimidazolyl,
most preferably substituted or non-substituted aromatic (C₆)carbocycle, preferably (C₆)carbocycle that is mono- or di-substituted in *meta* position by (C₃)-carbocycle or -(CF₃) or mono-substituted in *para* position by (C₃)-carbocycle or -(CF₃); and
(dd) linear or branched, substituted or non-substituted (C₁₋₁₀)alkyl ether, (C₂₋₁₀)alkenyl ether, (C₂₋₁₀)alkynyl ether and (C₄₋₁₀)carbocyclic ether;
with the proviso that **R⁸** is not 1,2,4-triazolyl if **X** is N, **Y** is C and the ring of Formula (II) is aromatic,
and wherein, if position (5) of the ring of Formula (II) is sp³-hybridized, **R⁸** is preferably (*R*)- or (*S*)-configured, more preferably (*R*)-configured;
**R¹⁰** is absent or selected from the group consisting of
(i) hydrogen;
(ii) methyl; and
(iii) cyclopropyl or phenyl that is mono-substituted in *para* position by a substituent selected from the group consisting of H, Cl, F, Br, methyl, -(CF₃) and cyclopropyl;
wherein one or more of **R², R³, R⁴, R⁵, R⁶, R⁷, R⁸, R⁹, R¹⁰** and **R¹¹** are either directly attached to the rings of Formulas (I) or (II) or are attached to a linker between **R², R³, R⁴, R⁵, R⁶, R⁷, R⁸, R⁹, R¹⁰** and/or **R¹¹** and the rings of Formulas (I) or (II), wherein the linker is selected from the group consisting of linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether, linear or branched, substituted or non-substituted (C₁₋₁₀)alkyl, (C₂₋₁₀)alkenyl and (C₂₋₁₀)alkynyl; and pharmaceutically acceptable salts or solvates thereof;
for use in the medical treatment of CNS- and neurodegenerative diseases.

2. The compound for use according to claim 1, wherein
in Formula (I), a double bond is present between positions (3) and (4), or between positions (2) and (3) and between positions (4) and (5), or no double bond is present in the ring; and
in Formula (II), no double bond is present in the ring or the ring is aromatic;
and/or
a is 0 or 1;
and/or
**R¹** is selected from the group consisting of
(i) hydrogen;
(ii) linear or branched, substituted or non-substituted (C₁₋₅)alkyl, more preferably methyl, ethyl and propyl, most preferably methyl;
(iii) substituted or non-substituted cyclopropyl and phenyl, preferably substituted phenyl, more preferably phenyl that is mono-substituted in *para* position by a substituent selected from the group consisting of H, Cl, F, Br, methyl, -(CF₃) and cyclopropyl; and
(iv) substituted or non-substituted, preferably non-substituted indazolyl, benzimidazolyl and benzodioxolyl connected via position (5) or (6), preferably via position (6) of the indazolyl and benzodioxolyl or position (5) of the benzimidazolyl.

3. The compound for use according to claim 1 or 2, wherein
**R²** is selected from the group consisting of
(i) hydrogen or oxo;
(ii) linear or branched, substituted or non-substituted (C₁₋₅)alkyl, more preferably methyl, ethyl and propyl, most preferably methyl; and
(iii) substituted or non-substituted indazolyl, benzimidazolyl and benzodioxolyl, preferably indazolyl, benzimidazolyl and benzodioxolyl connected via position (5) or (6) of the indazolyl, benzodioxolyl and benzimidazolyl; and/or
**R³** is selected from the group consisting of
(i) hydrogen;
(ii) linear or branched, substituted or non-substituted (C₁₋₅)alkyl, more preferably methyl, ethyl and propyl, most preferably methyl;
(iii) 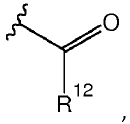 wherein **R¹²** is selected from the group consisting of
(aa) N; and
(bb) substituted or non-substituted cyclopropyl and phenyl, preferably phenyl that is mono-substituted in *para* position by cyclopropyl or -(CF₃) or di-substituted in *meta* position by cyclopropyl or -(CF₃) in each *meta* position; and
(iv) 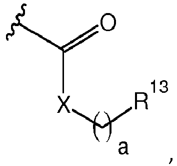 wherein **X** is N, **a** is 1 and **R¹³** is selected from the group consisting of substituted or non-substituted, preferably non-substituted indazolyl, benzimidazolyl and benzodioxolyl connected via position (6) or (5) of indazolyl, benzimidazolyl and benzodioxolyl, preferably via position (5) of the indazolyl and benzodioxolyl or position (6) of the benzimidazolyl; and/or
**R⁴** is selected from the group consisting of
(i) hydrogen and hydroxyl;
(ii) linear or branched, substituted or non-substituted (C₁₋₅)alkyl, more preferably methyl, ethyl and propyl, most preferably methyl;
(iii) 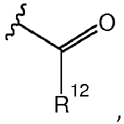 wherein **R¹²** is selected from the group consisting of
(aa) N; and
(bb) substituted or non-substituted cyclopropyl and phenyl, preferably phenyl that is mono-substituted in *para* or *meta* position by cyclopropyl or -(CF₃), or di-substituted in *meta* position by cyclopropyl or -(CF₃) in each *meta* position; and
(iv) 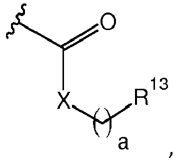 wherein **X** is N, **a** is 1 and **R¹³** is phenyl that is mono- or di-substituted in each *meta* position by cyclopropyl or -(CF₃) or mono-substituted *in para* position by cyclopropyl or -(CF₃);
wherein, if positions (2), (3) and/or (4) of the ring of Formula (I) are sp³-hybridized **R²** and **R⁴** and/or **R³** and **R⁴** are preferably in *cis* or *trans* configuration to each other, more preferably in *trans* configuration, preferably, **R²** is (*R*)- or (*S*)-, **R³** is (*R*)- or (*S*)- and/or **R⁴** is (*R*)- or (*S*)-configured, more preferably, **R²** is (*R*)-, **R³** is (*R*)- and/or **R⁴** is (*R*)-configured, or **R²** is (*S*)-, **R³** is (*S*)- and/or **R⁴** is (*S*)-configured;
and/or
**R⁵** is selected from the group consisting of
(i) hydrogen;
(ii) linear or branched, substituted or non-substituted (C₁₋₅)alkyl, more preferably methyl, ethyl and propyl, most preferably methyl;
(iii) substituted or non-substituted cyclopropyl and phenyl, preferably substituted phenyl, more preferably phenyl that is mono-substituted in *para* position by a substituent selected from the group consisting of H, Cl, F, Br, methyl, -(CF₃) and cyclopropyl;
(iv) cyclopenta-2,4-dien-1-yl; and
(v) substituted or non-substituted, preferably non-substituted imidazolyl and pyrazolyl connected via the imidazolyl-/pyrazolyl-position-(1)-nitrogen to the ring of Formula (I);
wherein, if position (5) of the ring of Formula (I) is sp³-hybridized, **R⁵** is preferably (*R*)- or (*S*)-configured, more preferably (*R*)-configured;
and/or
**R⁶** and **R¹¹** are independently selected from the group consisting of substituted or non-substituted, preferably non-substituted indazolyl, benzimidazolyl and benzodioxolyl connected via position (6) or (5), preferably via position (6) of the indazolyl and benzodioxolyl or position (5) of the benzimidazolyl;
wherein **R⁶** is not present if **Y** is S; and/or
wherein **R¹¹** is absent if the ring of Formula (II) has a double bond between positions (4) and (5) or between positions (3) and (4) of the ring of Formula (II);
and/or
**R⁷** and **R⁸** are independently selected from the group consisting of
(i) hydrogen;
(ii) linear or branched, substituted or non-substituted (C₁₋₅)alkyl, more preferably methyl, ethyl and propyl, most preferably methyl; and
(iii) 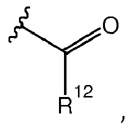 wherein **R¹²** is selected from the group consisting of
(aa) N; and
(bb) substituted or non-substituted cyclopropyl and phenyl, preferably phenyl that is mono-substituted in *para* position by cyclopropyl or -(CF₃), or di-substituted in *meta* position by cyclopropyl or -(CF₃) in each *meta* position;
wherein, for **R⁸,** the group further comprises 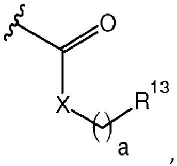 wherein **X** is N, **a** is 1 and **R¹³** is selected from the group consisting of substituted or non-substituted, preferably non-substituted indazolyl, benzimidazolyl and benzodioxolyl connected via position (6) or (5), preferably via position (5) of the indazolyl and benzodioxolyl or position (6) of the benzimidazolyl; and
wherein, if position (5) of the ring of Formula (II) is sp³-hybridized, **R⁸** is preferably (*R*)- or (*S*)-configured, more preferably (*R*)-configured;
and/or
**R⁹** is selected from the group consisting of
(i) hydrogen, methyl and cyclopropyl; and
(ii) substituted or non-substituted phenyl, preferably phenyl that is substituted in *para* position by a substituent selected from the group consisting of H, Cl, F, Br, methyl, -(CF₃) and cyclopropyl; and
wherein, if position (3) of the ring of Formula (II) is sp³-hybridized, **R⁹** is preferably (*R*)- or (*S*)- configured, more preferably (*S*)-configured;
and/or
**R¹⁰** is absent or selected from the group consisting of
(i) hydrogen;
(ii) methyl; and
(iii) cyclopropyl and phenyl that is mono-substituted in *para* position by a substituent selected from the group consisting of H, Cl, F, Br, methyl, -(CF₃) and cyclopropyl,
wherein **R¹⁰** is absent if **X** is C and/or if the ring of Formula (II) has a double bond between positions (1) and (2) or between positions (2) and (3) of the ring of Formula (II).

4. The compound for use according to any of claims 1 to 3, wherein the compound is a compound of Formula (I) and a double bond is located between positions (3) and (4) (Formula la) 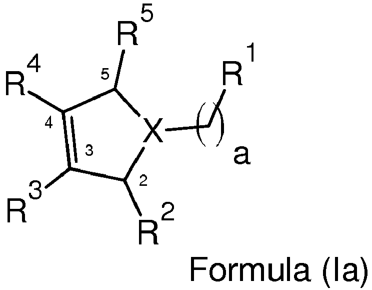 wherein **X** is N and **a** is 0, and wherein
**R¹** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R²** is oxo; and/or
**R³** is selected from the group consisting of
(i) hydrogen;
(ii) methyl; and
(iii) 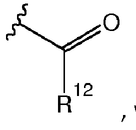 wherein **R¹²** is selected from the group consisting of
(aa) N; and
(bb) cyclopropyl and phenyl that is mono-substituted in *para* position by cyclopropyl or -(CF₃), or di-substituted in *meta* position by cyclopropyl or -(CF₃) in each *meta* position;
**R⁴** and/or is hydroxyl; and/or
**R⁵** is selected from the group consisting of
(i) hydrogen;
(ii) methyl;
(iii) cyclopropyl and phenyl that is mono-substituted in *para* position by a substituent selected from the group consisting of H, Cl, F, Br, methyl, -(CF₃) and cyclopropyl;
(iv) cyclopenta-2,4-dien-1-yl; and
(v) imidazolyl and pyrazolyl connected via the imidazolyl-/pyrazolyl-position-(1)-nitrogen to the ring of Formula (I);
wherein **R⁵** is preferably (*R*)- or (*S*)-configured, more preferably (*R*)-configured.

5. The compound for use according to any of claims 1 to 3, wherein the compound is a compound of Formula (I) and two double bonds are located between positions (2) and (3) and between positions (4) and (5), respectively (Formula Ib) 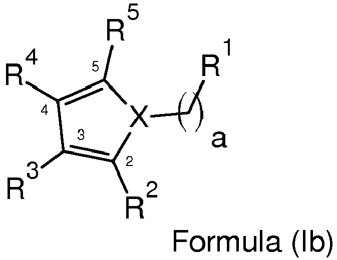 wherein **X** is N or C, preferably N, **a** is 0 or 1, preferably **a** is 0 if **X** is C, and wherein
**R¹** is selected from the group consisting of indazolyl, benzimidazolyl and benzodioxolyl connected via position (5) or (6), preferably via position (6) of the indazolyl and benzodioxolyl or position (5) of the benzimidazolyl; and/or
**R²** is selected from the group consisting of
(i) hydrogen; and
(ii) methyl; and/or
**R³** is selected from the group consisting of
(i) hydrogen;
(ii) methyl; and
(iii) 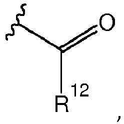 wherein **R¹²** is selected from the group consisting of
(aa) N; and
(bb) cyclopropyl and phenyl that is mono-substituted in *para* position by cyclopropyl or -(CF₃), or di-substituted in *meta* position by cyclopropyl or -(CF₃) in each *meta* position; and/or
**R⁴** is hydrogen; and/or
**R⁵** is selected from the group consisting of
(i) hydrogen;
(ii) methyl; and
(iii) cyclopropyl and phenyl that is mono-substituted in *para* position by a substituent selected from the group consisting of H, Cl, F, Br, methyl, -(CF₃) and cyclopropyl.

6. The compound for use according to any of claims 1 to 3, wherein the compound is a compound of Formula (I) and the bonds in the five-membered ring of Formula (I) are fully saturated (Formula Ic) 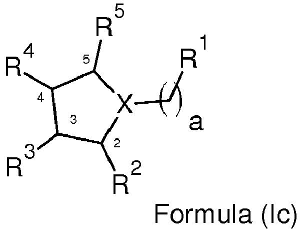 wherein **X** is N, **a** is 0 and wherein
**R¹** is selected from the group consisting of
(i) hydrogen;
(ii) methyl; and
(iii) cyclopropyl and phenyl that is mono-substituted in *para* position by a substituent selected from the group consisting of H, Cl, F, Br, methyl, -(CF₃) and cyclopropyl; and/or
**R²** is selected from the group consisting of
(i) hydrogen; and
(ii) indazolyl, benzimidazolyl and benzodioxolyl, preferably indazolyl, benzimidazolyl and benzodioxolyl connected via position (5) or (6), more preferably via position (5) of the indazolyl and benzodioxolyl or position (6) of the benzimidazolyl if **R³** is not methyl, most preferably via position (6) of the indazolyl and benzodioxolyl or position (5) of the benzimidazolyl if **R³** is methyl; and/or
**R³** is selected from the group consisting of
(i) hydrogen or methyl; and
(ii) 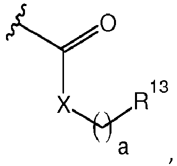 wherein **X** is N, **a** is 1 and **R¹³** is selected from the group consisting of indazolyl, benzimidazolyl and benzodioxolyl connected via position (6) or (5) of indazolyl, benzimidazolyl and benzodioxolyl, preferably via position (5) of the indazolyl and benzodioxolyl or position (6) of the benzimidazolyl; and/or
**R⁴** is selected from the group consisting of
(i) hydrogen;
(ii) methyl;
(iii) 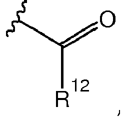 wherein **R¹²** is selected from the group consisting of
(aa) N; and
(bb) cyclopropyl and phenyl that is mono-substituted in *para* position by cyclopropyl or -(CF₃), or di-substituted in *meta* position by cyclopropyl or -(CF₃) in each *meta* position; and
(iv) 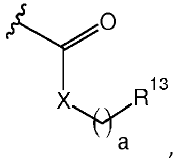 wherein **X** is N, **a** is 1 and **R¹³** is phenyl that is mono-substituted in *meta* position by cyclopropyl or -(CF₃), or di-substituted in each *meta* position by cyclopropyl or -(CF₃), or mono-substituted in *para* position by cyclopropyl or -(CF₃);
wherein, **R²** and **R⁴** and/or **R³** and **R⁴** are preferably in a *trans* configuration to each other, preferably, **R²** is (5)-, **R³** is (*S*)- and **R⁴** is (*R*)-configured; and/or
**R⁵** is hydrogen.

7. The compound for use according to any of claims 1 to 3, wherein the compound is a compound of Formula (II) and the bonds within the ring of Formula (II) are fully saturated (Formula IIa) 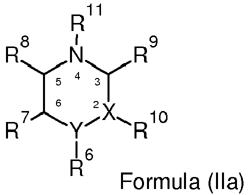 wherein **X** is C, **Y** is S and wherein
**R⁶** is not present; and/or
**R⁷** is selected from the group consisting of
(i) hydrogen;
(ii) methyl; and (iii) 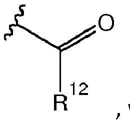 wherein **R¹²** is selected from the group consisting of
(aa) N; and
(bb) cyclopropyl or phenyl mono-substituted in *para* position by cyclopropyl or -(CF₃) or di-substituted in *meta* position by cyclopropyl or -(CF₃) in each *meta* position; and/or
**R⁸** is selected from the group consisting of
(i) hydrogen and methyl; and
(ii) 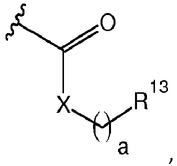 wherein **X** is N, **a** is 1 and **R¹³** is selected from the group consisting of indazolyl, benzimidazolyl and benzodioxolyl connected via position (6) or (5), preferably via position (5) of the indazolyl and benzodioxolyl or position (6) of the benzimidazolyl;
wherein **R⁸** is preferably (*R*)- or (*S*)-configured, more preferably (*R*)-configured; and/or
**R⁹** is selected from the group consisting of
(i) hydrogen, methyl and cyclopropyl; and
(ii) phenyl that is mono-substituted in *para* position by a substituent selected from the group consisting of H, Cl, F, Br, methyl, -(CF₃) and cyclopropyl;
wherein **R⁹** is preferably (*R*)- or (*S*)-configured, more preferably (*S*)-configured; and/or
**R¹⁰** is hydrogen; and/or
**R¹¹** is hydrogen or selected from the group consisting of indazolyl, benzimidazolyl and benzodioxolyl connected via position (6) or (5), preferably via position (6) of the indazolyl and benzodioxolyl or position (5) of the benzimidazolyl.

8. The compound for use according to any of claims 1 to 3, wherein the compound is a compound of Formula (II) and the ring of Formula (II) is aromatic (Formula IIb) 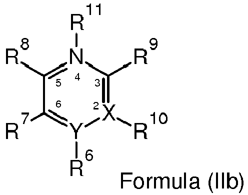 wherein **X** is N, **Y** is C and wherein
**R⁶** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R⁷** and **R⁸** are independently selected from the group consisting of
(i) hydrogen;
(ii) methyl; and
(iii) 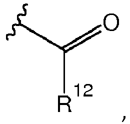 wherein **R¹²** is selected from the group consisting of
(aa) N; and
(bb) cyclopropyl and phenyl that is mono-substituted in *para* position by cyclopropyl or -(CF₃), or di-substituted in *meta* position by cyclopropyl or -(CF₃) in each *meta* position; and/or
**R⁹** is hydrogen; and/or
**R¹⁰** and/or **R¹¹** are absent.

9. The compound for use according to any of claims 1 to 3, wherein the compound is a compound of Formula (II) and two double bonds are located between positions (1) and (6) and between positions (4) and (5), respectively (Formula IIc) 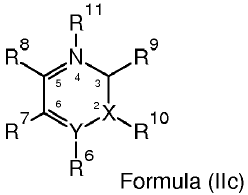 wherein X is N, Y is C and wherein
**R⁶** is selected from the group consisting of indazolyl, benzimidazolyl and benzodioxolyl connected via position (6) or (5), preferably via position (6) of the indazolyl and benzodioxolyl or position (5) of the benzimidazolyl; and/or
**R⁷** and **R⁸** are independently selected from the group consisting of hydrogen and methyl; and/or
**R⁹** is hydrogen; and/or
**R¹⁰** is selected from the group consisting of
(i) hydrogen;
(ii) methyl; and
(iii) cyclopropyl and phenyl that is mono-substituted in *para* position by a substituent selected from the group consisting of H, Cl, F, Br, methyl, -(CF₃) and cyclopropyl; and/or
**R¹¹** is absent.

10. The compound for use according to any of claims 1 to 9, wherein
**R¹** is selected from the group consisting of hydrogen, methyl, 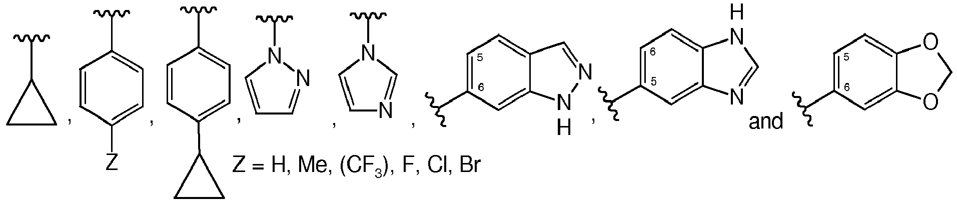 and/or
**R²** is selected from the group consisting of hydrogen, oxo, methyl, 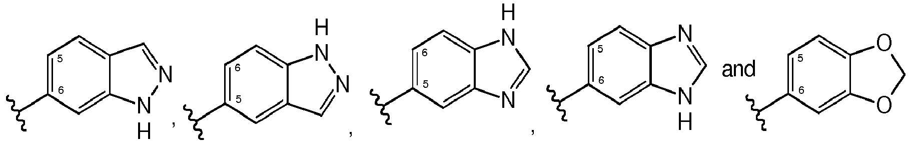 and/or
**R³** is selected from the group consisting of hydrogen, methyl, 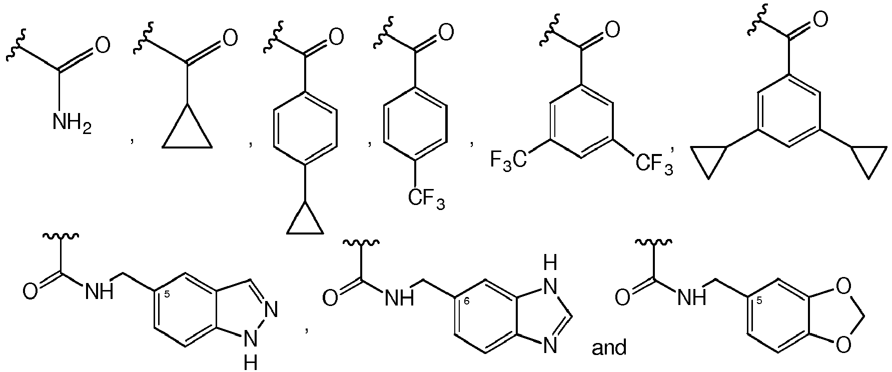 and/or
**R⁴** is selected from the group consisting of hydrogen, hydroxyl, methyl, 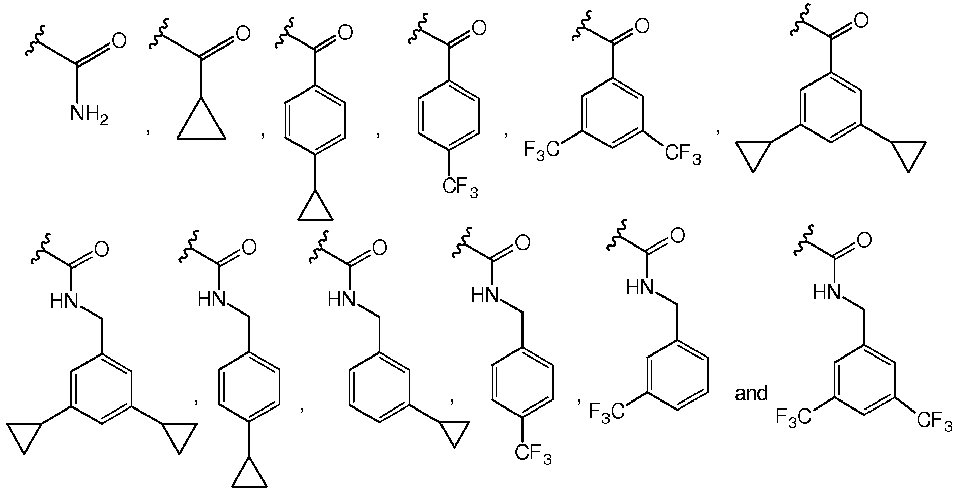 wherein, **R⁴** and/or **R³** and **R⁴** are preferably in *cis* or *trans* configuration to each other, more preferably in *trans* configuration, preferably, **R²** is (*R*)- or (*S*)-, **R³** is (*R*)- or (*S*)- and/or **R⁴** is (*R*)- or (*S*)-configured, more preferably, **R²** is (*R*)-, **R³** is (*R*)- and/or **R⁴** is (*R*)-configured, or **R²** is or (*S*)-, **R³** is (*S*)- and/or **R⁴** is (*S*)-configured, and/or
**R⁵** and **R⁹** are selected from the group consisting of hydrogen, methyl, cyclopenta-2,4-dien-1-yl, 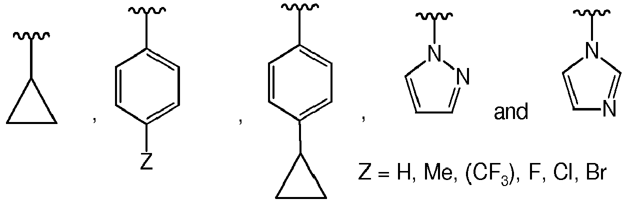 wherein **R⁹** is preferably not cyclopenta-2,4-dien-1-yl,
wherein, if position (5) of the ring of Formula (I) is sp³-hybridized, **R⁵** is preferably (*R*)- or (*S*)-configured, more preferably (*R*)-configured, and
wherein **R⁹** is preferably (*R*)- or (*S*)-configured, more preferably (*R*)-configured,
and/or
**R⁶** is not present if **Y** is S, and if **Y** is C, **R⁶** is selected from the group consisting of 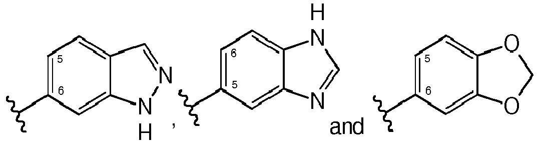 and/or **R⁷** and **R⁸** are selected from the group consisting of hydrogen, methyl, 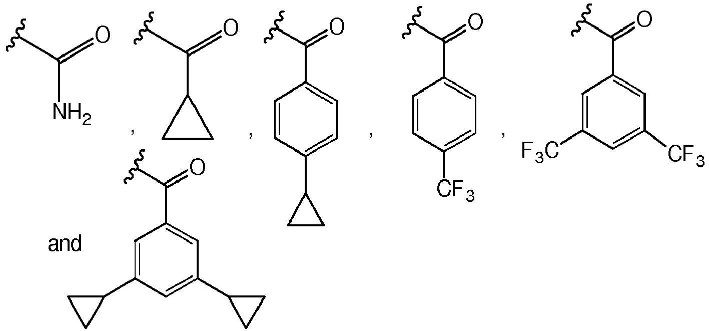 wherein **R⁸** is additionally selected from the group consisting of 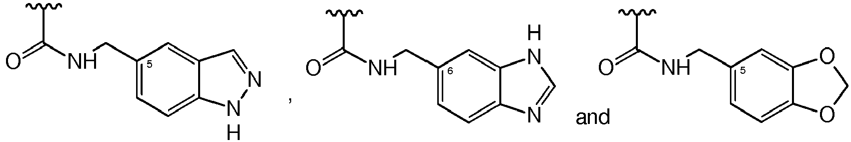 and wherein **R⁸** is preferably (*R*)- or (*S*)-configured, more preferably (*R*)-configured; and/or **R¹⁰** is absent or selected from the group consisting of hydrogen, methyl, 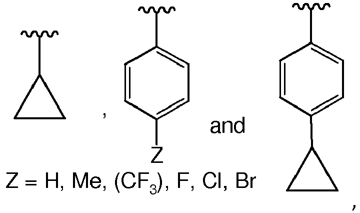 wherein **R¹⁰** is absent if **X** is C and/or if the ring of Formula (II) has a double bond between positions (1) and (2) or between positions (2) and (3) of the ring of Formula (II), and/or **R¹¹** is absent or selected from the group consisting of hydrogen, 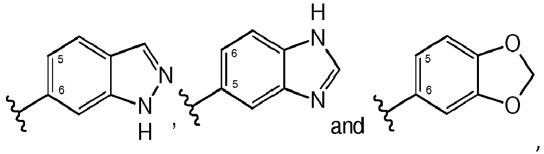 wherein **R¹¹** is absent if the ring of Formula (II) has a double bond between positions (4) and (5) or between positions (3) and (4) of the ring of Formula (II).

11. The compound for use according to any of claims 1 to 10, wherein the compound is selected from the group consisting of:
**(i)** a first residue selected from the group consisting of
1-(1,3-benzodioxol-5-yl)-3-hydroxy-5-oxo-2-methyl-2H-pyrrol-4-yl,
1-(1,3-benzodioxol-5-yl)-2-cyclopropyl-3-hydroxy-5-oxo-2H-pyrrol-4-yl,
1-(1,3-benzodioxol-5-yl)-3-hydroxy-5-oxo-2H-pyrrol-4-yl,
1-(1,3-benzodioxol-5-yl)-2-(cyclopenta-2,4-dien-1-yl)-5-oxo-3-hydroxy-2H-pyrrol-4-yl,
1-(1,3-benzodioxol-5-yl)-3-hydroxy-5-oxo-2-(pyrazol-1-yl)-2H-pyrrol-4-yl,
1-(1,3-benzodioxol-5-yl)-3-hydroxy-5-oxo-2-(imidazol-1-yl)-2H-pyrrol-4-yl,
1-(1,3-benzodioxol-5-yl)-3-hydroxy-5-oxo-2-phenyl-2H-pyrrol-4-yl,
1-(1,3-benzodioxol-5-yl)-3-hydroxy-5-oxo-2-(p-tolyl)-2H-pyrrol-4-yl,
1-(1,3-benzodioxol-5-yl)-2-(4-chlorophenyl)-3-hydroxy-5-oxo-2H-pyrrol-4-yl,
1-(1,3-benzodioxol-5-yl)-2-(4-fluorophenyl)-3-hydroxy-5-oxo-2H-pyrrol-4-yl,
1-(1,3-benzodioxol-5-yl)-2-(4-bromophenyl)-3-hydroxy-5-oxo-2H-pyrrol-4-yl,
1-(1,3-benzodioxol-5-yl)-2-(4-cyclopropylphenyl)-3-hydroxy-5-oxo-2H-pyrrol-4-yl,
1-(1,3-benzodioxol-5-yl)-3-hydroxy-5-oxo-2-[4-(trifluoromethyl)phenyl]-2H-pyrrol-4-yl,
3-hydroxy-1-(1H-indazol-6-yl)-5-oxo-2-[4-(trifluoromethyl)phenyl]-2H-pyrrol-4-yl,
3-hydroxy-1-(1H-indazol-6-yl)-5-oxo-2-phenyl-2H-pyrrol-4-yl,
3-hydroxy-1-(1H-indazol-6-yl)-5-oxo-2-(p-tolyl)-2H-pyrrol-4-yl,
2-(4-chlorophenyl)-3-hydroxy-1-(1H-indazol-6-yl)-5-oxo-2H-pyrrol-4-yl,
2-(4-fluorophenyl)-3-hydroxy-1-(1H-indazol-6-yl)-5-oxo-2H-pyrrol-4-yl,
2-(4-bromophenyl)-3-hydroxy-1-(1H-indazo1-6-yl)-5-oxo-2H-pyrrol-4-yl,
2-(4-cyclopropylphenyl)-3-hydroxy-1-(1H-indazol-6-yl)-5-oxo-2H-pyrrol-4-yl,
2-cyclopropyl-3-hydroxy-1-(1H-indazol-6-yl)-5-oxo-2H-pyrrol-4-yl,
3-hydroxy-1-(1H-indazol-6-yl)-5-oxo-2-methyl-2H-pyrrol-4-yl,
3-hydroxy-1-(1H-indazol-6-yl)-5-oxo-2H-pyrrol-4-yl,
3-hydroxy-1-(1H-indazol-6-yl)-5-oxo-2-(pyrazol-1-yl)-2H-pyrrol-4-yl,
2-(cyclopenta-2,4-dien-1-yl)-3-hydroxy-1-(1H-indazol-6-yl)-5-oxo-2H-pyrrol-4-yl,
3-hydroxy-2-(imidazol-1-yl)-1-(1H-indazol-6-yl)-5-oxo-2H-pyrrol-4-yl,
1-(1H-benzimidazol-5-yl)-3-hydroxy-5-oxo-2-dimethyl-2H-pyrrol-4-yl,
1-(1H-benzimidazol-5-yl)-2-cyclopropyl-3-hydroxy-5-oxo-2H-pyrrol-4-yl,
1-(1H-benzimidazol-5-yl)-3-hydroxy-5-oxo-2-(pyrazol-1-yl)-2H-pyrrol-4-yl,
1-(1H-benzimidazol-5-yl)-3-hydroxy-5-oxo-2H-pyrrol-4-yl,
1-(1H-benzimidazol-5-yl)-3-hydroxy-5-oxo-2-(imidazol-1-yl)-2H-pyrrol-4-yl,
1-(1H-benzimidazol-5-yl)-2-(cyclopenta-2,4-dien-1-yl)-3-hydroxy-5-oxo-2H-pyrrol-4-yl,
1-(1H-benzimidazol-5-yl)-2-(4-fluorophenyl)-3-hydroxy-5-oxo-2H-pyrrol-4-yl,
1-(1H-benzimidazol-5-yl)-3-hydroxy-5-oxo-2-[4-(trifluoromethyl)phenyl]-2H-pyrrol-4-yl,
1-(1H-benzimidazol-5-yl)-3-hydroxy-5-oxo-2-phenyl-2H-pyrrol-4-yl,
1-(1H-benzimidazol-5-yl)-3-hydroxy-5-oxo-2-(p-tolyl)-2H-pyrrol-4-yl,
1-(1H-benzimidazol-5-yl)-2-(4-chlorophenyl)-3-hydroxy-5-oxo-2H-pyrrol-4-yl,
1-(1H-benzimidazol-5-yl)-2-(4-bromophenyl)-3-hydroxy-5-oxo-2H-pyrrol-4-yl,
1-(1H-benzimidazol-5-yl)-2-(4-cyclopropylphenyl)-3-hydroxy-5-oxo-2H-pyrrol-4-yl,
wherein the numbering of the 2H-pyrrole ring is as follows: 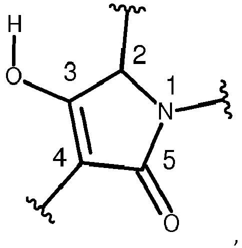
1-(1,3-benzodioxol-5-ylmethyl)-2-methyl-5-(p-tolyl)pyrrol-3-yl,
1-(1,3-benzodioxol-5-ylmethyl)-5-(4-chlorophenyl)-2-methyl-pyrrol-3-yl,
1-(1,3-benzodioxol-5-ylmethyl)-5-(4-fluorophenyl)-2-methyl-pyrrol-3-yl,
1-(1,3-benzodioxol-5-ylmethyl)-2-methyl-5-[4-(trifluoromethyl)phenyl]pyrrol-3-yl,
1-(1,3-benzodioxol-5-ylmethyl)-5-(4-bromophenyl)-2-methyl-pyrrol-3-yl,
1-(1,3-benzodioxol-5-ylmethyl)-5-(4-cyclopropylphenyl)-2-methyl-pyrrol-3-yl,
1-(1,3-benzodioxol-5-ylmethyl)-2-methyl-5-phenyl-pyrrol-3-yl,
1-(1,3-benzodioxol-5-ylmethyl)-5-phenyl-pyrrol-3-yl,
1-(1,3-benzodioxol-5-ylmethyl)-5-(p-tolyl)pyrrol-3-yl,
1-(1,3-benzodioxol-5-ylmethyl)-5-(4-fluorophenyl)pyrrol-3-yl,
1-(1,3-benzodioxol-5-ylmethyl)-5-[4-(trifluoromethyl)phenyl]pyrrol-3-yl,
1-(1,3-benzodioxol-5-ylmethyl)-5-(4-chlorophenyl)pyrrol-3-yl,
1-(1,3-benzodioxol-5-ylmethyl)-5-(4-bromophenyl)pyrrol-3-yl,
1-(1,3-benzodioxol-5-ylmethyl)-5-(4-cyclopropylphenyl)pyrrol-3-yl,
1-(1,3-benzodioxol-5-ylmethyl)-2,5-dimethyl-pyrrol-3-yl,
1-(1,3-benzodioxol-5-ylmethyl)-2-methyl-pyrrol-3-yl,
1-(1,3-benzodioxol-5-ylmethyl)-5-cyclopropyl-2-methyl-pyrrol-3-yl,
1-(1,3-benzodioxol-5-ylmethyl)pyrrol-3-yl,
1-(1,3-benzodioxol-5-ylmethyl)-5-methyl-pyrrol-3-yl,
1-(1,3-benzodioxol-5-ylmethyl)-5-cyclopropyl-pyrrol-3-yl,
1-(1H-indazol-6-ylmethyl)-2-methyl-5-phenyl-pyrrol-3-yl,
1-(1H-indazol-6-ylmethyl)-2-methyl-5-(p-tolyl)pyrrol-3-yl,
5-(4-chlorophenyl)-1-(1H-indazol-6-ylmethyl)-2-methyl-pyrrol-3-yl,
5-(4-fluorophenyl)-1-(1H-indazol-6-ylmethyl)-2-methyl-pyrrol-3-yl,
1-(1H-indazol-6-ylmethyl)-2-methyl-5-[4-(trifluoromethyl)phenyl]pyrrol-3-yl,
5-(4-bromophenyl)-1-(1H-indazol-6-ylmethyl)-2-methyl-pyrrol-3-yl,
5-(4-cyclopropylphenyl)-1-(1H-indazol-6-ylmethyl)-2-methyl-pyrrol-3-yl,
1-(1H-indazol-6-ylmethyl)-5-phenyl-pyrrol-3-yl,
1-(1H-indazol-6-ylmethyl)-5-(p-tolyl)pyrrol-3-yl,
5-(4-fluorophenyl)-1-(1H-indazol-6-ylmethyl)pyrrol-3-yl,
1-(1H-indazol-6-ylmethyl)-5-[4-(trifluoromethyl)phenyl]pyrrol-3-yl,
5-(4-chlorophenyl)-1-(1H-indazol-6-ylmethyl)pyrrol-3-yl,
5-(4-bromophenyl)-1-(1H-indazol-6-ylmethyl)pyrrol-3-yl,
5-(4-cyclopropylphenyl)-1-(1H-indazol-6-ylmethyl)pyrrol-3-yl,
5-cyclopropyl-1-(1H-indazol-6-ylmethyl)-2-methyl-pyrrol-3-yl,
1-(1H-indazol-6-ylmethyl)-2-methyl-pyrrol-3-yl,
1-(1H-indazol-6-ylmethyl)-2,5-dimethyl-pyrrol-3-yl,
1-(1H-indazol-6-ylmethyl)pyrrol-3-yl,
5-cyclopropyl-1-(1H-indazol-6-ylmethyl)pyrrol-3-yl,
1-(1H-indazol-6-ylmethyl)-5-methyl-pyrrol-3-yl,
1-(1H-benzimidazol-5-ylmethyl)-2-methyl-5-phenyl-pyrrol-3-yl,
1-(1H-benzimid azol-5-ylmethyl)-2-methyl-5-(p-tolyl)pyrrol-3-yl,
1-(1H-benzimidazol-5-ylmethyl)-2-methyl-5-(p-tolyl)pyrrol-3-yl,
1-(1H-benzimidazol-5-ylmethyl)-5-(4-fluorophenyl)-2-methyl-pyrrol-3-yl,
1-(1H-benzimidazol-5-ylmethyl)-5-(4-cyclopropylphenyl)-2-methyl-pyrrol-3-yl,
1-(1H-benzimidazol-5-ylmethyl)-5-(4-bromophenyl)-2-methyl-pyrrol-3-yl,
1-(1H-benzimidazol-5-ylmethyl)-2-methyl-5-[4-(trifluoromethyl)phenyl]pyrrol-3-yl,
1-(1H-benzimidazol-5-ylmethyl)-5-phenyl-pyrrol-3-yl,
1-(1H-benzimidazol-5-ylmethyl)-5-(p-tolyl)pyrrol-3-yl,
1-(1H-benzimidazol-5-ylmethyl)-5-[4-(trifluoromethyl)phenyl]pyrrol-3-yl,
1-(1H-benzimidazol-5-ylmethyl)-5-(4-chlorophenyl)pyrrol-3-yl,
1-(1H-benzimidazol-5-ylmethyl)-5-(4-fluorophenyl)pyrrol-3-yl,
1-(1H-benzimidazol-5-ylmethyl)-5-(4-bromophenyl)pyrrol-3-yl,
1-(1H-benzimidazol-5-ylmethyl)-5-(4-cyclopropylphenyl)pyrrol-3-yl,
1-(1H-benzimidazol-5-ylmethyl)-2,5-dimethyl-pyrrol-3-yl,
1-(1H-benzimidazol-5-ylmethyl)-2-methyl-pyrrol-3-yl,
1-(1H-benzimidazol-5-ylmethyl)-5-cyclopropyl-2-methyl-pyrrol-3-yl,
1-(1H-benzimidazol-5-ylmethyl)-5-methyl-pyrrol-3-yl,
1-(1H-benzimidazol-5-ylmethyl)pyrrol-3-yl,
1-(1H-benzimidazol-5-ylmethyl)-5-cyclopropyl-pyrrol-3-yl,
1-(1,3-benzodioxol-5-yl)-5-(p-tolyl)pyrrol-3-yl,
1-(1,3-benzodioxol-5-yl)-5-phenyl-pyrrol-3-yl,
1-(1,3-benzodioxol-5-yl)-5-(4-fluorophenyl)pyrrol-3-yl,
1-(1,3-benzodioxol-5-yl)-5-[4-(trifluoromethyl)phenyl]pyrrol-3-yl,
1-(1,3-benzodioxol-5-yl)-5-(4-bromophenyl)pyrrol-3-yl,
1-(1,3-benzodioxol-5-yl)-5-(4-chlorophenyl)pyrrol-3-yl,
1-(1,3-benzodioxol-5-yl)-5-(4-cyclopropylphenyl)pyrrol-3-yl,
1-(1,3-benzodioxol-5-yl)-2-methyl-5-(p-tolyl)pyrrol-3-yl,
1-(1,3-benzodioxol-5-yl)-2-methyl-5-phenyl-pyrrol-3-yl,
1-(1,3-benzodioxol-5-yl)-5-(4-fluorophenyl)-2-methyl-pyrrol-3-yl,
1-(1,3-benzodioxol-5-yl)-2-methyl-5-[4-(trifluoromethyl)phenyl]pyrrol-3-yl,
1-(1,3-benzodioxol-5-yl)-5-(4-chlorophenyl)-2-methyl-pyrrol-3-yl,
1-(1,3-benzodioxol-5-yl)-5-(4-bromophenyl)-2-methyl-pyrrol-3-yl,
1-(1,3-benzodioxol-5-yl)-5-(4-cyclopropylphenyl)-2-methyl-pyrrol-3-yl,
1-(1,3-benzodioxol-5-yl)-2,5-dimethyl-pyrrol-3-yl,
1-(1,3-benzodioxol-5-yl)-2-methyl-pyrrol-3-yl,
1-(1,3-benzodioxol-5-yl)-5-cyclopropyl-2-methyl-pyrrol-3-yl,
1-(1,3-benzodioxol-5-yl)-5-methyl-pyrrol-3-yl,
1-(1,3-benzodioxol-5-yl)pyrrol-3-yl,
1-(1,3-benzodioxol-5-yl)-5-cyclopropyl-pyrrol-3-yl,
1-(1H-indazol-6-yl)-5-(p-tolyl)pyrrol-3-yl,
5-(4-chlorophenyl)-1-(1H-indazol-6-yl)pyrrol-3-yl,
5-(4-bromophenyl)-1-(1H-indazol-6-yl)pyrrol-3-yl,
5-(4-fluorophenyl)-1-(1H-indazol-6-yl)pyrrol-3-yl,
1-(1H-indazol-6-yl)-5-phenyl-pyrrol-3-yl,
5-(4-cyclopropylphenyl)-1-(1H-indazol-6-yl)pyrrol-3-yl,
1-(1H-indazol-6-yl)-5-[4-(trifluoromethyl)phenyl]pyrrol-3-yl,
1-(1H-indazol-6-yl)-2-methyl-5-(p-tolyl)pyrrol-3-yl,
5-(4-chlorophenyl)-1-(1H-indazol-6-yl)-2-methyl-pyrrol-3-yl,
5-(4-bromophenyl)-1-(1H-indazol-6-yl)-2-methyl-pyrrol-3-yl,
5-(4-fluorophenyl)-1-(1H-indazol-6-yl)-2-methyl-pyrrol-3-yl,
1-(1H-indazol-6-yl)-2-methyl-5-phenyl-pyrrol-3-yl,
5-(4-cyclopropylphenyl)-1-(1H-indazol-6-yl)-2-methyl-pyrrol-3-yl,
1-(1H-indazol-6-yl)-2-methyl-5-[4-(trifluoromethyl)phenyl]pyrrol-3-yl,
1-(1H-indazol-6-yl)-2,5-dimethyl-pyrrol-3-yl,
1-(1H-indazol-6-yl)-2-methyl-pyrrol-3-yl,
5-cyclopropyl-1-(1H-indazol-6-yl)-2-methyl-pyrrol-3-yl,
1-(1H-indazol-6-yl)-5-methyl-pyrrol-3-yl,
1-(1H-indazol-6-yl)pyrrol-3-yl,
5-cyclopropyl-1-(1H-indazol-6-yl)pyrrol-3-yl,
1-(1H-benzimidazol-5-yl)-5-(p-tolyl)pyrrol-3-yl,
1-(1H-benzimidazol-5-yl)-5-(4-chlorophenyl)pyrrol-3-yl,
1-(1H-benzimidazol-5-yl)-5-(4-bromophenyl)pyrrol-3-yl,
1-(1H-benzimidazol-5-yl)-5-(4-fluorophenyl)pyrrol-3-yl,
1-(1H-benzimidazol-5-yl)-5-phenyl-pyrrol-3-yl,
1-(1H-benzimidazol-5-yl)-5-(4-cyclopropylphenyl)pyrrol-3-yl,
1-(1H-benzimidazol-5-yl)-5-[4-(trifluoromethyl)phenyl]pyrrol-3-yl,
1-(1H-benzimidazol-5-yl)-2-methyl-5-(p-tolyl)pyrrol-3-yl,
1-(1H-benzimidazol-5-yl)-5-(4-chlorophenyl)-2-methyl-pyrrol-3-yl,
1-(1H-benzimidazol-5-yl)-5-(4-bromophenyl)-2-methyl-pyrrol-3-yl,
1-(1H-benzimidazol-5-yl)-5-(4-fluorophenyl)-2-methyl-pyrrol-3-yl,
1-(1H-benzimidazol-5-yl)-2-methyl-5-phenyl-pyrrol-3-yl,
1-(1H-benzimidazol-5-yl)-5-(4-cyclopropylphenyl)-2-methyl-pyrrol-3-yl,
1-(1H-benzimidazol-5-yl)-2-methyl-5-[4-(trifluoromethyl)phenyl]pyrrol-3-yl,
1-(1H-benzimidazol-5-yl)-2,5-dimethyl-pyrrol-3-yl,
1-(1H-benzimidazol-5-yl)-2-methyl-pyrrol-3-yl,
1-(1H-benzimidazol-5-yl)-5-cyclopropyl-2-methyl-pyrrol-3-yl,
1-(1H-benzimidazol-5-yl)-5-methyl-pyrrol-3-yl,
1-(1H-benzimidazol-5-yl)pyrrol-3-yl,
1-(1H-benzimidazol-5-yl)-5-cyclopropyl-pyrrol-3-yl,
wherein the numbering of the pyrrole ring is as follows: 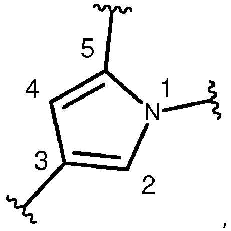
(3R)-3-(1,3-benzodioxol-5-ylmethylcarbamoyl)-5-phenyl-thiomorpholin-2-yl,
(3R)-3-(1,3-benzodioxol-5-ylmethylcarbamoyl)-5-(p-tolyl)thiomorpholin-2-yl,
(3R)-3-(1,3-benzodioxol-5-ylmethylcarbamoyl)-5-(4-fluorophenyl)thiomorpholin-2-yl,
(3R)-3-(1,3-benzodioxol-5-ylmethylcarbamoyl)-5-(4-bromophenyl)thiomorpholin-2-yl,
(3R)-3-(1,3-benzodioxol-5-ylmethylcarbamoyl)-5-(4-chlorophenyl)thiomorpholin-2-yl,
(3R)-3-(1,3-benzodioxol-5-ylmethylcarbamoyl)-5-(4-cyclopropylphenyl)thiomorpholin-2-yl,
(3R)-3-(1,3-benzodioxol-5-ylmethylcarbamoyl)-5-[4-(trifluoromethyl)phenyl]thio-morpholin-2-yl,
(3R)-3-(1H-indazol-5-ylmethylcarbamoyl)-5-phenyl-thiomorpholin-2-yl,
(3R)-3-(1H-indazol-5-ylmethylcarbamoyl)-5-(p-tolyl)thiomorpholin-2-yl,
(3R)-5-(4-fluorophenyl)-3-(1H-indazol-5-ylmethylcarbamoyl)thiomorpholin-2-yl,
(3R)-5-(4-bromophenyl)-3-(1H-indazol-5-ylmethylcarbamoyl)thiomorpholin-2-yl,
(3R)-5-(4-chlorophenyl)-3-(1H-indazol-5-ylmethylcarbamoyl)thiomorpholin-2-yl,
(3R)-5-(4-cyclopropylphenyl)-3-(1H-indazol-5-ylmethylcarbamoyl)thiomorpholin-2-yl,
(3R)-3-(1H-indazol-5-ylmethylcarbamoyl)-5-[4-(trifluoromethyl)phenyl]thiomorpholin-2-yl,
(3R)-3-(3H-benzimidazol-5-ylmethylcarbamoyl)-5-phenyl-thiomorpholin-2-yl,
(3R)-3-(3H-benzimidazol-5-ylmethylcarbamoyl)-5-(p-tolyl)thiomorpholin-2-yl,
(3R)-3-(3H-benzimidazol-5-ylmethylcarbamoyl)-5-(4-fluorophenyl)thiomorpholin-2-yl,
(3R)-3-(3H-benzimidazol-5-ylmethylcarbamoyl)-5-[4-(trifluoromethyl)phenyl]thio-morpholin-2-yl,
(3R)-3-(3H-benzimidazol-5-ylmethylcarbamoyl)-5-(4-bromophenyl)thiomorpholin-2-yl,
(3R)-3-(3H-benzimidazol-5-ylmethylcarbamoyl)-5-(4-chlorophenyl)thiomorpholin-2-yl,
(3R)-3-(3H-benzimidazol-5-ylmethylcarbamoyl)-5-(4-cyclopropylphenyl)thio-morpholin-2-yl,
4-(1,3-benzodioxol-5-yl)-5-(4-fluorophenyl)thiomorpholin-2-yl,
4-(1,3-benzodioxol-5-yl)-5-(4-bromophenyl)thiomorpholin-2-yl,
4-(1,3-benzodioxol-5-yl)-5-(4-chlorophenyl)thiomorpholin-2-yl,
4-(1,3-benzodioxol-5-yl)-5-methyl-thiomorpholin-2-yl,
4-(1,3-benzodioxol-5-yl)-5-cyclopropyl-thiomorpholin-2-yl,
4-(1,3-benzodioxol-5-yl)-5-(4-cyclopropylphenyl)thiomorpholin-2-yl,
4-(1,3-benzodioxol-5-yl)-5-(p-tolyl)thiomorpholin-2-yl,
4-(1,3-benzodioxol-5-yl)-5-phenyl-thiomorpholin-2-yl,
4-(1,3-benzodioxol-5-yl)-5-[4-(trifluoromethyl)phenyl]thiomorpholin-2-yl,
4-(1,3-benzodioxol-5-yl)thiomorpholin-2-yl,
4-(1,3-benzodioxol-5-yl)-5-(4-fluorophenyl)-3-methyl-thiomorpholin-2-yl,
4-(1,3-benzodioxol-5-yl)-5-(4-bromophenyl)-3-methyl-thiomorpholin-2-yl,
4-(1,3-benzodioxol-5-yl)-5-(4-chlorophenyl)-3-methyl-thiomorpholin-2-yl,
4-(1,3-benzodioxol-5-yl)-3,5-dimethyl-thiomorpholin-2-yl,
4-(1,3-benzodioxol-5-yl)-5-cyclopropyl-3-methyl-thiomorpholin-2-yl,
4-(1,3-benzodioxol-5-yl)-5-(4-cyclopropylphenyl)-3-methyl-thiomorpholin-2-yl,
4-(1,3-benzodioxol-5-yl)-3-methyl-5-(p-tolyl)thiomorpholin-2-yl,
4-(1,3-benzodioxol-5-yl)-3-methyl-5-phenyl-thiomorpholin-2-yl,
4-(1,3-benzodioxol-5-yl)-3-methyl-5-[4-(trifluoromethyl)phenyl]thiomorpholin-2-yl,
4-(1,3-benzodioxol-5-yl)-3-methyl-thiomorpholin-2-yl,
5-(4-fluorophenyl)-4-(1H-indazol-6-yl)thiomorpholin-2-yl,
5-(4-bromophenyl)-4-(1H-indazol-6-yl)thiomorpholin-2-yl,
5-(4-chlorophenyl)-4-(1H-indazol-6-yl)thiomorpholin-2-yl,
4-(1H-indazol-6-yl)-5-methyl-thiomorpholin-2-yl,
5-cyclopropyl-4-(1H-indazol-6-yl)thiomorpholin-2-yl,
5-(4-cyclopropylphenyl)-4-(1H-indazol-6-yl)thiomorpholin-2-yl,
4-(1H-indazol-6-yl)-5-(p-tolyl)thiomorpholin-2-yl,
4-(1H-indazol-6-yl)-5-phenyl-thiomorpholin-2-yl,
4-(1H-indazol-6-yl)-5-[4-(trifluoromethyl)phenyl]thiomorpholin-2-yl,
4-(1H-indazol-6-yl)thiomorpholin-2-yl,
5-(4-fluorophenyl)-4-(1H-indazol-6-yl)-3-methyl-thiomorpholin-2-yl,
5-(4-bromophenyl)-4-(1H-indazol-6-yl)-3-methyl-thiomorpholin-2-yl,
5-(4-chlorophenyl)-4-(1H-indazol-6-yl)-3-methyl-thiomorpholin-2-yl,
4-(1H-indazol-6-yl)-3,5-dimethyl-thiomorpholin-2-yl,
5-(4-cyclopropylphenyl)-4-(1H-indazol-6-yl)-3-methyl-thiomorpholin-2-yl,
4-(1H-indazol-6-yl)-3-methyl-5-(p-tolyl)thiomorpholin-2-yl,
4-(1H-indazol-6-yl)-3-methyl-5-phenyl-thiomorpholin-2-yl,
5-cyclopropyl-4-(1H-indazol-6-yl)-3-methyl-thiomorpholin-2-yl,
4-(1H-indazol-6-yl)-3-methyl-thiomorpholin-2-yl,
4-(1H-indazol-6-yl)-3-methyl-5-[4-(trifluoromethyl)phenyl]thiomorpholin-2-yl,
4-(1H-benzimidazol-5-yl)-5-(4-fluorophenyl)thiomorpholin-2-yl,
4-(1H-benzimidazol-5-yl)-5-(4-bromophenyl)thiomorpholin-2-yl,
4-(1H-benzimidazol-5-yl)-5-(4-chlorophenyl)thiomorpholin-2-yl,
4-(1H-benzimidazol-5-yl)-5-methyl-thiomorpholin-2-yl,
4-(1H-benzimidazol-5-yl)-5-cyclopropyl-thiomorpholin-2-yl,
4-(1H-benzimidazol-5-yl)-5-(4-cyclopropylphenyl)thiomorpholin-2-yl,
4-(1H-benzimidazol-5-yl)-5-(p-tolyl)thiomorpholin-2-yl,
4-(1H-benzimidazol-5-yl)-5-phenyl-thiomorpholin-2-yl,
4-(1H-benzimidazol-5-yl)-5-[4-(trifluoromethyl)phenyl]thiomorpholin-2-yl,
4-(1H-benzimidazol-5-yl)thiomorpholin-2-yl,
4-(1H-benzimidazol-5-yl)-5-(4-fluorophenyl)-3-methyl-thiomorpholin-2-yl,
4-(1H-benzimidazol-5-yl)-5-(4-bromophenyl)-3-methyl-thiomorpholin-2-yl,
4-(1H-benzimidazol-5-yl)-5-(4-chlorophenyl)-3-methyl-thiomorpholin-2-yl,
4-(1H-benzimidazol-5-yl)-3,5-dimethyl-thiomorpholin-2-yl,
4-(1H-benzimidazol-5-yl)-5-cyclopropyl-3-methyl-thiomorpholin-2-yl,
4-(1H-benzimidazol-5-yl)-5-(4-cyclopropylphenyl)-3-methyl-thiomorpholin-2-yl,
4-(1H-benzimidazol-5-yl)-3-methyl-5-(p-tolyl)thiomorpholin-2-yl,
4-(1H-benzimidazol-5-yl)-3-methyl-5-phenyl-thiomorpholin-2-yl,
4-(1H-benzimidazol-5-yl)-3-methyl-5-[4-(trifluoromethyl)phenyl]thiomorpholin-2-yl,
4-(1H-benzimidazol-5-yl)-3-methyl-thiomorpholin-2-yl,
wherein the numbering of the thiomorpholine ring is as follow: 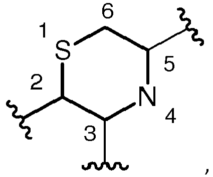 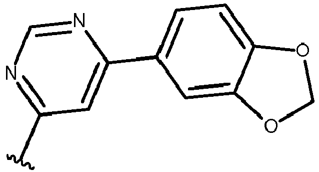
6-(1,3-benzodioxol-5-yl)pyrimidin-4-yl, 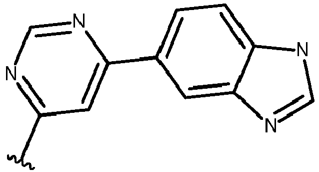
6-(1H-benzimidazol-5-yl)pyrimidin-4-yl, 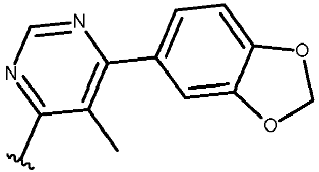
6-(1,3-benzodioxol-5-yl)-5-methyl-pyrimidin-4-yl, 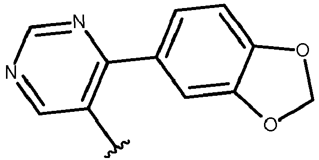
4-(1,3-benzodioxol-5-yl)pyrimidin-5-yl, 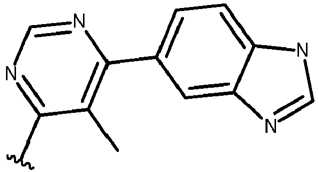
6-(1H-benzimidazol-5-yl)-5-methyl-pyrimidin-4-yl, 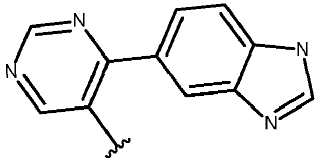
4-(1H-benzimidazol-5-yl)pyrimidin-5-yl, 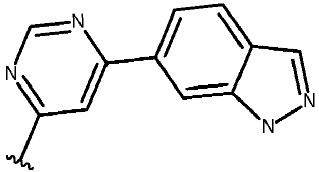
6-(1H-indazol-6-yl)pyrimidin-4-yl, 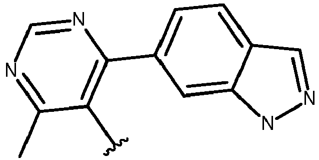
6-(1H-indazol-6-yl)-4-methyl-pyrimidin-5-yl, and 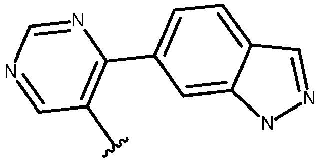
6-(1H-indazol-6-yl)pyrimidin-5-yl;
covalently bound to a second residue selected from the group consisting of hydrogen, methyl, 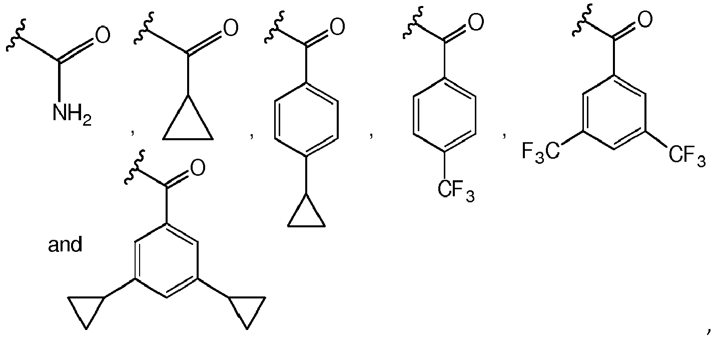 and
**(ii)** a first residue selected from the group consisting of
6-(1,3-benzodioxol-5-yl)-2H-pyrimidin-1-yl,
6-(1,3-benzodioxol-5-yl)-4-methyl-2H-pyrimidin-1-yl,
6-(1H-benzimidazol-5-yl)-2H-pyrimidin-1-yl,
6-(1H-benzimidazol-5-yl)-4-methyl-2H-pyrimidin-1-yl,
6-(1,3-benzodioxol-5-yl)-4,5-dimethyl-2H-pyrimidin-1-yl,
6-(1,3-benzodioxol-5-yl)-5-methyl-2H-pyrimidin-1-yl,
6-(1H-benzimidazol-5-yl)-4,5-dimethyl-2H-pyrimidin-1-yl,
6-(1H-indazol-6-yl)-2H-pyrimidin-1-yl,
6-(1H-indazol-6-yl)-4-methyl-2H-pyrimidin-1-yl,
6-(1H-benzimidazol-5-yl)-5-methyl-2H-pyrimidin-1-yl,
6-(1H-indazol-6-yl)-4,5-dimethyl-2H-pyrimidin-1-yl,
6-(1H-indazol-6-yl)-5-methyl-2H-pyrimidin-1-yl,
wherein the 2H-pyrimidine ring is numbered as follows: 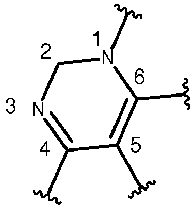
(4R)-3-(1,3-benzodioxol-5-ylmethylcarbamoyl)-4-[[3-(trifluoromethyl)phenyl]methyl-carbamoyl]pyrrolidin-1-yl,
(4R)-3-(1,3-benzodioxol-5-ylmethylcarbamoyl)-4-[[3,5-bis(trifluoromethyl)phenyl]-methylcarbamoyl]pyrrolidin-1-yl,
(4R)-3-(1,3-benzodioxol-5-ylmethylcarbamoyl)-4-[[4-(trifluoromethyl)phenyl]methyl-carbamoyl]pyrrolidin-1-yl,
(4R)-3-(1,3-benzodioxol-5-ylmethylcarbamoyl)-4-[[3-(cyclopropylphenyl)phenyl]-methylcarbamoyl]pyrrolidin-1-yl,
(4R)-3-(1,3-benzodioxol-5-ylmethylcarbamoyl)-4-[[3,5-bis(cyclopropylphenyl)phenyl]-methylcarbamoyl]pyrrolidin-1-yl,
(4R)-3-(1,3-benzodioxol-5-ylmethylcarbamoyl)-4-[[4-(cyclopropylphenyl)phenyl]-methylcarbamoyl]pyrrolidin-1-yl,
(4R)-3-(1H-indazol-5-ylmethylcarbamoyl)-4-[[3-(trifluoromethyl)phenyl]methyl-carbamoyl]pyrrolidin-1-yl,
(4R)-3-(1H-indazol-5-ylmethylcarbamoyl)-4-[[3,5-bis(trifluoromethyl)phenyl]methyl-carbamoyl]pyrrolidin-1-yl,
(4R)-3-(1H-indazol-5-ylmethylcarbamoyl)-4-[[4-(trifluoromethyl)phenyl]methyl-carbamoyl]pyrrolidin-1-yl,
(4R)-3-(1H-indazol-5-ylmethylcarbamoyl)-4-[[3-(cyclopropyl)phenyl]methyl-carbamoyl]pyrrolidin-1-yl,
(4R)-3-(1H-indazol-5-ylmethylcarbamoyl)-4-[[3,5-bis(cyclopropyl)phenyl]methyl-carbamoyl]pyrrolidin-1-yl,
(4R)-3-(1H-indazol-5-ylmethylcarbamoyl)-4-[[4-(cyclopropyl)phenyl]methyl-carbamoyl]pyrrolidin-1-yl,
(4R)-3-(3H-benzimidazol-5-ylmethylcarbamoyl)-4-[[3-(trifluoromethyl)phenyl]methyl-carbamoyl]pyrrolidin-1-yl,
(4R)-3-(3H-benzimidazol-5-ylmethylcarbamoyl)-4-[[3,5-bis(trifluoromethyl)phenyl]-methylcarbamoyl]pyrrolidin-1-yl,
(4R)-3-(3H-benzimidazol-5-ylmethylcarbamoyl)-4-[[4-(trifluoromethyl)phenyl]methyl-carbamoyl]pyrrolidin-1-yl,
(4R)-3-(3H-benzimidazol-5-ylmethylcarbamoyl)-4-[[3-(cyclopropyl)phenyl]methyl-carbamoyl]pyrrolidin-1-yl,
(4R)-3-(3H-benzimidazol-5-ylmethylcarbamoyl)-4-[[3,5-bis(cyclopropyl)phenyl]methyl-carbamoyl]pyrrolidin-1-yl,
(4R)-3-(3H-benzimidazol-5-ylmethylcarbamoyl)-4-[[4-(cyclopropyl)phenyl]methyl-carbamoyl]pyrrolidin-1-yl,
(4R)-2-(1,3-benzodioxol-5-yl)-4-[[3-(trifluoromethyl)phenyl]methyl-carbamoyl]pyrrolidin-1-yl,
(4R)-2-(1,3-benzodioxol-5-yl)-4-[[3,5-bis(trifluoromethyl)phenyl]methylcarbamoyl]-pyrrolidin-1-yl,
(4R)-2-(1,3-benzodioxol-5-yl)-4-[[4-(trifluoromethyl)phenyl]methyl-carbamoyl]pyrrolidin-1-yl,
(4R)-2-(1,3-benzodioxol-5-yl)-4-methyl-pyrrolidin-1-yl,
(4R)-2-(1,3-benzodioxol-5-yl)-4-[[3-(cyclopropyl)phenyl]methylcarbamoyl]pyrrolidin-1-yl,
(4R)-2-(1,3-benzodioxol-5-yl)-4-[[3,5-bis(cyclopropyl)phenyl]methyl-carbamoyl]pyrrolidin-1-yl,
(4R)-2-(1,3-benzodioxol-5-yl)-4-[[4-(cyclopropyl)phenyl]methylcarbamoyl]pyrrolidin-1-yl,
(4R)-2-(1H-indazol-5-yl)-4-[[3-(trifluoromethyl)phenyl]methyl-carbamoyl]pyrrolidin-1-yl,
(4R)-2-(1H-indazol-5-yl)-4-[[3,5-bis(trifluoromethyl)phenyl]methyl-carbamoyl]pyrrolidin-1-yl,
(4R)-2-(1H-indazol-5-yl)-4-[[4-(trifluoromethyl)phenyl]methyl-carbamoyl]pyrrolidin-1-yl,
(4R)-2-(1H-indazol-5-yl)-3-methyl-pyrrolidin-1-yl,
(4R)-2-(1H-indazol-5-yl)-4-[[3-(cyclopropyl)phenyl]methylcarbamoyl]pyrrolidin-1-yl,
(4R)-2-(1H-indazol-5-yl)-4-[[3,5-bis(cyclopropyl)phenyl]methyl-carbamoyl]pyrrolidin-1-yl,
(4R)-2-(1H-indazol-5-yl)-4-[[4-(cyclopropyl)phenyl]methylcarbamoyl]pyrrolidin-1-yl,
(4R)-2-(3H-benzimidazol-5-yl)-4-[[3-(trifluoromethyl)phenyl]methyl-carbamoyl]pyrrolidin-1-yl,
(4R)-2-(3H-benzimidazol-5-yl)-4-[[3,5-bis(trifluoromethyl)phenyl]methylcarbamoyl]-pyrrolidin-1-yl,
(4R)-2-(3H-benzimidazol-5-yl)-4-[[4-(trifluoromethyl)phenyl]methyl-carbamoyl]pyrrolidin-1-yl,
(4R)-2-(3H-benzimidazol-5-yl)-3-methyl-pyrrolidin-1-yl,
(4R)-2-(3H-benzimidazol-5-yl)-4-[[3-(cyclopropyl)phenyl]methylcarbamoyl]pyrrolidin-1-yl,
(4R)-2-(3H-benzimidazol-5-yl)-4-[[3,5-bis(cyclopropyl)phenyl]methyl-carbamoyl]pyrrolidin-1-yl,
(4R)-2-(3H-benzimidazol-5-yl)-4-[[4-(cyclopropyl)phenyl]methylcarbamoyl]pyrrolidin-1-yl,
(4R)-2-(1,3-benzodioxol-5-yl)-3-methyl-4-[[3-(trifluoromethyl)phenyl]methyl-carbamoyl]pyrrolidin-1-yl,
(4R)-2-(1,3-benzodioxol-5-yl)-3-methyl-4-[[3,5-bis(trifluoromethyl)phenyl]methyl-carbamoyl]pyrrolidin-1-yl,
(4R)-2-(1,3-benzodioxol-5-yl)-3-methyl-4-[[4-(trifluoromethyl)phenyl]methyl-carbamoyl]pyrrolidin-1-yl],
(4R)-2-(1,3-benzodioxol-5-yl)-3-methyl-pyrrolidin-1-yl,
(4R)-2-(1,3-benzodioxol-5-yl)-3-methyl-4-[[3-(cyclopropyl)phenyl]methyl-carbamoyl]pyrrolidin-1-yl,
(4R)-2-(1,3-benzodioxol-5-yl)-3-methyl-4-[[3,5-bis(cyclopropyl)phenyl]methyl-carbamoyl]pyrrolidin-1-yl,
(4R)-2-(1,3-benzodioxol-5-yl)-3-methyl-4-[[4-(cyclopropyl)phenyl]methyl-carbamoyl]pyrrolidin-1-yl,
(4R)-2-(1H-indazol-6-yl)-3-methyl-4-[[3-(trifluoromethyl)phenyl]methyl-carbamoyl]pyrrolidin-1-yl,
(4R)-2-(1H-indazol-6-yl)-3-methyl-4-[[3,5-bis(trifluoromethyl)phenyl]methyl-carbamoyl]pyrrolidin-1-yl,
(4R)-2-(1H-indazol-6-yl)-3-methyl-4-[[4-(trifluoromethyl)phenyl]methyl-carbamoyl]pyrrolidin-1-yl,
(4R)-2-(1H-indazol-6-yl)-3-methyl-pyrrolidin-1-yl
(4R)-2-(1H-indazol-6-yl)-3-methyl-4-[[3-(cyclopropyl)phenyl]methyl-carbamoyl]pyrrolidin-1-yl,
(4R)-2-(1H-indazol-6-yl)-3-methyl-4-[[3,5-bis(cyclopropyl)phenyl]methyl-carbamoyl]pyrrolidin-1-yl,
(4R)-2-(1H-indazol-6-yl)-3-methyl-4-[[4-(cyclopropyl)phenyl]methyl-carbamoyl]pyrrolidin-1-yl,
(4R)-2-(1H-benzimidazol-5-yl)-4-[[3-(trifluoromethyl)phenyl]methyl-carbamoyl]pyrrolidin-1-yl,
(4R)-2-(1H-benzimidazol-5-yl)-4-[[3,5-bis(trifluoromethyl)phenyl]methyl-carbamoyl]pyrrolidin-1-yl,
(4R)-2-(1H-benzimidazol-5-yl)-4-[[4-(trifluoromethyl)phenyl]methyl-carbamoyl]pyrrolidin-1-yl,
(4R)-2-(1H-benzimidazol-5-yl)-3-methyl-pyrrolidin-1-yl,
(4R)-2-(1H-benzimidazol-5-yl)-4-[[3-(cyclopropyl)phenyl]methylcarbamoyl]pyrrolidin-1-yl,
(4R)-2-(1H-benzimidazol-5-yl)-4-[[3,5-bis(cyclopropyl)phenyl]methyl-carbamoyl]pyrrolidin-1-yl, and
(4R)-2-(1H-benzimidazol-5-yl)-4-[[4-(cyclopropyl)phenyl]methylcarbamoyl]pyrrolidin-1-yl,
wherein the pyrrolidine ring is numbered as follows: 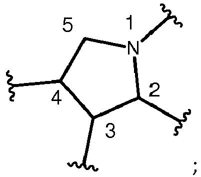
covalently bound to a second residue selected from the group consisting of hydrogen, methyl, 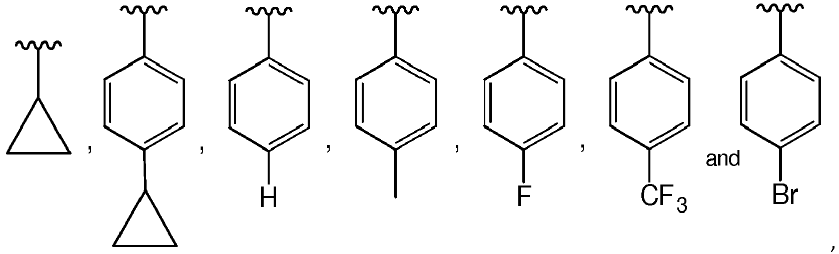
wherein the first residue is covalently bound to the second residue at the -yl position of the first residue;
preferably a compound selected from the group consisting of 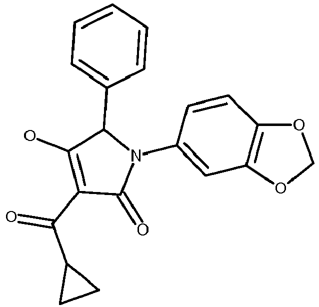
1-(1,3-benzodioxol-5-yl)-4-(cyclopropanecarbonyl)-3-hydroxy-2-phenyl-2H-pyrrol-5-one; 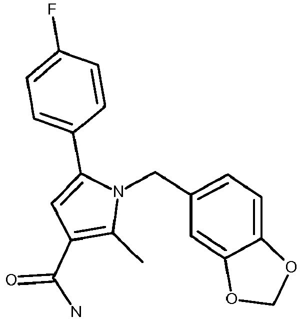
1-(1,3-benzodioxol-5-ylmethyl)-5-(4-fluorophenyl)-2-methyl-pyrrole-3-carboxamide; 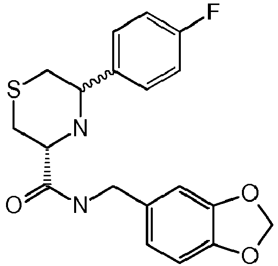
(3R)-N-(1,3-benzodioxol-5-ylmethyl)-5-(4-fluorophenyl)thiomorpholine-3-carboxamide; 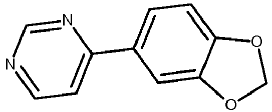
4-(1,3-benzodioxol-5-yl)pyrimidine; 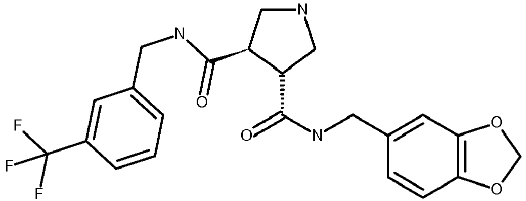
(4R)-N3-(1,3-benzodioxol-5-ylmethyl)-N4-[[3-(trifluoromethyl)phenyl]methyl]pyrrolidine-3,4-dicarboxamide; 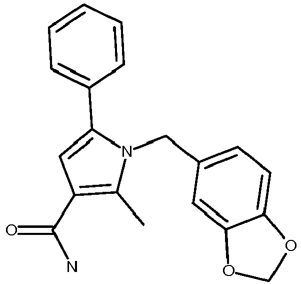
1-(1,3-benzodioxol-5-ylmethyl)-2-methyl-5-phenyl-pyrrole-3-carboxamide; 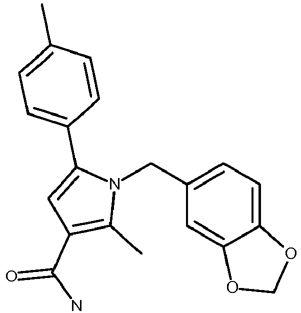
1-(1,3-benzodioxol-5-ylmethyl)-2-methyl-5-(p-tolyl)pyrrole-3-carboxamide; and 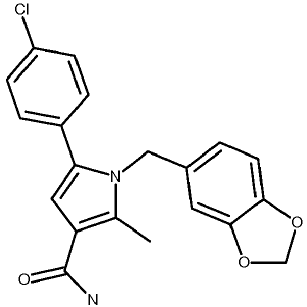
1-(1,3-benzodioxol-5-ylmethyl)-5-(4-chlorophenyl)-2-methyl-pyrrole-3-carboxamide.

12. A compound selected from the group consisting of 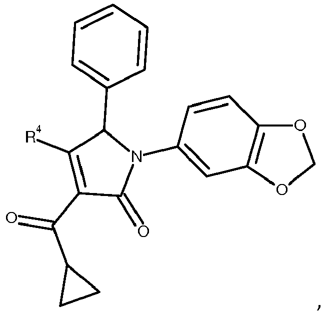 wherein **R⁴** is selected from the group consisting of hydroxyl, -O-**R¹⁴**, and -O-C(=O)-**R¹⁴**,
wherein **R¹⁴** is selected from the group consisting of
(aa) linear or branched, substituted or non-substituted (C₁₋₁₀)alkyl, preferably (C₁₋₅)alkyl, more preferably methyl, ethyl and propyl, most preferably methyl, (C₂₋₁₀)alkenyl, and (C₂₋₁₀)alkynyl;
(bb) substituted or non-substituted aromatic or non-aromatic (C₃₋₁₀)carbocycle, preferably (C₃₋₆)cylcoalkyl, more preferably (C₃)carbocycle and (C₆)carbocycle, preferably (C₆)carbocycle, more preferably phenyl that is mono-substituted in *para* position by (C₃)carbocycle or -(CF₃) or di-substituted in *meta* position by (C₃)carbocycle or -(CF₃); and
(cc) substituted or non-substituted aromatic or non-aromatic, preferably aromatic, (C₃₋₆)-heterocycle having 1 to 3 heteroatoms each independently selected from N, O and S;
preferably a compound selected from the group consisting of 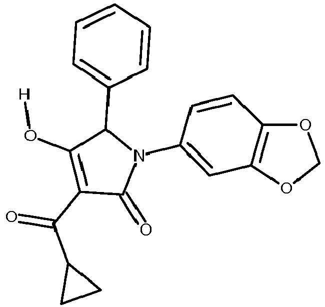
1-(1,3-benzodioxol-5-yl)-4-(cyclopropanecarbonyl)-3-hydroxy-2-phenyl-2H-pyrrol-5-one; 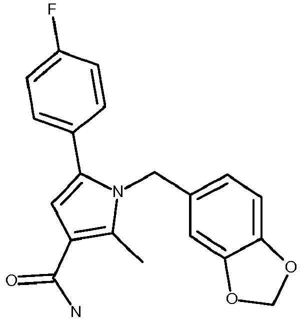
1-(1,3-benzodioxol-5-ylmethyl)-5-(4-fluorophenyl)-2-methyl-pyrrole-3-carboxamide; 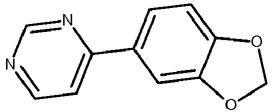
4-(1,3-benzodioxol-5-yl)pyrimidine; and 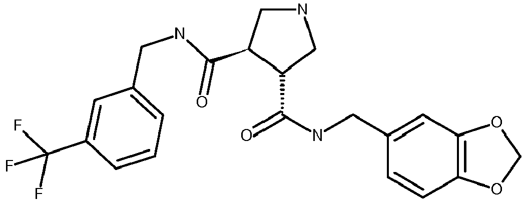
(4R)-N3-(1,3-benzodioxol-5-ylmethyl)-N4-[[3-(trifluoromethyl)phenyl]methyl]pyrrolidine-3,4-dicarboxamide for use according to claim 1.

13. The compound for use according to any of claims 1 to 12, wherein the compound inhibits the PHF (paired helical filament) Tau hyperphosphorylation, preferably also inhibits phosphorylation of the serine/arginine-rich splicing factor 1 (SRSF1, ASF-1, SF2) by a kinase, preferably by the G-protein-coupled receptor kinase 2 (GRK2, ADRBK1), more preferably also inhibits the formation of Abeta peptides and Abeta plaques, more preferably also inhibits neurodegeneration and/or neuronal loss, preferably hippocampal neuronal loss.

14. A pharmaceutical composition, comprising as active substance a compound for use according to any one of claims 1 to 13 or a pharmaceutically acceptable derivative thereof, optionally combined with excipients and/or carriers.

15. The compound or pharmaceutical composition for use according to any of claims 1 to 14, wherein CNS- and neurodegenerative diseases are selected from the group consisting of for use in the treatment of CNS- and neurodegenerative diseases selected from the group consisting of dementia-associated CNS- and neurodegenerative disorders, preferably schizophrenia with dementia, depression-associated CNS- and neurodegenerative disorders, preferably depression and depression-related symptoms, more preferably anhedonia and anorexia, brain injury, preferably traumatic brain injury, cerebrovascular disease-induced neurodegeneration, preferably ischemic stroke-induced neurodegeneration, hypertension-induced neurodegeneration, atherosclerosis-induced neurodegeneration, amyloid angiopathy-induced neurodegeneration, preferably small-vessel cerebrovascular disease, motor neuron disease, ALS, multiple sclerosis, familial and sporadic forms of Alzheimer's Disease, vascular dementia, Morbus Parkinson, chromosome-17-linked Morbus Parkinson, frontotemporal dementia, Korsakoff's psychosis, Lewy Body diseases, progressive supranuclear palsy, corticobasal degeneration, Pick's disease, Huntington's disease, thalamic degeneration, prion-associated diseases, preferably Creutzfeld-Jacob disease, HIV-associated dementia, diabetes-induced neuropathy, neurodegenerative symptoms of ageing, preferably loss of appetite or greying of hair, cognitive-related disorders, mild cognitive impairment, age-associated memory impairment, age-associated cognitive decline, vascular cognitive impairment, central and peripheral neuronal symptoms of atherosclerosis and ischemia, stress-related CNS- and neurodegenerative disorders, attention deficit disorders, attention deficit hyperactivity disorders, memory disturbances in children, and progeria infantilis.

16. The compound or pharmaceutical composition for use according to any of claims 1 to 15, wherein the medical treatment is selected from the group consisting of
(i) therapeutic and prophylactic treatment of familial and sporadic forms of Alzheimer's Disease;
(ii) therapeutic and prophylactic treatment of tauopathies;
(iii) therapeutic and prophylactic treatment of Morbus Parkinson;
(iv) therapeutic and prophylactic treatment of diabetes-induced neuropathy, preferably diabetes type 2; and
(v) therapeutic and prophylactic treatment of dementias associated with neurodegeneration.

17. A method for the therapeutic or prophylactic treatment of a patient suffering or likely of suffering from a CNS- or neurodegenerative disease, preferably a mammalian patient, more preferably a human patient, the method comprising the step of administering a therapeuticcally or prophylactically effective amount of a compound or pharmaceutical composition for use according to any one of claims 1 to 14 to the patient in need of such treatment.
